# EUROPEAN PATENT APPLICATION

(11) **EP 2 700 634 A1**
(43) Date of publication of application: **26.02.2014**
(21) Application number: 12181047.7
(22) Date of filing: 20.08.2012
(51) Int. Cl.: C07D 231/14, A01N 43/56

(54) **5-difluoromethylpyrazole amides having herbicidal activity**

(71) Applicant: BASF SE, 67056 Ludwigshafen (DE)
(72) Inventor: Moberg, William Karl, 67433 Neustadt (DE); Major, Julia, 67251 Freinsheim (DE); Seitz, Thomas, 68519 Viernheim (DE); Parra Rapado, Liliana, 77654 Offenburg (DE); Newton, Trevor William, 67435 Neustadt (DE); Evans, Richard Roger, 67117 Limburgerhof (DE)

(57) **Abstract**

The present invention provides a provides a pyrazole amide compound of the formula I 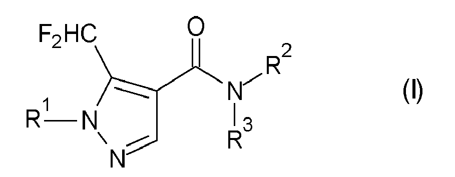 or an agriculturally acceptable salt thereof, wherein the variables in the formula I are defined as in the description. Pyrazole amides of formula I are useful as herbicides.

## Description

The present invention relates to pyrazole amide compounds of the general formula I defined below and to their use as herbicides. Moreover, the invention relates to compositions for crop protection and to a method for controlling unwanted vegetation.

WO 2009/116151 A1 and WO 2009/116558 A1 describe certain 1-phenyl-5-difluoromethylpyrazole-4-carboxamide derivatives having herbicidal activity.

However, the herbicidal properties of these known compounds with regard to the harmful plants are not always entirely satisfactory. In agriculture, there is a constant demand to develop novel active ingredients, which complement or outperform present methods of treatment regarding activity, selectivity and environmental safety.

It is therefore an object of the present invention to provide compounds having improved herbicidal action. To be provided are in particular compounds which have high herbicidal activity, in particular even at low application rates, and which are sufficiently compatible with crop plants for commercial utilization.

These and further objects are achieved by the pyrazole amide compound of the formula I, defined below, and by its agriculturally acceptable salts.

Accordingly, the present invention provides a pyrazole amide compound of the formula I 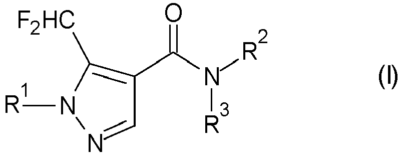 wherein
- R¹: is H, C₁-C₁₀-alkyl, C₁-C₁₀-haloalkyl, C₂-C₁₀-hydroxyalkyl, C₁-C₆-alkoxy-C₁-C₆-alkyl, C₁-C₆-haloalkoxy-C₁-C₆-alkyl, C₁-C₆-alkylthio-C₁-C₆-alkyl, C₁-C₆-haloalkylthio-C₁-C₆-alkyl, C₁-C₆-alkylsulfinyl-C₁-C₆-alkyl, C₁-C₆-haloalkylsulfinyl-C₁-C₆-alkyl, C₁-C₆-alkylsulfonyl-C₁-C₆-alkyl, C₁-C₆-haloalkylsulfonyl-C₁-C₆-alkyl, C₃-C₆-cycloalkyl, 1-methylcycloprop-1-yl, 2-methylcycloprop-1-yl, 2,2-dimethylcycloprop-1-yl, 2,2,3,3-tetramethylcycloprop-1-yl, C₃-C₆-halocycloalkyl, C₃-C₆-cycloalkyl-C₁-C₆-alkyl, C₃-C₆-halocycloalkyl-C₁-C₆-alkyl, C₂-C₁₀-alkenyl, C₂-C₁₀-haloalkenyl, C₂-C₁₀-hydroxyalkenyl, C₃-C₁₀-alkadienyl, C₃-C₆-alkynyl, C₃-C₆-haloalkynyl, CH₂CN, CH(CN)₂, N,N-di-(C₁-C₆)-alkylamino-C₁-C₆-alkyl, C₁-C₆-dialkoxy-C₁-C₆-alkyl, C₁-C₆-dialkylthio-C₁-C₆-alkyl, C₁-C₃-alkoxy-C₁-C₃-alkylthio-C₁-C₆-alkyl, or a heterocyclic group selected from the formulae H1, H2 or H3 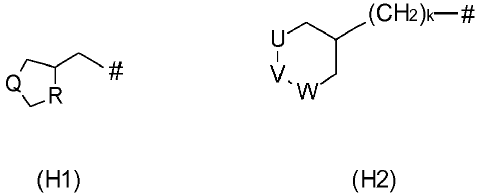 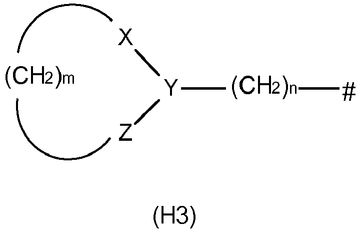 wherein
each of Q and R in the formula H1 is O or S;
U-V-W in the formula H2 is selected from the group consisting of CH₂-CH₂-O, CH₂-CH₂-NH, CH₂-CH₂-N(CH₃), CH₂-O-CH₂, CH₂-NH-CH₂, CH₂-N(CH₃)-CH₂, O-CH₂-O, O-CH₂-S, and S-CH₂-S;
k in the formula H2 is 0 or 1;
X-Y-Z in the formula H3 is selected from the group consisting of CH₂-N-CH₂, O-CH-CH₂, O-CH-O, S-CH-CH₂, S-CH-S, and O-CH-S;
m in the formula H3 is 1, 2 or 3;
n in the formula H3 is 0, 1 or 2, with the proviso that, when n is 0, X-Y-Z is not CH₂-N-CH₂;
# in each of the formulae H1, H2 or H3 denotes the bonding site to the remainder of the formula I;
- R²: is hydrogen, C₁-C₄-alkyl, C₃-C₆-cycloalkyl or C₁-C₄-alkoxy; and
- R³: is hydrogen, C₁-C₄-alkyl, C₃-C₆-cycloalkyl or C₁-C₄-alkoxy;
or an agriculturally acceptable salt thereof.

The present invention also provides the use of pyrazole amide compounds of formula I as herbicides, i.e. for controlling harmful plants.

The present invention also provides agrochemical compositions comprising at least one pyrazole amide compound of formula I and auxiliaries customary for formulating crop protection agents.

The present invention also provides a method for preparing a composition as described herein, which comprises mixing a herbicidally effective amount of at least one pyrazole amide compound of the formula I or of an agriculturally acceptable salt thereof, and auxiliaries customary for formulating crop protection agents.

The present invention furthermore provides a method for controlling unwanted vegetation where a herbicidal effective amount of at least one pyrazole amide compound of formula I is allowed to act on plants, their seeds and/or their habitat. Application can be done before, during and/or after, preferably during and/or after, the emergence of the undesirable plants.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention.

As used herein, the terms "controlling" and "combating" are synonyms.
As used herein, the terms "undesirable vegetation" and "harmful plants" are synonyms.

If the pyrazole amide compounds of formula I as described herein are capable of forming geometrical isomers, for example E/Z isomers, it is possible to use both, the pure isomers and mixtures thereof, in the compositions according to the invention.

If the pyrazole amide compounds of formula I as described herein have one or more centers of chirality and, as a consequence, are present as enantiomers or diastereomers, it is possible to use both, the pure enantiomers and diastereomers and their mixtures, in the compositions according to the invention.

If the pyrazole amide compounds of formula I as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

The organic moieties mentioned in the definition of the variables R¹, R² and R³ are - like the term halogen - collective terms for individual enumerations of the individual group members. The term halogen denotes in each case fluorine, chlorine, bromine or iodine especially fluorine and chlorine. All hydrocarbon chains, i.e. all alkyl, can be straight-chain or branched, the prefix Cₓ-C_{y} denoting in each case the possible number of carbon atoms in the group.

Examples of such meanings are:
- C₁-C₂-alkyl and also the C₁-C₂-alkyl moieties of C₁-C₂-alkoxy-C₁-C₂-alkyl include CH₃ and C₂H₅;
- C₁-C₄-alkyl and also the C₁-C₄-alkyl moieties of C₃-C₆-cydoalkyl-C₁-C₄-alkyl: C₁-C₂-alkyl as mentioned above, and also, for example, n-propyl, CH(CH₃)₂, n-butyl, CH(CH₃)-C₂H₅, CH₂-CH(CH₃)₂ and C(CH₃)₃;
- C₁-C₆-alkyl and also the C₁-C₆-alkyl moieties of C₁-C₆-alkoxy-C₁-C₆-alkyl, C₁-C₆-haloalkoxy-C₁-C₆-alkyl, C₁-C₆-alkylthio-C₁-C₆-alkyl, C₁-C₆-haloalkylthio-C₁-C₆-alkyl, C₁-C₆-alkylsulfinyl-C₁-C₆-alkyl, C₁-C₆-haloalkylsulfinyl-C₁-C₆-alkyl, C₁-C₆-alkylsulfonyl-C₁-C₆-alkyl, C₁-C₆-haloalkylsulfonyl-C₁-C₆-alkyl, C₃-C₆-cydoalkyl-C₁-C₆-alkyl, C₃-C₆-halocydoalkyl-C₁-C₆-alkyl, N,N-di-(C₁-C₆)-alkylamino-C₁-C₆-alkyl, C₁-C₆-dialkoxy-C₁-C₆-alkyl, C₁-C₆-dialkylthio-C₁-C₆-alkyl, C₁-C₃-alkoxy-C₁-C₃-alkylthio-C₁-C₆-alkyl: C₁-C₄-alkyl as mentioned above, and also, for example, n-pentyl, 1-methylbutyl, 2-methylbutyl, 3-methylbutyl, 2,2-dimethylpropyl, 1,1-dimethylpropyl, 1,2-dimethylpropyl, and 1-ethylpropyl n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl, or n-hexyl;
- C₁-C₁₀-alkyl: C₁-C₆-alkyl as mentioned above, and also, for example, 3,7-dimethyl-1-octyl;
- C₁-C₄-haloalkyl: a C₁-C₄-alkyl radical as mentioned above which is partially or fully substituted by fluorine, chlorine, bromine and/or iodine, for example, chloromethyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 1-fluoroethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 1,1-difluoro-2-propyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl, 1-trifluoromethyl-1,2,2,2-tetrafluoroethyl and 1,1,1-trifluoroprop-2-yl;
- C₁-C₆-haloalkyl and also the C₁-C₆-haloalkyl moieties of C₁-C₆-haloalkylthio-C₁-C₆-alkyl, C₁-C₆-haloalkylsulfinyl-C₁-C₆-alkyl, and C₁-C₆-haloalkylsulfonyl-C₁-C₆-alkyl: C₁-C₄-haloalkyl as mentioned above, and also, for example, 3-chloro-3-methyl-1-butyl, 5-fluoropentyl, 5-chloropentyl, 5-bromopentyl, 5-iodopentyl and undecafluoropentyl 6-fluorohexyl, 6-chlorohexyl, 6-bromohexyl, 6-iodohexyl and dodecafluorohexyl;
- C₁-C₁₀ haloalkyl: C₁-C₆-haloalkyl as mentioned above, and also, for example, 7-chloro-3,7-dimethyl-1-octyl;
- C₂-C₄-hydroxyalkyl: a C₁-C₄-alkyl radical as mentioned above which is substituted by one or more hydroxyl groups, for example, 2-hydroxyethyl, 3-hydroxypropyl, 2,3-dihydroxypropyl, 1,3-dihydroxy-2-propyl, 2,3-dihydroxybutyl, 3,4-dihydroxy-1-butyl, 3,4-dihydroxy-2-butyl or 2,3,4-trihydroxybutyl;
- C₂-C₁₀-hydroxyalkyl: C₂-C₄-hydroxyalkyl as mentioned above, and also, for example, 3-hydroxy-3-methyl-1-butyl, 5-hydroxypentyl, 6-hydroxyhexyl or 7-hydroxy-3,7-dimethyl-1-octyl;
- C₂-C₅-alkenyl: ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl and 1-ethyl-2-propenyl;
- C₃-C₅-alkenyl: C₂-C₅-alkenyl as mentioned above with the exception of C₂-alkenyl radicals;
- C₂-C₁₀-alkenyl: C₂-C₅-alkenyl as mentioned above, and also, for example, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl, 3,7-dimethyl-2-octen-1-yl and 3,7-dimethyl-6-octen-1-yl;
- C₂-C₁₀-haloalkenyl: a C₂-C₁₀-alkenyl radical as mentioned above which is partially or fully substituted by fluorine, chlorine, bromine and/or iodine, for example 2-dibromoethenyl, 2-fluoro-2-bromoethenyl, 2-fluoroprop-2-en-1-yl, 2-chloroprop-2-en-1-yl, 3-chloroprop-2-en-1-yl, 2,3-dichloroprop-2-en-1-yl, 3,3-dichloroprop-2-en-1-yl, 2,3,3-trichloro-2-en-1-yl, 2,3-dichlorobut-2-en-1-yl, 2-bromoprop-2-en-1-yl, 3-bromoprop-2-en-1-yl, 2,3-dibromoprop-2-en-1-yl, 3,3-dibromoprop-2-en-1-yl, 2,3,3-tribromo-2-en-1-yl or 2,3-dibromobut-2-en-1-yl, and 7-chloro-3,7-dimethyl-2-octene-1-yl;
- C₃-C₅-haloalkenyl: C₂-C₆-haloalkenyl as mentioned above with the exception of C₂- and C₆-C₁₀-haloalkenyl radicals;
- C₂-C₁₀-hydroxyalkenyl: unsaturated straight-chain or branched hydrocarbon radicals having 2 to 10 carbon atoms and one double bond in any position (as mentioned above), where one or more of the hydrogen atoms in these groups are replaced by OH groups, for example 3-hydroxymethyl-2-butenyl, 3-hydroxymethyl-1-butenyl, 3-hydroxy-3-methyl-2-butenyl and 3,7-dimethyl-7-hydroxyoct-2-ene-1-yl;
- C₃-C₁₀-alkadienyl: unsaturated straight-chain or branched hydrocarbon radicals having 3 to 10 carbon atoms and two double bonds in any position, for example allenyl, 1-methylallenyl, 3-methylallenyl, 3,3-dimethylallenyl, 1,3,3-trimethylallenyl, penta-2,4-dien-1-yl, 5-methylhexa-2,4-dien-1-yl and 3,7-dimethylocta-2,6-dien-1-yl;
- C₃-C₄-alkynyl: for example 1-propynyl, 2-propynyl, 1-butynyl, 2-butynyl, 3-butynyl, and 1-methyl-2-propynyl;
- C₃-C₆-alkynyl and also the alkynyl moieties of C₃-C₆-haloalkynyl: C₃-C₄-alkynyl as mentioned above, and also, for example, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl and 1-ethyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
- C₃-C₆-cycloalkyl and also the cycloalkyl moieties of C₃-C₆-cycloalkyl-C₁-C₆-alkyl: monocyclic saturated hydrocarbons having 3 to 6 ring members, such as cyclopropyl, cyclobutyl, cyclopentyl and cyclohexyl;
- C₃-C₆-halocycloalkyl and also the C₃-C₆-halocycloalkyl moieties of C₃-C₆-halocycloalkyl-C₁-C₆-alkyl: a C₃-C₆-cycloalkyl radical as mentioned above which is partially or fully substituted by fluorine, chlorine, bromine and/or iodine, in particular fluorine and chlorine, for example 2,2-difluorocyclopropyl and 2,2-dichlorocyclopropyl;
- C₁-C₂-alkoxy and also the C₁-C₂-alkoxy moieties of C₁-C₂-alkoxy-C₁-C₂-alkyl: methoxy and ethoxy;
- C₁-C₃-alkoxy and also the C₁-C₃-alkoxy moieties of C₁-C₃-alkoxy-C₁-C₃-alkylthio-C₁-C₆-alkyl: C₁-C₂-alkoxy as mentioned above, and also, for example, propoxy and 1-methylethoxy;
- C₁-C₄-alkoxy: C₁-C₃-alkoxy as mentioned above, and also, for example, butoxy, 1-methylpropoxy, 2-methylpropoxy and 1,1-dimethylethoxy;
- C₁-C₆-alkoxy and also the C₁-C₆-alkoxy moieties of C₁-C₆-alkoxy-C₁-C₆-alkyl and C₁-C₆-dialkoxy-C₁-C₆-alkyl: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy;
- C₁-C₆-haloalkoxy and also the C₁-C₆-haloalkoxy moieties of C₁-C₆-haloalkoxy-C₁-C₆-alkyl: for example OCH₂F, OCHF₂, OCF₃, OCH₂Cl OCHCl₂, OCCl₃, chlorofluoromethoxy, di-chlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy, nonafluorobutoxy, 5-fluoropentoxy, 5-chloropentoxy, 5-bromopentoxy, 5-iodopentoxy, undecafluoropentoxy, 6-fluorohexoxy, 6-chlorohexoxy, 6-bromohexoxy, 6-iodohexoxy or dodecafluorohexoxy;
- C₁-C₃-alkylthio: C₁-C₃-alkoxy as defined above, where oxygen is replaced by sulfur;
- C₁-C₆-alkylthio, C₁-C₆-alkylsulfinyl, and C₁-C₆-alkylsulfonyl: C₁-C₆-alkoxy as defined above, where oxygen is replaced by sulfur, SO or SO₂, respectively; and
- C₁-C₆-haloalkylthio, C₁-C₆-haloalkylsulfinyl and C₁-C₆-haloalkylsulfonyl: C₁-C₆-haloalkoxy as defined above, where the oxygen is replaced by sulfur, S=O or O=S=O, respectively.

The preferred embodiments of the invention mentioned herein below have to be understood as being preferred either independently from each other or in combination with one another.

Preferably, R¹ is selected from the group consisting of C₁-C₄-alkyl, cyclopropyl, 1-methylcycloprop-1-yl, cyclopropylmethyl, cyclobutyl, C₁-C₄-haloalkyl, C₂-C₄-hydroxyalkyl, C₁-C₂-alkoxy-Ci-C₂-alkyl, C₂-C₅-alkenyl, allenyl, C₃-C₅-haloalkenyl, C₃-C₄-alkynyl, CH₂CN, and CH(CN)₂.

More preferably, R¹ is selected from the group consisting of ethyl, 1-propyl, allyl, cyclopropyl, 1-methylcycloprop-1-yl, cyclopropylmethyl, cyclobutyl and t-butyl.

In another embodiment, R¹ is a heterocyclic group of the formula H1. The hetorocyclic group H1 can be selected from the group consisting of the heterocyclic groups H1.1, H1.2, H1.3, and H1.4 as shown below:

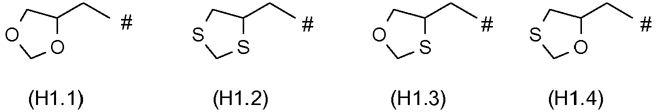

Preferred heterocyclic groups H1 are selected from H1.1 and H1.2.

In another embodiment, R¹ is a heterocyclic group of the formula H2. The hetorocyclic group H2 can be selected from the group consisting of the heterocyclic groups H2.1, H2.2, H2.3, H2.4, H2.5, H2.6, H2.7, H2.8, H2.9, H2.10, H2.11, H2.12, H2.13, H2.14, H2.15, H2.16, H2.17, and H2.18 as shown below:

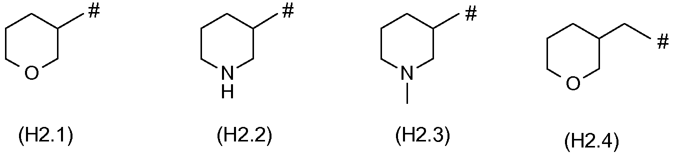

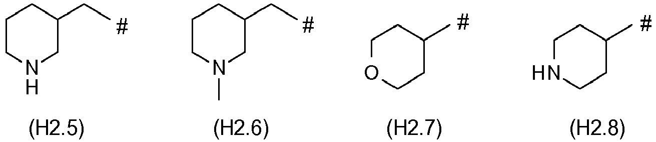

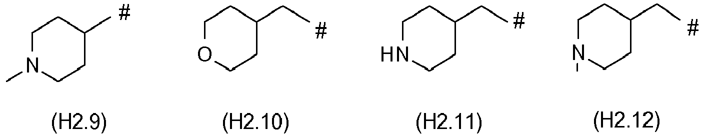

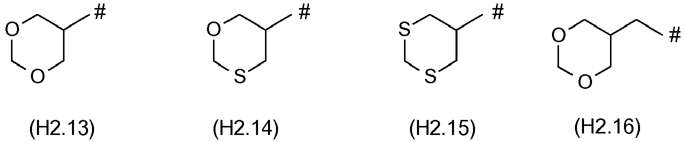

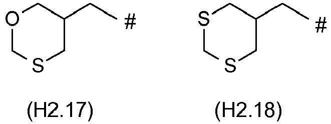

Preferred heterocyclic groups H2 are selected from the group consisting of the heterocyclic groups H2.1, H2.4, H2.7, H2.9, H2.13, H2.15, H2.16 and H2.18.

In another embodiment, R¹ is a heterocyclic group of the formula H3. Preferably, the heterocyclic group H3 can be selected from the group consisting of the heterocyclic groups H3.1, H3.2, H3.3, H3.4 and H3.5 as shown below:

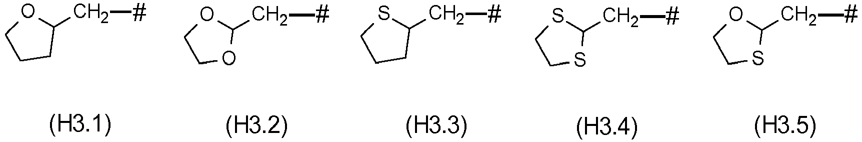

Preferred heterocyclic groups H3 are selected from the group consisting of the heterocyclic groups H3.1, H3.3 and H3.4.

In a preferred embodiment, R² is C₁-C₄-alkyl or C₃-C₆-cycloalkyl, more preferably C₃-C₆-cycloalkyl and in particular cyclopropyl.

In another preferred embodiment, R³ is hydrogen or methyl and in particular hydrogen.

According to a another preferred embodiment of the invention, preference is given to those pyrazole amide compounds of formula I wherein
- R¹: is C₁-C₄-alkyl, cyclopropyl, 1-methylcycloprop-1-yl, cyclopropylmethyl, cyclobutyl, C₁-C₄-haloalkyl, C₂-C₄-hydroxyalkyl, C₁-C₂-alkoxy-C₁-C₂-alkyl, C₂-C₅-alkenyl, allenyl, C₃-C₅-haloalkenyl, C₃-C₄-alkynyl, CH₂CN or CH(CN)₂;
- R²: is C₁-C₄-alkyl or C₃-C₆-cycloalkyl; and
- R³: is hydrogen or methyl;
and their agriculturally acceptable salts.

Particular preference is given to those pyrazole amide compounds of formula I wherein
- R¹: is C₁-C₄-alkyl, cyclopropyl, 1-methylcycloprop-1-yl, cyclopropylmethyl, cyclobutyl, C₁-C₄-haloalkyl, C₂-C₄-hydroxyalkyl, C₁-C₂-alkoxy-C₁-C₂-alkyl, C₂-C₅-alkenyl, allenyl, C₃-C₅-haloalkenyl, C₃-C₄-alkynyl, CH₂CN or CH(CN)₂;
- R²: is cyclopropyl; and
- R³: is hydrogen;
and their agriculturally acceptable salts.

According to another preferred embodiment of the invention, preference is given to those pyrazole amide compounds of formula I wherein
- R²: is cyclopropyl; and
- R³: is hydrogen;
and their agriculturally acceptable salts. These compounds correspond to the formula I.1 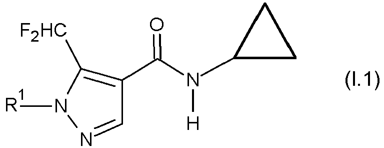 where the variables have the meanings defined at the outset and preferably the meanings mentioned as preferred.

Even more particular preference is given to those pyrazole amide compounds of formula I wherein
- R¹: is ethyl, 1-propyl, allyl, cyclopropyl, 1-methylcycloprop-1-yl, cyclopropylmethyl, cyclobutyl or t-butyl;
- R²: is cyclopropyl; and
- R³: is hydrogen;
and their agriculturally acceptable salts. These compounds correspond to the formula 1.1 as depicted above wherein R¹ is ethyl, 1-propyl, allyl, cyclopropyl, 1-methylcycloprop-1-yl, cyclopropylmethyl, cyclobutyl or t-butyl.

A further embodiment relates to agriculturally acceptable salts of the compound of the formula I. As a rule, the nature of the salt of the compound of formula I is immaterial. In general, suitable salts are the salts of protic acids strong enough to protonate the pyrazole ring, and whose anions do not adversely affect the herbicidal activity of pyrazole amide compounds of formula I. Suitable acids are, in particular, hydrogen chloride, hydrogen bromide, sulfuric acid, phosphoric acid, or nitric acid. The repective salts are the chlorides, bromides, sulfates, phosphates and nitrates of the compound of formula I.

In particular with a view to their use, preference is given to the compounds of the formula I compiled in the tables below. The groups mentioned for a substituent in the tables are furthermore per se, independently of the combination in which they are mentioned, a particularly preferred aspect of the substituent in question.
Table 1
   Compounds of the formula I, in which R¹ is H and the combination of R² and R³ for a compound corresponds in each case to one row of table A
Table 2
   Compounds of the formula I, in which R¹ is methyl and the combination of R² and R³ for a compound corresponds in each case to one row of table A
Table 3
   Compounds of the formula I, in which R¹ is CHF₂ and the combination of R² and R³ for a compound corresponds in each case to one row of table A
Table 4
   Compounds of the formula I, in which R¹ is ethyl and the combination of R² and R³ for a compound corresponds in each case to one row of table A
Table 5
   Compounds of the formula I, in which R¹ is CH₂OCH₃ and the combination of R² and R³ for a compound corresponds in each case to one row of table A
Table 6
   Compounds of the formula I, in which R¹ is CH₂OCH₂CH₃ and the combination of R² and R³ for a compound corresponds in each case to one row of table A
Table 7
   Compounds of the formula I, in which R¹ is CH₂CH₂F and the combination of R² and R³ for a compound corresponds in each case to one row of table A
Table 8
   Compounds of the formula I, in which R¹ is CH₂CH₂Cl and the combination of R² and R³ for a compound corresponds in each case to one row of table A
Table 9
   Compounds of the formula I, in which R¹ is CH₂CHF₂ and the combination of R² and R³ for a compound corresponds in each case to one row of table A
Table 10
   Compounds of the formula I, in which R¹ is CH₂CHCl₂ and the combination of R² and R³ for a compound corresponds in each case to one row of table A
Table 11
   Compounds of the formula I, in which R¹ is CH₂CF₃ and the combination of R² and R³ for a compound corresponds in each case to one row of table A
Table 12
   Compounds of the formula I, in which R¹ is CH₂CCl₃ and the combination of R² and R³ for a compound corresponds in each case to one row of table A
Table 13
   Compounds of the formula I, in which R¹ is CH₂CH₂OH and the combination of R² and R³ for a compound corresponds in each case to one row of table A
Table 14
   Compounds of the formula I, in which R¹ is CH₂CH₂OCH₃ and the combination of R² and R³ for a compound corresponds in each case to one row of table A
Table 15
   Compounds of the formula I, in which R¹ is CH₂CH₂OCH₂CH₃ and the combination of R² and R³ for a compound corresponds in each case to one row of table A
Table 16
   Compounds of the formula I, in which R¹ is n-propyl and the combination of R² and R³ for a compound corresponds in each case to one row of table A
Table 17
   Compounds of the formula I, in which R¹ is i-propyl and the combination of R² and R³ for a compound corresponds in each case to one row of table A
Table 18
   Compounds of the formula I, in which R¹ is cyclopropyl and the combination of R² and R³ for a compound corresponds in each case to one row of table A
Table 19
   Compounds of the formula I, in which R¹ is 1-methyl-cycloprop-1-yl and the combination of R² and R³ for a compound corresponds in each case to one row of table A
Table 20
   Compounds of the formula I, in which R¹ is 1-fluoro-cycloprop-1-yl and the combination of R² and R³ for a compound corresponds in each case to one row of table A
Table 21
   Compounds of the formula I, in which R¹ is 2,2-difluorocyclopropyl and the combination of R² and R³ for a compound corresponds in each case to one row of table A
Table 22
   Compounds of the formula I, in which R¹ is allyl and the combination of R² and R³ for a compound corresponds in each case to one row of table A
Table 23
   Compounds of the formula I, in which R¹ is 2-chloroallyl and the combination of R² and R³ for a compound corresponds in each case to one row of table A
Table 24
   Compounds of the formula I, in which R¹ is 3-chloroallyl and the combination of R² and R³ for a compound corresponds in each case to one row of table A
Table 25
   Compounds of the formula I, in which R¹ is 3,3-dichloroallyl and the combination of R² and R³ for a compound corresponds in each case to one row of table A
Table 26
   Compounds of the formula I, in which R¹ is 2,3,3-trichloroallyl and the combination of R² and R³ for a compound corresponds in each case to one row of table A
Table 27
   Compounds of the formula I, in which R¹ is 2-fluoroallyl and the combination of R² and R³ for a compound corresponds in each case to one row of table A
Table 28
   Compounds of the formula I, in which R¹ is 3-fluoroallyl and the combination of R² and R³ for a compound corresponds in each case to one row of table A
Table 29
   Compounds of the formula I, in which R¹ is 3,3-difluoroallyl and the combination of R² and R³ for a compound corresponds in each case to one row of table A
Table 30
   Compounds of the formula I, in which R¹ is 2,3,3-trifluoroallyl and the combination of R² and R³ for a compound corresponds in each case to one row of table A
Table 31
   Compounds of the formula I, in which R¹ is 3,3-dimethylallyl and the combination of R² and R³ for a compound corresponds in each case to one row of table A
Table 32
   Compounds of the formula I, in which R¹ is 1,1-difluorprop-2-yl and the combination of R² and R³ for a compound corresponds in each case to one row of table A
Table 33
   Compounds of the formula I, in which R¹ is CH₂CN and the combination of R² and R³ for a compound corresponds in each case to one row of table A
Table 34
   Compounds of the formula I, in which R¹ is propargyl and the combination of R² and R³ for a compound corresponds in each case to one row of table A
Table 35
   Compounds of the formula I, in which R¹ is n-butyl and the combination of R² and R³ for a compound corresponds in each case to one row of table A
Table 36
   Compounds of the formula I, in which R¹ is i-butyl and the combination of R² and R³ for a compound corresponds in each case to one row of table A
Table 37
   Compounds of the formula I, in which R¹ is s-butyl and the combination of R² and R³ for a compound corresponds in each case to one row of table A
Table 38
   Compounds of the formula I, in which R¹ is t-butyl and the combination of R² and R³ for a compound corresponds in each case to one row of table A
Table 39
   Compounds of the formula I, in which R¹ is CH₂CH₂SCH₃ and the combination of R² and R³ for a compound corresponds in each case to one row of table A
Table 40
   Compounds of the formula I, in which R¹ is CH₂CH₂SOCH₃ and the combination of R² and R³ for a compound corresponds in each case to one row of table A
Table 41
   Compounds of the formula I, in which R¹ is CH₂CH₂SO₂CH₃ and the combination of R² and R³ for a compound corresponds in each case to one row of table A
Table 42
   Compounds of the formula I, in which R¹ is 2-butyn-1-yl and the combination of R² and R³ for a compound corresponds in each case to one row of table A
Table 43
   Compounds of the formula I, in which R¹ is CH(CN)₂ and the combination of R² and R³ for a compound corresponds in each case to one row of table A
Table 44
   Compounds of the formula I, in which R¹ is the heterocyclic group of the formula H3.1 as shown above and the combination of R² and R³ for a compound corresponds in each case to one row of table A
Table 45
   Compounds of the formula I, in which R¹ is the heterocyclic group of the formula H3.2 as shown above and the combination of R² and R³ for a compound corresponds in each case to one row of table A
Table 46
   Compounds of the formula I, in which R¹ is CF₃ and the combination of R² and R³ for a compound corresponds in each case to one row of table A
Table 47
   Compounds of the formula I, in which R¹ is vinyl and the combination of R² and R³ for a compound corresponds in each case to one row of table A
Table 48
   Compounds of the formula I, in which R¹ is allenyl and the combination of R² and R³ for a compound corresponds in each case to one row of table A
Table 49
   Compounds of the formula I, in which R¹ is cyclopropylmethyl and the combination of R² and R³ for a compound corresponds in each case to one row of table A
Table 50
   Compounds of the formula I, in which R¹ is cyclobutyl and the combination of R² and R³ for a compound corresponds in each case to one row of table A
Table 51
   Compounds of the formula I, in which R¹ is cyclopentyl and the combination of R² and R³ for a compound corresponds in each case to one row of table A
Table 52
   Compounds of the formula I, in which R¹ is 4,4-difluoro-3-butenyl and the combination of R² and R³ for a compound corresponds in each case to one row of table

**Table A**

| No. | R2 | R3 |
|---|---|---|
| A.1 | H | H |
| A.2 | methyl | H |
| A.3 | methoxy | H |
| A.4 | ethyl | H |
| A.5 | ethoxy | H |
| A.6 | n-propyl | H |
| A.7 | i-propyl | H |
| A.8 | cyclopropyl | H |
| A.9 | n-butyl | H |
| A.10 | i-butyl | H |
| A.11 | s-butyl | H |
| A.12 | t-butyl | H |
| A.13 | methyl | methyl |
| A.14 | methoxy | methyl |
| A.15 | ethyl | methyl |
| A.16 | ethoxy | methyl |
| A.17 | n-propyl | methyl |
| A.18 | i-propyl | methyl |
| A.19 | cyclopropyl | methyl |
| A.20 | n-butyl | methyl |
| A.21 | i-butyl | methyl |
| A.22 | s-butyl | methyl |
| A.23 | t-butyl | methyl |

The pyrazole amide compounds of formula I according to the invention can be prepared by standard processes of organic chemistry, for example by any the following methods.

The pyrazole amide compounds of formula I may be synthesized from the corresponding esters II by methods well known in the art, as outlined in the following scheme:

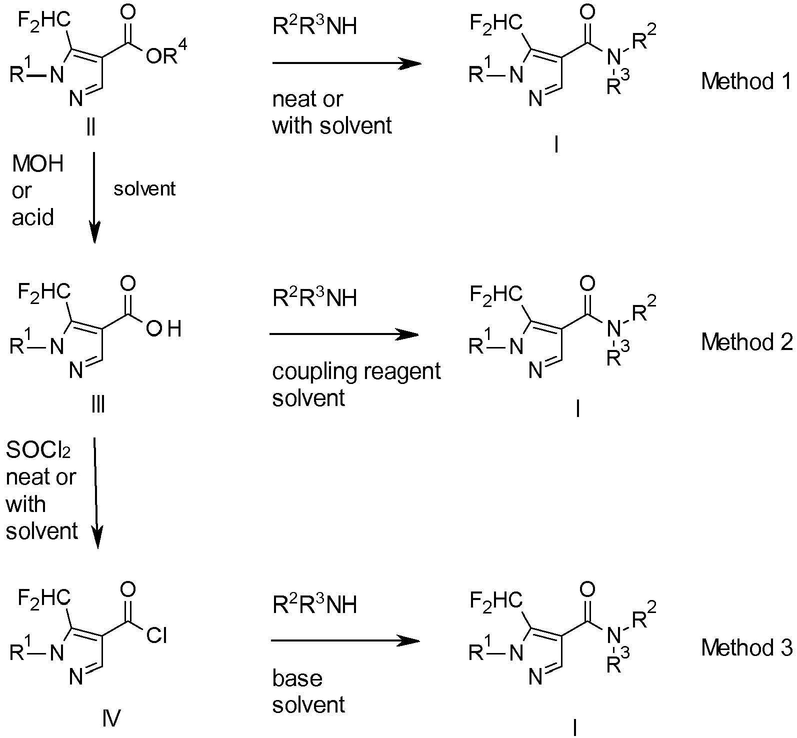

According to method 1, the ester II wherein R¹ has the same meaning as in formula I and R⁴ is an alkyl or optionally substituted phenyl group (preferably C₁-C₄-alkyl, and more preferably methyl, ethyl or t-butyl) is reacted with an amine R²R³NH wherein R¹ and R² have the same meanings as in formula I, optionally in the presence of at least one inert solvent. Any solvent or mixture of solvents that does not react with the ester II can be used, preferably water, methanol, ethanol, isopropanol, tetrahydrofuran, 5-methyltetrahydrofuran, methyl t-butyl ether, methyl cyclopentyl ether, dioxane or toluene, more preferably methanol, ethanol or isopropanol.

According to method 2, the compound of formula I is prepared by hydrolyzing the ester II to the corresponding acid III and reacting the acid III with the amine R²R³NH in the presence of a coupling reagent. In one embodiment, the conversion of the ester II to the acid III can be accomplished by basic hydrolysis. The basic hydrolysis is preferably carried out using an alkali metal hydroxide or tetraalkylammonium hydroxide (MOH, where M is an alkali metal or a tetraalkylammonium ion) in water or water-ethanol mixtures, preferably sodium hydroxide or potassium hydroxide in a water-ethanol mixture. In another embodiment, useful for groups R¹ that are sensitive to strong base, ester hydrolysis is accomplished with (CH₃)₃SnOH (MOH, where M is (CH₃)₃Sn) in 1,2-dichlorethane, as described in Angew. Chem. Int. Ed. 44, 2005, 1378 -1382). This method is for example useful for making acids III having R¹=propargyl or CH₂CH₂X (where X is halogen), since these are partially or wholly converted to acids I with R¹=allenyl or vinyl, respectively, during hydrolysis with alkali metal hydroxides in ethanol-water at reflux. In another embodiment, the conversion of the ester II to the acid III can be accomplished by acid catalysis. Particularly preferred is the reaction of esters II where R⁴ is t-butyl with trifluoroacetic acid, either as solvent or diluted with a co-solvent such as methylene chloride, or with solutions of HCl in dioxane. The coupling of the acid III with R²R³NH is then accomplished with any of the many peptide-coupling reagents known in the art, for example carbonyldiimidazole, dicyclohexylcarbodiimide and the like, in an inert solvent such as methylene chloride, tetrahydrofuran, dioxane or dimethylformamide.

According to method 3, the compound of formula I is prepared by converting the acid III to the corresponding acid chloride IV and reacting the acid chloride IV with R²R³NH in an inert solvent, optionally in the presence of a base. The conversion of the acid III to the acid chloride IV may be accomplished by using thionyl chloride as solvent, optionally with an inert co-solvent, preferably toluene. The reaction of the acid chloride IV with R²R³NH is carried out in an inert solvent such as methylene chloride, chloroform, tetrahydrofuran, 5-methyltetrahydrofuran, methyl t-butyl ether, methyl cyclopentyl ether, dioxane, toluene or the like, optionally with a base as HCl scavenger, preferably triethylamine.

The individual reaction steps of the methods 1 to 3 as outlined above can be conducted at any temperature between 0°C and the boiling point of the solvent used. One skilled in the art will determine the best solvent, temperature and reaction time by monitoring the formation of desired products, and of byproducts if any, by standard methods such as thin-layer, high-pressure liquid or gas chromatography.

The preparation of the pyrazole ester intermediate II as shown above is outlined in following reation scheme:

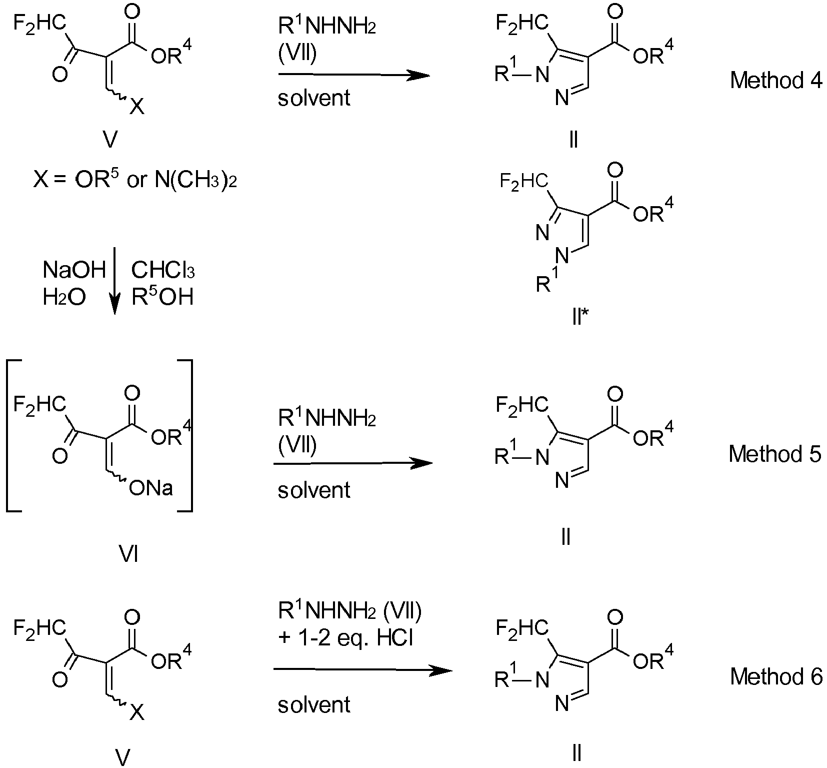

The ester II may be made by reaction of the appropriate precursor V, either as enol ether (X=OR⁵, where R⁵ is C₁-C-₄ alkyl) or dimethylaminovinyl precursor (X = N(CH₃)₂), with the hydrazine VII in an inert solvent (see Method 4 above). The solvent can be, for example, methanol, ethanol, isopropanol, tetrahydrofuran, 5-methyltetrahydrofuran, methyl t-butyl ether, methyl cyclopentyl ether, dioxane or toluene. Preferably R⁴ is ethyl, X is ethoxy, and the solvent is ethanol. When R¹ is sufficiently electron-withdrawing (e.g. CF₃CH₂), Method 4 gives the desired isomer II predominantly or exclusively, with little or no undesired isomer II*.

For groups R¹ that are not electron-withdrawing, one of the Methods 5 and 6 as shown above is preferable to minimize or eliminate formation of undesired isomer II*.

In method 5, the precursor V is first reacted with one equivalent of aqueous sodium hydroxide to give intermediate VI, optionally in the presence of added chloroform. Intermediate VI is then further reacted *in situ with* the hydrazine VII, as described in Japanese Patent 2007/326784.

In method 6, the mono- or bis-HCl salt of the hydrazine VII is reacted with the intermediate V, in this case preferably in the enol ether form (i.e. X= OR⁵), and more preferably with R⁴ and R⁵ both ethyl.

Of methods 5 and 6, method 6 is generally preferred.

Suitable solvents for any of the reaction steps of methods 4 to 6 are methanol, ethanol, methylene chloride, chloroform, tetrahydrofuran, 5-methyltetrahydrofuran, methyl t-butyl ether, methyl cyclopentyl ether, dioxane, toluene or the like, preferably ethanol. These reactions may be conducted at any temperature between -20°C and the boiling point of the solvent used, preferably between -20°C and room temperature. One skilled in the art will determine the best solvent, temperature and reaction time by monitoring the formation of desired products, and of byproducts if any, by standard methods such as thin-layer, high-pressure liquid or gas chromatography.

It is also possible to prepare the esters II by direct alkylation of the unsubstituted pyrazole (R¹ = H) with suitable alkylating agents in the presence of base. In this case mixtures of pyrazole isomers II and II* are generally obtained.

Undesired isomer II*, if present, may be separated from desired isomer II by distillation, crystallization or chromatography.

Intermediates V wherein R⁴ is ethyl and X is either ethoxy or N,N-dimethylamino are both commercially available, and these are suitable for preparation of any compounds of the formula I. They may also be made from the appropriate beta-ketoester by reaction with triethylorthoformate in the presence of acetic anhydride, or with HC(OCH₃)₂N(CH₃)₂ (DMF-acetal) or HC(N(CH₃)₂)₃, respectively.

Some of the hydrazines VII required to prepare the pyrazole ester intermediates II are commercially available. For those hydrazines VII that are not commercially available, several methods are known in the art to prepare them, as shown in the following scheme.

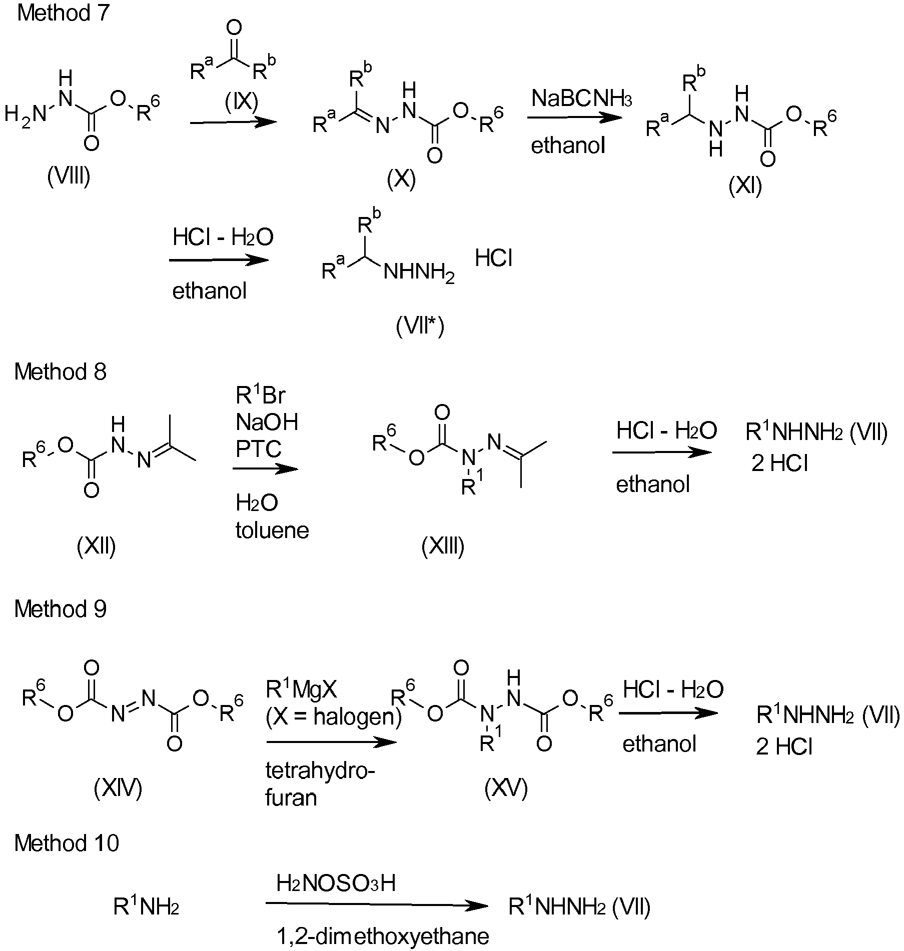

In the compounds VIII, X, XI, XII, XIII, XIV, and XV of the above methods 7 to 9, R⁶ is t-butyl or benzyl, preferably t-butyl. In the compounds IX, X, XI and VII* of method 7, R^{a}R^{b}CH- represents a group R¹ as defined above, including those in which R^{a} is H. For suitable conditions for method 7, see Polish J. Chem 76, 2002, 1693. The imine reduction can also be accomplished with diborane (see J. Org. Chem. 46, 1981, 5413). For suitable conditions for method 8, which involves a phase-transfer catalyzed (PTC) N-alkylation, see Synlett 13, 2004, 2355. For suitable conditions for method 9, see WO 2010/032200. In addition to the acidic deprotection methods shown, it is also possible to deprotect compounds having R⁶= CH₂-C₆H₅ with hydrogen and a catalyst; this can be done directly on the intermediate produced in method 7 or 9, or after removal of the acetone protecting group of the intermediate produced in method 8. For suitable conditions for method 10, see Inorg. Chem. 19, 1980, 2846.

The pyrazole amide compounds of formula I are suitable as herbicides. They are suitable as such or as an appropriately formulated composition (agrochemical composition). As used in this application, the terms "formulated composition" and "herbicidal composition" / "composition" are synonyms.

The herbicidal compositions comprising the compounds of formula I control vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

Depending on the application method in question, the compounds I or compositions comprising them can additionally be employed in a further number of crop plants for eliminating undesirable plants. Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are the following: Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (Sorghum vulgare), Triticale, Triticum aestivum, Triticum durum, Vicia faba, Vitis vinifera and Zea mays.

The compounds of formula I according to the invention can also be used in genetically modified plants. The term "genetically modified plants" is to be understood as plants whose genetic material has been modified by the use of recombinant DNA techniques to include an inserted sequence of DNA that is not native to that plant species' genome or to exhibit a deletion of DNA that was native to that species' genome, wherein the modification(s) cannot readily be obtained by cross breeding, mutagenesis or natural recombination alone. Often, a particular genetically modified plant will be one that has obtained its genetic modification(s) by inheritance through a natural breeding or propagation process from an ancestral plant whose genome was the one directly treated by use of a recombinant DNA technique. Typically, one or more genes have been integrated into the genetic material of a genetically modified plant in order to improve certain properties of the plant. Such genetic modifications also include but are not limited to targeted post-translational modification of protein(s), oligo- or polypeptides. e. g., by inclusion therein of amino acid mutation(s) that permit, decrease, or promote glycosylation or polymer additions such as prenylation, acetylation farnesylation, or PEG moiety attachment.

Plants that have been modified by breeding, mutagenesis or genetic engineering, e.g. have been rendered tolerant to applications of specific classes of herbicides, such as auxin herbicides such as dicamba or 2,4-D; bleacher herbicides such as hydroxyphenylpyruvate dioxygenase (HPPD) inhibitors or phytoene desaturase (PDS) inhibitors; acetolactate synthase (ALS) inhibitors such as sulfonyl ureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by mutgenesis and conventional methods of breeding, e. g., Clearfield® summer rape (Canola, BASF SE, Germany) being tolerant to imidazolinones, e. g., imazamox, or ExpressSun® sunflowers (DuPont, USA) being tolerant to sulfonyl ureas, e. g., tribenuron. Genetic engineering methods have been used to render cultivated plants such as soybean, cotton, corn, beets and rape, tolerant to herbicides such as glyphosate, imidazolinones and glufosinate, some of which are under development or commercially available under the brands or trade names RoundupReady® (glyphosate tolerant, Monsanto, USA), Cultivance® (imidazolinone tolerant, BASF SE, Germany) and LibertyLink® (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as delta-endotoxins, e. g., CrylA(b), CrylA(c), CrylF, CrylF(a2), CrylIA(b), CryIIIA, CryIIIB(b1) or Cry9c; vegetative insecticidal proteins (VIP), e. g., VIP1, VIP2, VIP3 or VIP3A; insecticidal proteins of bacteria colonizing nematodes, e. g., Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as including pre-toxins, hybrid proteins, truncated or otherwise modified proteins. Hybrid proteins are characterized by a new combination of protein domains, (see, e. g., WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g., in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g., Agrisure® CB) and Bt176 from Syngenta Seeds SAS, France, (corn cultivars producing the Cry1Ab toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e.g., EP-A 392 225), plant disease resistance genes (e. g., potato culti-vars, which express resistance genes acting against Phytophthora infestans derived from the Mexican wild potato, Solanum bulbocastanum) or T4-lyso-zym (e.g., potato cultivars capable of synthesizing these proteins with increased resistance against bacteria such as Er-winia amylovora). The methods for producing such genetically modified plants are generally known to the person skilled in the art and are described, e.g., in the publications mentioned above.

Furthermore, plants are also covered that are by the use of recombinant DNA techniques capable to synthesize one or more proteins to increase the productivity (e.g., bio-mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of ingredients or new ingredients, specifically to improve human or animal nutrition, e. g., oil crops that produce health-promoting long-chain omega-3 fatty acids or unsaturated omega-9 fatty acids (e. g., Nexera® oilseed rape, Dow AgroSciences, Canada).

Furthermore, plants are also covered that contain by the use of recombinant DNA techniques a modified amount of ingredients or new ingredients, specifically to improve raw material production, e.g., potatoes that produce increased amounts of amylopectin (e.g. Amflora®, Amadea® and Modena® potatoes, BASF SE, Germany), or are disease-resistant (e.g. Fortuna® potato, BASF SE, Germany).

Furthermore, it has been found that the compounds of formula I are also suitable for the defoliation and/or desiccation of plant parts, for which crop plants such as cotton, potato, oilseed rape, sunflower, soybean or field beans, in particular cotton, are suitable. In this regard, compositions for the desiccation and/or defoliation of plants, processes for preparing these compositions and methods for desiccating and/or defoliating plants using the compounds of the formula I have been found.
As desiccants, the compounds of the formula I are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

The invention also relates to agrochemical compositions comprising an auxiliary and at least one compound I according to the invention.

An agrochemical composition comprises a pesticidally effective amount of a compound I. The term "effective amount" denotes an amount of the composition or of the compounds I, which is sufficient for controlling unwanted plants, especially for controlling unwanted plants in cultivated plants and which does not result in a substantial damage to the treated plants. Such an amount can vary in a broad range and is dependent on various factors, such as the plants to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for agrochemical composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical compositions types are defined in the "Catalogue of pesticide formulation types and international coding system", Technical Monograph No. 2, 6th Ed. May 2008, CropLife International.

The agrochemical compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for agrochemical composition types and their preparation are:
i) Water-soluble concentrates (SL, LS)
   10-60 wt% of a compound of formula I according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound of formula I according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of compound of formula I according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of compound of formula I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound of formula I according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound of formula I according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula I according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound of formula I according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a compound of formula I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a compound of formula I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of a polyurea microcapsules. The monomers amount to 1-10 wt%. The wt% relate to the total CS composition.
ix) Dustable powders (DP, DS)
   1-10 wt% of a compound of formula I according to the invention are ground finely and mixed intimately with solid carrier (e.g. finely divided kaolin) ad 100 wt%.
x) Granules (GR, FG)
   0.5-30 wt% of a compound of formula I according to the invention is ground finely and associated with solid carrier (e.g. silicate) ad 100 wt%. Granulation is achieved by extrusion, spray-drying or the fluidized bed.
xi) Ultra-low volume liquids (UL)
   1-50 wt% of a compound of formula I according to the invention are dissolved in organic solvent (e.g. aromatic hydrocarbon) ad 100 wt%.

The agrochemical compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another preferred embodiment of the invention, the rates of application of the compound of formula I according to the present invention (total amount of the compound of formula I) are from 0.1 g/ha to 3000 g/ha, preferably 10 g/ha to 1000 g/ha of active substance (a.s.), depending on the control target, the season, the target plants and the growth stage.

In another preferred embodiment of the invention, the application rates of the compound of formula I are in the range from 0.1 g/ha to 5000 g/ha and preferably in the range from 1 g/ha to 2500 g/ha or from 5 g/ha to 2000 g/ha of active substance (a.s.).

In another preferred embodiment of the invention, the application rate of the compound of formula I is 0.1 to 1000 g/ha, preferably1 to 750 g/ha, more preferably 5 to 500 g/ha, of active substance.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another preferred embodiment of the invention, to treat the seed, the compounds I are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the agrochemical composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the agrochemical composition according to the invention such as parts of a kit or parts of a binary or ternary mixture may be mixed by the user himself in a spray tank and further auxiliaries may be added, if appropriate.

In a further embodiment, either individual components of the agrochemical composition according to the invention or partially premixed components, e. g. components comprising compounds I and/or active substances from the groups b1) to b15), may be mixed by the user in a spray tank and further auxiliaries and additives may be added, if appropriate.

In a further embodiment, either individual components of the agrochemical composition according to the invention or partially premixed components, e. g. components comprising compounds I and/or active substances from the groups b1) to b15), can be applied jointly (e.g. after tank mix) or consecutively.

The compounds of formula I or the herbicidal compositions comprising them can be applied pre- , post-emergence or pre-plant, or together with the seed of a crop plant. It is also possible to apply the herbicidal composition or active compounds by applying seed, pretreated with the herbicidal compositions or active compounds, of a crop plant. If the active ingredients are less well tolerated by certain crop plants, application techniques may be used in which the herbicidal compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the compounds of formula I or the herbicidal compositions can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula I according to the invention or the compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds.
The seed used can be seed of the useful plants mentioned above, but also the seed of transgenic plants or plants obtained by customary breeding methods.

To widen the spectrum of action and to achieve synergistic effects, the compounds of formula I may be mixed with a large number of representatives of other herbicidal or growth-regulating active ingredient groups and then applied concomitantly. Suitable components for mixtures are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

It may furthermore be beneficial to apply the compounds of formula I alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

Moreover, it may be useful to apply the compounds of formula I in combination with safeners. Safeners are chemical compounds which prevent or reduce damage on useful plants without having a major impact on the herbicidal action of the compounds of the formula I towards unwanted plants. They can be applied either before sowings (e.g. on seed treatments, shoots or seedlings) or in the pre-emergence application or post-emergence application of the useful plant. The safeners and the compounds of formula I can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

In one embodiment of the present invention the compositions according to the present invention comprise at least one compound of formula I and at least one further active compound B (herbicide B).

The further active compound B is preferably selected from the herbicides of class b1) to b15):
b1) lipid biosynthesis inhibitors;
b2) acetolactate synthase inhibitors (ALS inhibitors);
b3) photosynthesis inhibitors;
b4) protoporphyrinogen-IX oxidase inhibitors,
b5) bleacher herbicides;
b6) enolpyruvyl shikimate 3-phosphate synthase inhibitors (EPSP inhibitors);
b7) glutamine synthetase inhibitors;
b8) 7,8-dihydropteroate synthase inhibitors (DHP inhibitors);
b9) mitosis inhibitors;
b10) inhibitors of the synthesis of very long chain fatty acids (VLCFA inhibitors);
b11) cellulose biosynthesis inhibitors;
b12) decoupler herbicides;
b13) auxinic herbicides;
b14) auxin transport inhibitors; and
b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters; including their agriculturally acceptable salts or derivatives.

According to another embodiment of the invention the compositions contain at least one inhibitor of the lipid biosynthesis (herbicide b1). These are compounds that inhibit lipid biosynthesis. Inhibition of the lipid biosynthesis can be affected either through inhibition of acetylCoA carboxylase (hereinafter termed ACC herbicides) or through a different mode of action (hereinafter termed non-ACC herbicides). The ACC herbicides belong to the group A of the HRAC classification system whereas the non-ACC herbicides belong to the group N of the HRAC classification.

According to another embodiment of the invention the compositions contain at least one ALS inhibitor (herbicide b2). The herbicidal activity of these compounds is based on the inhibition of acetolactate synthase and thus on the inhibition of the branched chain amino acid biosynthesis. These inhibitors belong to the group B of the HRAC classification system.

According to another embodiment of the invention the compositions contain at least one inhibitor of photosynthesis (herbicide b3). The herbicidal activity of these compounds is based either on the inhibition of the photosystem II in plants (so-called PSII inhibitors, groups C1, C2 and C3 of HRAC classification) or on diverting the electron transfer in photosystem I in plants (so-called PSI inhibitors, group D of HRAC classification) and thus on an inhibition of photosynthesis. Amongst these, PSII inhibitors are preferred.

According to another embodiment of the invention the compositions contain at least one inhibitor of protoporphyrinogen-IX-oxidase (herbicide b4). The herbicidal activity of these compounds is based on the inhibition of the protoporphyrinogen-IX-oxidase. These inhibitors belong to the group E of the HRAC classification system.

According to another embodiment of the invention the compositions contain at least one bleacher-herbicide (herbicide b5). The herbicidal activity of these compounds is based on the inhibition of the carotenoid biosynthesis. These include compounds which inhibit carotenoid biosynthesis by inhibition of phytoene desaturase (so-called PDS inhibitors, group F1 of HRAC classification), compounds that inhibit the 4-hydroxyphenylpyruvate-dioxygenase (HPPD inhibitors, group F2 of HRAC classification), compounds that inhibit DOXsynthase (group F4 of HRAC class) and compounds which inhibit carotenoid biosynthesis by an unknown mode of action (bleacher - unknown target, group F3 of HRAC classification).

According to another embodiment of the invention the compositions contain at least one EPSP synthase inhibitor (herbicide b6). The herbicidal activity of these compounds is based on the inhibition of enolpyruvyl shikimate 3-phosphate synthase, and thus on the inhibition of the amino acid biosynthesis in plants. These inhibitors belong to the group G of the HRAC classification system.

According to another embodiment of the invention the compositions contain at least one glutamine synthetase inhibitor (herbicide b7). The herbicidal activity of these compounds is based on the inhibition of glutamine synthetase, and thus on the inhibition of the aminoacid biosynthesis in plants. These inhibitors belong to the group H of the HRAC classification system.

According to another embodiment of the invention the compositions contain at least one DHP synthase inhibitor (herbicide b8). The herbicidal activity of these compounds is based on the inhibition of 7,8-dihydropteroate synthase. These inhibitors belong to the group I of the HRAC classification system.

According to another embodiment of the invention the compositions contain at least one mitosis inhibitor (herbicide b9). The herbicidal activity of these compounds is based on the disturbance or inhibition of microtubule formation or organization, and thus on the inhibition of mitosis. These inhibitors belong to the groups K1 and K2 of the HRAC classification system. Among these, compounds of the group K1, in particular dinitroanilines, are preferred.

According to another embodiment of the invention the compositions contain at least one VLCFA inhibitor (herbicide b10). The herbicidal activity of these compounds is based on the inhibition of the synthesis of very long chain fatty acids and thus on the disturbance or inhibition of cell division in plants. These inhibitors belong to the group K3 of the HRAC classification system.

According to another embodiment of the invention the compositions contain at least one cellulose biosynthesis inhibitor (herbicide b11). The herbicidal activity of these compounds is based on the inhibition of the biosynthesis of cellulose and thus on the inhibition of the synthesis of cell walls in plants. These inhibitors belong to the group L of the HRAC classification system.

According to another embodiment of the invention the compositions contain at least one decoupler herbicide (herbicide b12). The herbicidal activity of these compounds is based on the disruption of the cell membrane. These inhibitors belong to the group M of the HRAC classification system.

According to another embodiment of the invention the compositions contain at least one auxinic herbicide (herbicide b13). These include compounds that mimic auxins, i.e. plant hormones, and affect the growth of the plants. These compounds belong to the group O of the HRAC classification system.

According to another embodiment of the invention the compositions contain at least one auxin transport inhibitor (herbicide b14). The herbicidal activity of these compounds is based on the inhibition of the auxin transport in plants. These compounds belong to the group P of the HRAC classification system.

As to the given mechanisms of action and classification of the active substances, see e.g. "HRAC, Classification of Herbicides According to Mode of Action", http://www.plantprotection.org/hrac/MOA.html).

Preference is given to those compositions according to the present invention comprising at least one herbicide B selected from herbicides of class b2, b3, b4, b5, b6, b9 and b10.

Specific preference is given to those compositions according to the present invention which comprise at least one herbicide B selected from the herbicides of class b4, b6 b9 and b10. Particular preference is given to those compositions according to the present invention which comprise at least one herbicide B selected from the herbicides of class b4, b6 and b10.

Examples of herbicides B which can be used in combination with the compounds of formula I according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro-4-cyclopropyl-[1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
   imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam,
   pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
   sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
   among these, a preferred embodiment of the invention relates to those compositions comprising at least one imidazolinone herbicide;
b3) from the group of the photosynthesis inhibitors:
   amicarbazone, inhibitors of the photosystem II, e.g. triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H* pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 45100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione, 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, 2-(2,2,7-Trifluoro-3-oxo-4-prop-2-ynyl-3,4-dihydro-2H-benzo[1,4]oxazin-6-yl)-4,5,6,7-tetrahydro-isoindole-1,3-dione, 1-Methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione and (Z)-4-[2-Chloro-5-(4-chloro-5-difluoromethoxy-1-methyl-1H-pyrazol-3-yl)-4-fluoro-phenoxy]-3-methyl-but-2-enoic acid methyl ester;
b5) from the group of the bleacher herbicides:
   PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, clomazone, isoxaflutole, mesotrione, pyrasulfotole, pyrazolynate, pyrazoxyfen, sulcotrione, tefuryltrione, tembotrione, topramezone and bicyclopyrone, bleacher, unknown target: aclonifen, amitrole and flumeturon;
b6) from the group of the EPSP synthase inhibitors:
   glyphosate, glyphosate-isopropylammonium, glyposate-potassium and glyphosate-trimesium (sulfosate);
b7) from the group of the glutamine synthase inhibitors:
   bilanaphos (bialaphos), bilanaphos-sodium, glufosinate, glufosinate-P and glufosinate-ammonium;
b8) from the group of the DHP synthase inhibitors:
   asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: chlorpropham, propham and carbetamide, among these, compounds of group K1, in particular dinitroanilines are preferred;
b10) from the group of the VLCFA inhibitors:
   chloroacetamides such as acetochlor, alachlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide and napropamide, tetrazolinones such fentrazamide, and other herbicides such as anilofos, cafenstrole, fenoxasulfone, ipfencarbazone, piperophos, pyroxasulfone and isoxazoline compounds of the formulae II.1, II.2, II.3, II.4, II.5, II.6, II.7, II.8 and II.9 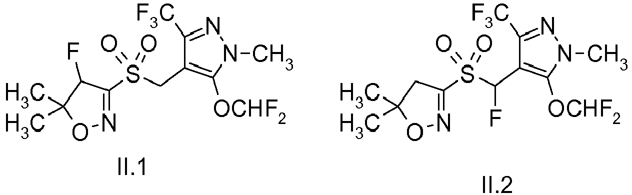 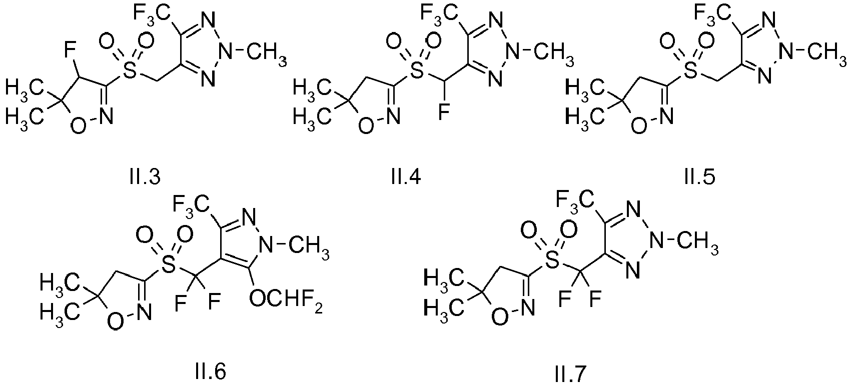 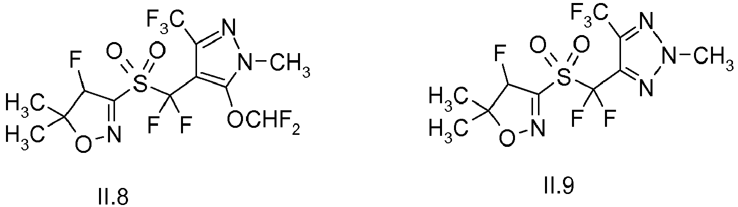 the isoxazoline compounds of the formula II are known in the art, e.g. from WO 2006/024820, WO 2006/037945, WO 2007/071900 and WO 2007/096576;
      among the VLCFA inhibitors, preference is given to chloroacetamides and oxyacetamides;
b11) from the group of the cellulose biosynthesis inhibitors:
   chlorthiamid, dichlobenil, flupoxam, isoxaben and 1-Cyclohexyl-5-pentafluorphenyloxy-1⁴-[1,2,4,6]thiatriazin-3-ylamine;
b12) from the group of the decoupler herbicides:
   dinoseb, dinoterb and DNOC and its salts;
b13) from the group of the auxinic herbicides:
   2,4-D and its salts and esters, 2,4-DB and its salts and esters, aminopyralid and its salts such as aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, aminocyclopyrachlor and its salts and esters, and halauxifen (CAS 943832-60-8) and its salts and esters;
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, triaziflam, tridiphane, 6-chloro-3-(2-cyclopropyl-6-methylphenoxy)-4-pyridazinol (CAS 499223-49-3) and its salts and esters, and cyclopyrimorate (CAS 499231-24-2).

Preferred herbicides B that can be used in combination with the compounds of the formula I according to the present invention are:
b1) from the group of the lipid biosynthesis inhibitors:
   clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro-4-cyclopropyl-[1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, dimepiperate, EPTC, esprocarb, ethofumesate, molinate, orbencarb, prosulfocarb, thiobencarb and triallate;
b2) from the group of the ALS inhibitors:
   amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisulfuron, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl, tritosulfuron and triafamone;
b3) from the group of the photosynthesis inhibitors:
   ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine and thidiazuron;
b4) from the group of the protoporphyrinogen-IX oxidase inhibitors:
   acifluorfen-sodium, bencarbazone, benzfendizone, butafenacil, carfentrazone-ethyl, cinidon-ethyl, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen-ethyl, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 45100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione, 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, 2-(2,2,7-Trifluoro-3-oxo-4-prop-2-ynyl-3,4-dihydro-2H-benzo[1,4]oxazin-6-yl)-4,5,6,7-tetrahydro-isoindole-1,3-dione and 1-Methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione;
b5) from the group of the bleacher herbicides:
   aclonifen, beflubutamid, benzobicyclon, clomazone, diflufenican, flurochloridone, flurtamone, isoxaflutole, mesotrione, norflurazon, picolinafen, pyrasulfotole, pyrazolynate, sulcotrione, tefuryltrione, tembotrione, topramezone, bicyclopyrone, 4-(3-trifluoromethylphenoxy)-2-(4-trifluoromethylphenyl)pyrimidine (CAS 180608-33-7), amitrole and flumeturon;
b6) from the group of the EPSP synthase inhibitors:
   glyphosate, glyphosate-isopropylammonium, glyphosate-potassium and glyphosate-trimesium (sulfosate);
b7) from the group of the glutamine synthase inhibitors:
   glufosinate, glufosinate-P, glufosinate-ammonium;
b8) from the group of the DHP synthase inhibitors: asulam;
b9) from the group of the mitosis inhibitors:
   benfluralin, dithiopyr, ethalfluralin, oryzalin, pendimethalin, thiazopyr and trifluralin;
b10) from the group of the VLCFA inhibitors:
   acetochlor, alachlor, anilofos, butachlor, cafenstrole, dimethenamid, dimethenamid-P, fentrazamide, flufenacet, mefenacet, metazachlor, metolachlor, S-metolachlor, naproanilide, napropamide, pretilachlor, fenoxasulfone, ipfencarbazone, pyroxasulfone thenylchlor and isoxazoline-compounds of the formulae II.1, II.2, II.3, II.4, II.5, II.6, II.7, II.8 and II.9 as mentioned above;
b11) from the group of the cellulose biosynthesis inhibitors: dichlobenil, flupoxam, isoxaben and 1-Cyclohexyl-5-pentafluorphenyloxy-1⁴-[1,2,4,6]thiatriazin-3-ylamine;
b13) from the group of the auxinic herbicides:
   2,4-D and its salts and esters, aminopyralid and its salts such as aminopyralid-tris(2-hydroxypropyl)ammonium and its esters, clopyralid and its salts and esters, dicamba and its salts and esters, dichlorprop-P and its salts and esters, fluroxypyr-meptyl, MCPA and its salts and esters, MCPB and its salts and esters, mecoprop-P and its salts and esters, picloram and its salts and esters, quinclorac, quinmerac, triclopyr and its salts and esters, aminocyclopyrachlor and its salts and esters, and halauxifen and its salts and esters;
b14) from the group of the auxin transport inhibitors: diflufenzopyr and diflufenzopyr-sodium;
b15) from the group of the other herbicides: bromobutide, cinmethylin, cumyluron, dalapon, difenzoquat, difenzoquat-metilsulfate, DSMA, dymron (= daimuron), flamprop, flamprop-isopropyl, flamprop-methyl, flamprop-M-isopropyl, flamprop-M-methyl, indanofan, indaziflam, metam, methylbromide, MSMA, oxaziclomefone, pyributicarb, triaziflam, tridiphane and cyclopyrimorate (CAS 499231-24-2).

Particularly preferred herbicides B that can be used in combination with the compounds of the formula I according to the present invention are:
b1) from the group of the lipid biosynthesis inhibitors: clodinafop-propargyl, cycloxydim, cyhalofop-butyl, fenoxaprop-P-ethyl, pinoxaden, profoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro-4-cyclopropyl-[1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); esprocarb, prosulfocarb, thiobencarb and triallate;
b2) from the group of the ALS inhibitors: bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, nicosulfuron, penoxsulam, propoxycarbazon-sodium, propyrisulfuron, pyrazosulfuron-ethyl, pyroxsulam, rimsulfuron, sulfosulfuron, thiencarbazon-methyl, tritosulfuron and triafamone;
b3) from the group of the photosynthesis inhibitors: ametryn, atrazine, diuron, fluometuron, hexazinone, isoproturon, linuron, metribuzin, paraquat, paraquat-dichloride, propanil, terbutryn and terbuthylazine;
b4) from the group of the protoporphyrinogen-IX oxidase inhibitors: flumioxazin, oxyfluorfen, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione, 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione and 2-(2,2,7-Trifluoro-3-oxo-4-prop-2-ynyl-3,4-dihydro-2H-benzo[1,4]oxazin-6-yl)-4,5,6,7-tetrahydro-isoindole-1,3-dione, and 1-Methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione;
b5) from the group of the bleacher herbicides: clomazone, diflufenican, flurochloridone, isoxaflutole, mesotrione, picolinafen, sulcotrione, tefuryltrione, tembotrione, topramezone, bicyclopyrone, amitrole and flumeturon;
b6) from the group of the EPSP synthase inhibitors: glyphosate, glyphosate-isopropylammonium and glyphosate-trimesium (sulfosate);
b7) from the group of the glutamine synthase inhibitors: glufosinate, glufosinate-P and glufosinate-ammonium;
b9) from the group of the mitosis inhibitors: pendimethalin and trifluralin;
b10) from the group of the VLCFA inhibitors: acetochlor, cafenstrole, dimethenamid-P, fentrazamide, flufenacet, mefenacet, metazachlor, metolachlor, S-metolachlor, fenoxasulfone, ipfencarbazone and pyroxasulfone; likewise, preference is given to isoxazoline compounds of the formulae II.1, II.2, II.3, II.4, II.5, II.6, II.7, II.8 and II.9 as mentioned above;
b11) from the group of the cellulose biosynthesis inhibitors: isoxaben;
b13) from the group of the auxinic herbicides: 2,4-D and its salts and esters, aminopyralid and its salts and its esters, clopyralid and its salts and esters, dicamba and its salts and esters, fluroxypyr-meptyl, quinclorac, aminocyclopyrachlor and its salts and esters, and halauxifen and its salts and esters;
b14) from the group of the auxin transport inhibitors: diflufenzopyr and diflufenzopyr-sodium,
b15) from the group of the other herbicides: dymron (= daimuron), indanofan, indaziflam, oxaziclomefone and triaziflam.

In another embodiment of the present invention the compositions according to the present invention comprise at least one compound of formula I and at least one safener C.

Safeners are chemical compounds which prevent or reduce damage on useful plants without having a major impact on the herbicidal action of the herbicidal active components of the present compositions towards unwanted plants. They can be applied either before sowings (e.g. on seed treatments, shoots or seedlings) or in the pre-emergence application or post-emergence application of the useful plant. The safeners and the compounds of formula I and/or the herbicides B can be applied simultaneously or in succession.

Examples of preferred safeners C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Especially preferred safeners C are benoxacor, cloquintocet, cyprosulfamide, dichlormid, fenchlorazole, fenclorim, flurazole, fluxofenim, furilazole, isoxadifen, mefenpyr, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C are benoxacor, cloquintocet, cyprosulfamide, dichlormid, fenchlorazole, fenclorim, furilazole, isoxadifen, mefenpyr, naphtalic anhydride, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

If the herbicides B and/or the safeners C are capable of forming geometrical isomers, for example E/Z isomers, both the pure isomers and mixtures thereof may be used in the compositions according to the invention.
If the herbicides B and/or the safeners C have one of more centers of chirality and are thus present as enantiomers or diastereomers, both the pure enantiomers and diastereomers and mixtures thereof may be used in the compositions according to the invention.

If the herbicides B and/or the safeners C have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

Active compounds B and C having a carboxyl group can be employed in the form of the acid, in the form of an agriculturally suitable salt as mentioned above or else in the form of an agriculturally acceptable derivative in the compositions according to the invention, for example as amides, such as mono- and di-C₁-C₆-alkylamides or arylamides, as esters, for example as allyl esters, propargyl esters, C₁-C₁₀-alkyl esters, alkoxyalkyl esters, tefuryl ((tetrahydrofuran-2-yl)methyl) esters and also as thioesters, for example as C₁-C₁₀-alkylthio esters. Preferred mono- and di-C₁-C₆-alkylamides are the methyl and the dimethylamides. Preferred arylamides are, for example, the anilides and the 2-chloroanilides. Preferred alkyl esters are, for example, the methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄-alkoxy-C₁-C₄-alkyl esters are the straight-chain or branched C₁-C₄-alkoxy ethyl esters, for example the 2-methoxyethyl, 2-ethoxyethyl, 2-butoxyethyl (butotyl), 2-butoxypropyl or 3-butoxypropyl ester. An example of a straight-chain or branched C₁-C₁₀-alkylthio ester is the ethylthio ester.

In the case of dicamba, suitable salts include those, where the counterion is an agriculturally acceptable cation. For example, suitable salts of dicamba are dicamba-sodium, dicambapotassium, dicamba-methylammonium, dicamba-dimethylammonium, dicambaisopropylammonium, dicamba-diglycolamine, dicamba-olamine, dicamba-diolamine, dicambatrolamine, dicamba-N,N-bis-(3-aminopropyl)methylamine and dicamba-diethylenetriamine. Examples of a suitable ester are dicamba-methyl and dicamba-butotyl.
Suitable salts of 2,4-D are 2,4-D-ammonium, 2,4-D-dimethylammonium, 2,4-D-diethylammonium, 2,4-D-diethanolammonium (2,4-D-diolamine), 2,4-D-triethanolammonium, 2,4-D-isopropylammonium, 2,4-D-triisopropanolammonium, 2,4-D-heptylammonium, 2,4-D-dodecylammonium, 2,4-D-tetradecylammonium, 2,4-D-triethylammonium, 2,4-D-tris(2-hydroxypropyl)ammonium, 2,4-D-tris(isopropyl)ammonium, 2,4-D-trolamine, 2,4-D-lithium, 2,4-D-sodium. Examples of suitable esters of 2,4-D are 2,4-D-butotyl, 2,4-D-2-butoxypropyl, 2,4-D-3-butoxypropyl, 2,4-D-butyl, 2,4-D-ethyl, 2,4-D-ethylhexyl, 2,4-D-isobutyl, 2,4-D-isooctyl, 2,4-D-isopropyl, 2,4-D-meptyl, 2,4-D-methyl, 2,4-D-octyl, 2,4-D-pentyl, 2,4-D-propyl, 2,4-D-tefuryl. Suitable salts of 2,4-DB are for example 2,4-DB-sodium, 2,4-DB-potassium and 2,4-DB-dimethylammonium. Suitable esters of 2,4-DB are for example 2,4-DB-butyl and 2,4-DB-isoctyl. Suitable salts of dichlorprop are for example dichlorprop-potassium and dichlorpropdimethylammonium. Examples of suitable esters of dichlorprop are dichlorprop-butotyl and dichlorprop-isoctyl.
Suitable salts and esters of MCPA include MCPA-butotyl, MCPA-butyl, MCPA-dimethylammonium, MCPA-diolamine, MCPA-ethyl, MCPA-thioethyl, MCPA-2-ethylhexyl, MCPA-isobutyl, MCPA-isoctyl, MCPA-isopropyl, MCPA-isopropylammonium, MCPA-methyl, MCPA-olamine, MCPA-potassium, MCPA-sodium and MCPA-trolamine.
A suitable salt of MCPB is MCPB sodium. A suitable ester of MCPB is MCPB-ethyl.
Suitable salts of clopyralid are clopyralid-potassium, clopyralid-olamine and clopyralid-tris-(2-hydroxypropyl)ammonium. Example of suitable esters of clopyralid is clopyralid-methyl. Examples of a suitable ester of fluroxypyr are fluroxypyr-meptyl and fluroxypyr-2-butoxy-1-methylethyl, wherein fluroxypyr-meptyl is preferred.
Suitable salts of picloram are picloram-dimethylammonium, picloram-potassium, picloram-triisopropanolammonium, picloram-triisopropylammonium and picloram-trolamine. A suitable ester of picloram is picloram-isoctyl.
A suitable salt of triclopyr is triclopyr-triethylammonium. Suitable esters of triclopyr are for example triclopyr-ethyl and triclopyr-butotyl.
Suitable salts and esters of chloramben include chloramben-ammonium, chloramben-diolamine, chloramben-methyl, chloramben-methylammonium and chloramben-sodium. Suitable salts and esters of 2,3,6-TBA include 2,3,6-TBA-dimethylammonium, 2,3,6-TBA-lithium, 2,3,6-TBA-potassium and 2,3,6-TBA-sodium.
Suitable salts and esters of aminopyralid include aminopyralid-potassium and aminopyralid-tris(2-hydroxypropyl)ammonium.
Suitable salts of glyphosate are for example glyphosate-ammonium, glyphosate-diammonium, glyphoste-dimethylammonium, glyphosate-isopropylammonium, glyphosate-potassium, glyphosate-sodium, glyphosate-trimesium as well as the ethanolamine and diethanolamine salts, preferably glyphosate-diammonium, glyphosate-isopropylammonium and glyphosate-trimesium (sulfosate).
A suitable salt of glufosinate is for example glufosinate-ammonium.
A suitable salt of glufosinate-P is for example glufosinate-P-ammonium.
Suitable salts and esters of bromoxynil are for example bromoxynil-butyrate, bromoxynil-heptanoate, bromoxynil-octanoate, bromoxynil-potassium and bromoxynil-sodium.
Suitable salts and esters of ioxonil are for example ioxonil-octanoate, ioxonil-potassium and ioxonil-sodium.
Suitable salts and esters of mecoprop include mecoprop-butotyl, mecoprop-dimethylammonium, mecoprop-diolamine, mecoprop-ethadyl, mecoprop-2-ethylhexyl, mecoprop-isoctyl, mecoprop-methyl, mecoprop-potassium, mecoprop-sodium and mecoprop-trolamine.
Suitable salts of mecoprop-P are for example mecoprop-P-butotyl, mecoprop-P-dimethylammonium, mecoprop-P-2-ethylhexyl, mecoprop-P-isobutyl, mecoprop-P-potassium and mecoprop-P-sodium.
A suitable salt of diflufenzopyr is for example diflufenzopyr-sodium.
A suitable salt of naptalam is for example naptalam-sodium.
Suitable salts and esters of aminocyclopyrachlor are for example aminocyclopyrachlor-dimethylammonium, aminocyclopyrachlor-methyl, aminocyclopyrachlor-triisopropanolammonium, aminocyclopyrachlor-sodium and aminocyclopyrachlor-potassium.
A suitable salt of quinclorac is for example quinclorac-dimethylammonium.
A suitable salt of quinmerac is for example quinclorac-dimethylammonium.
A suitable salt of imazamox is for example imazamox-ammonium.
Suitable salts of imazapic are for example imazapic-ammonium and imazapic-isopropylammonium.
Suitable salts of imazapyr are for example imazapyr-ammonium and imazapyr-isopropylammonium.
A suitable salt of imazaquin is for example imazaquin-ammonium.
Suitable salts of imazethapyr are for example imazethapyr-ammonium and imazethapyr-isopropylammonium.
A suitable salt of topramezone is for example topramezone-sodium.

According to a preferred embodiment of the invention, the composition comprises as herbicidal active compound B or component B, at least one, preferably exactly one herbicide B.

According to another preferred embodiment of the invention, the composition comprises as herbicidal active compound B or component B, at least two, preferably exactly two herbicides B different from each other.

According to another preferred embodiment of the invention, the composition comprises as herbicidal active compound B or component B, at least three, preferably exactly three herbicides B different from each other.

According to another preferred embodiment of the invention, the composition comprises as safening component C at least one, preferably exactly one safener C.

According to another preferred embodiment of the invention, the composition comprises as component B, at least one, preferably exactly one herbicide B, and at lest one, preferably exactly one, safener C.

According to another preferred embodiment of the invention, the composition comprises as component B, preferably exactly two herbicides B different from each other, and at lest one, preferably exactly one, safener C.

According to another preferred embodiment of the invention, the composition comprises as component B, at least three, preferably exactly three herbicides B different from each other, and at lest one, preferably exactly one, safener C.

According to another preferred embodiment of the invention, the composition comprises as component A at least one, preferably exactly one compound of formula I, preferably of the formula I.1, and as component B, at least one, preferably exactly one, herbicide B.

According to another preferred embodiment of the invention, the composition comprises as component A at least one, preferably exactly one compound of formula I, preferably of the formula I.1, and as component B, at least two, preferably exactly two, herbicides B different from each other.

According to another preferred embodiment of the invention, the composition comprises as component A at least one, preferably exactly one compound of formula I, preferably of the formula I.1, and as component B, at least three, preferably exactly three herbicides, B different from each other.

According to another preferred embodiment of the invention, the composition comprises as active componets at least one, preferably exactly one compound of formula I, preferably of the formula I.1, and at least one, preferably exactly one, herbicide B.

According to another preferred embodiment of the invention, the composition comprises as active components at least one, preferably exactly one compound of formula I, preferably of formula I.1, and at least two, preferably exactly two, herbicides B different from each other.

According to another preferred embodiment of the invention, the composition comprises as active components at least one, preferably exactly one compound of formula I, preferably of formula I.1, and at least three, preferably exactly three, herbicides B different from each other.

According to another preferred embodiment of the invention, the composition comprises as active componets at least one, preferably exactly one compound of formula I, preferably of formula I.1, and at least one, preferably exactly one, safener C.

According to another preferred embodiment of the invention, the composition comprises as active componets at least one, preferably exactly one compound of formula I, preferably of formula I.1, at least one, preferably exactly one, herbicide B, and at least one, preferably exactly one safener C.

According to another preferred embodiment of the invention, the composition comprises as active componets at least one, preferably exactly one compound of formula I, preferably of formula I.1, at least two, preferably exactly two herbicides B different from each other, and at least one, preferably exactly one, safener C.

According to another preferred embodiment of the invention, the composition comprises as active componets at least one, preferably exactly one compound of formula I, preferably of formula I.1, at least three, preferably exactly three herbicides B different from each other, and at least one, preferably exactly one, safener C.

According to another preferred embodiment of the invention, the composition comprises, in addition to a compound of formula I, especially an active compound of the formula I.1, at least one and especially exactly one herbicidally active compound from group b1), in particular selected from the group consisting of clodinafop-propargyl, cycloxydim, cyhalofop-butyl, fenoxaprop-P-ethyl, pinoxaden, profoxydim, tepraloxydim, tralkoxydim, esprocarb, prosulfocarb, thiobencarb and triallate.

According to another preferred embodiment of the invention, the composition comprises, in addition to a compound of formula I, especially an active compound of the formula I.1, at least one and especially exactly one herbicidally active compound from group b2), in particular selected from the group consisting of bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, mesosulfuron, metazosulfuron, nicosulfuron, penoxsulam, propoxycarbazon-sodium, pyrazosulfuron-ethyl, pyroxsulam, rimsulfuron, sulfosulfuron, thiencarbazon-methyl and tritosulfuron.

According to another preferred embodiment of the invention, the composition comprises, in addition to a compound of formula I, especially an active compound of the formula I.1, at least one and especially exactly one herbicidally active compound from group b3), in particular selected from the group consisting of ametryn, atrazine, diuron, fluometuron, hexazinone, isoproturon, linuron, metribuzin, paraquat, paraquat-dichloride, propanil, terbutryn and terbuthylazine.

According to another preferred embodiment of the invention, the composition comprises, in addition to a compound of formula I, especially an active compound of the formula I.1, at least one and especially exactly one herbicidally active compound from group b4), in particular selected from the group consisting of flumioxazin, oxyfluorfen, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)-phenoxy]-2-pyridyloxy]acetate (CAS 353292-31-6; S-3100), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione, 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, 2-(2,2,7-Trifluoro-3-oxo-4-prop-2-ynyl-3,4-dihydro-2H-benzo[1,4]oxazin-6-yl)-4,5,6,7-tetrahydro-isoindole-1,3-dione and 1-Methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione.

According to another preferred embodiment of the invention, the composition comprises, in addition to a compound of formula I, especially an active compound of the formula I.1, at least one and especially exactly one herbicidally active compound from group b5), in particular selected from the group consisting of clomazone, diflufenican, flurochloridone, isoxaflutole, mesotrione, picolinafen, sulcotrione, tefuryltrione, tembotrione, topramezone, bicyclopyrone, amitrole and flumeturon.

According to another preferred embodiment of the invention, the composition comprises, in addition to a compound of formula I, especially an active compound of the formula I.1, at least one and especially exactly one herbicidally active compound from group b6), in particular selected from the group consisting of glyphosate, glyphosate-isopropylammonium and glyphosate-trimesium (sulfosate).

According to another preferred embodiment of the invention, the composition comprises, in addition to a compound of formula I, especially an active compound of the formula I.1, at least one and especially exactly one herbicidally active compound from group b7), in particular selected from the group consisting of glufosinate, glufosinate-P and glufosinate-ammonium.

According to another preferred embodiment of the invention, the composition comprises, in addition to a compound of formula I, especially an active compound of the formula I.1, at least one and especially exactly one herbicidally active compound from group b9), in particular selected from the group consisting of pendimethalin and trifluralin.

According to another preferred embodiment of the invention, the composition comprises, in addition to a compound of formula I, especially an active compound of the formula I.1, at least one and especially exactly one herbicidally active compound from group b10), in particular selected from the group consisting of acetochlor, cafenstrole, dimethenamid-P, fentrazamide, flufenacet, mefenacet, metazachlor, metolachlor, S-metolachlor, fenoxasulfone and pyroxasulfone. Likewise, preference is given to compositions comprising in addition to a compounds of formula I, especially an active compound of the formula I.1, at least one and especially exactly one herbicidally active compound from group b10), in particular selected from the group consisting of isoxazoline compounds of the formulae II.1, II.2, II.3, II.4, II.5, II.6, II.7, II.8 and II.9, as defined above.

According to another preferred embodiment of the invention, the composition comprises, in addition to a compound of formula I, especially an active compound of the formula I.1, at least one and especially exactly one herbicidally active compound from group b11), in particular isoxaben. Likewise, preference is given to compositions comprising in addition to a compound of formula I, especially an active compound of the formula I.1, at least one and especially exactly one herbicidally active compound from group b11), in particular selected from the group consisting of piperazine compounds of formula III as defined above.

According to another preferred embodiment of the invention, the composition comprises, in addition to a compound of formula I, especially an active compound of the formula I.1, at least one and especially exactly one herbicidally active compound from group b13), in particular selected from the group consisting of 2,4-D and its salts and esters, aminopyralid and its salts such as aminopyralid-tris(2-hydroxypropyl)ammonium and its esters, clopyralid and its salts and esters, dicamba and its salts and esters, fluroxypyr-meptyl, quinclorac, quinmerac and aminocyclopyrachlor and its salts and esters.

According to another preferred embodiment of the invention, the composition comprises, in addition to a compound of formula I, especially an active compound of the formula I.1, at least one and especially exactly one herbicidally active compound from group b14), in particular selected from the group consisting of diflufenzopyr and diflufenzopyr-sodium.

According to another preferred embodiment of the invention, the composition comprises, in addition to a compound of formula I, especially an active compound of the formula I.1, at least one and especially exactly one herbicidally active compound from group b15), in particular selected from the group consisting of dymron (= daimuron), indanofan, indaziflam, oxaziclomefone and triaziflam.

According to another preferred embodiment of the invention, the composition comprises, in addition to a compound of formula I, especially an active compound of the formula I.1, at least one and especially exactly one herbicidally active compound from the safeners C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Further preferred embodiments relate to ternary compositions which correspond to the binary compositions mentioned above and additionally comprise a safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Here and below, the term "binary compositions" includes compositions comprising one or more, for example 1, 2 or 3, active compounds of the formula I and either one or more, for example 1, 2 or 3, herbicides B or one or more safeners. Correspondingly, the term "ternary compositions" includes compositions comprising one or more, for example 1, 2 or 3, active compounds of the formula I, one or more, for example 1, 2 or 3, herbicides B and one or more, for example 1, 2 or 3, safeners C.

In binary compositions comprising at least one compound of the formula I as component A and at least one herbicide B, the weight ratio of the active compounds A:B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising at least one compound of the formula I as component A and at least one safener C, the weight ratio of the active compounds A:C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising both at least one compound of formula I as component A, at least one herbicide B and at least one safener C, the relative proportions by weight of the components A:B are generally in the range of from 1:1000 to 1000:1, preferably in the range of from 1:500 to 500:1, in particular in the range of from 1:250 to 250:1 and particularly preferably in the range of from 1:75 to 75:1, the weight ratio of the components A:C is generally in the range of from 1:1000 to 1000:1, preferably in the range of from 1:500 to 500:1, in particular in the range of from 1:250 to 250:1 and particularly preferably in the range of from 1:75 to 75:1, and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1. The weight ratio of components A + B to component C is preferably in the range of from 1:500 to 500:1, in particular in the range of from 1:250 to 250:1 and particularly preferably in the range of from 1:75 to 75:1.
Particularly preferred herbicides B are the herbicides B as defined above; in particular the herbicides B.1 - B.187 listed below in table B:

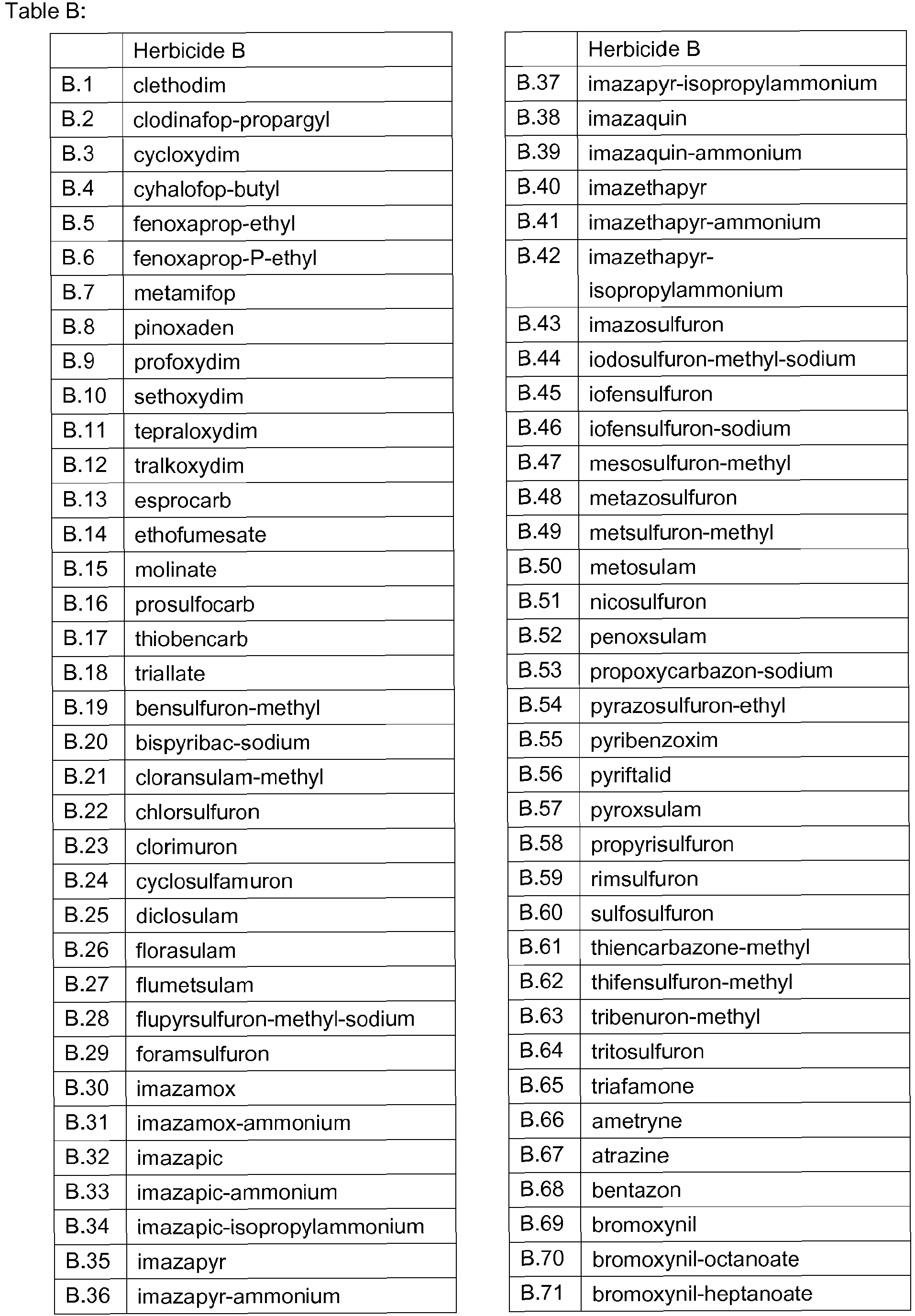

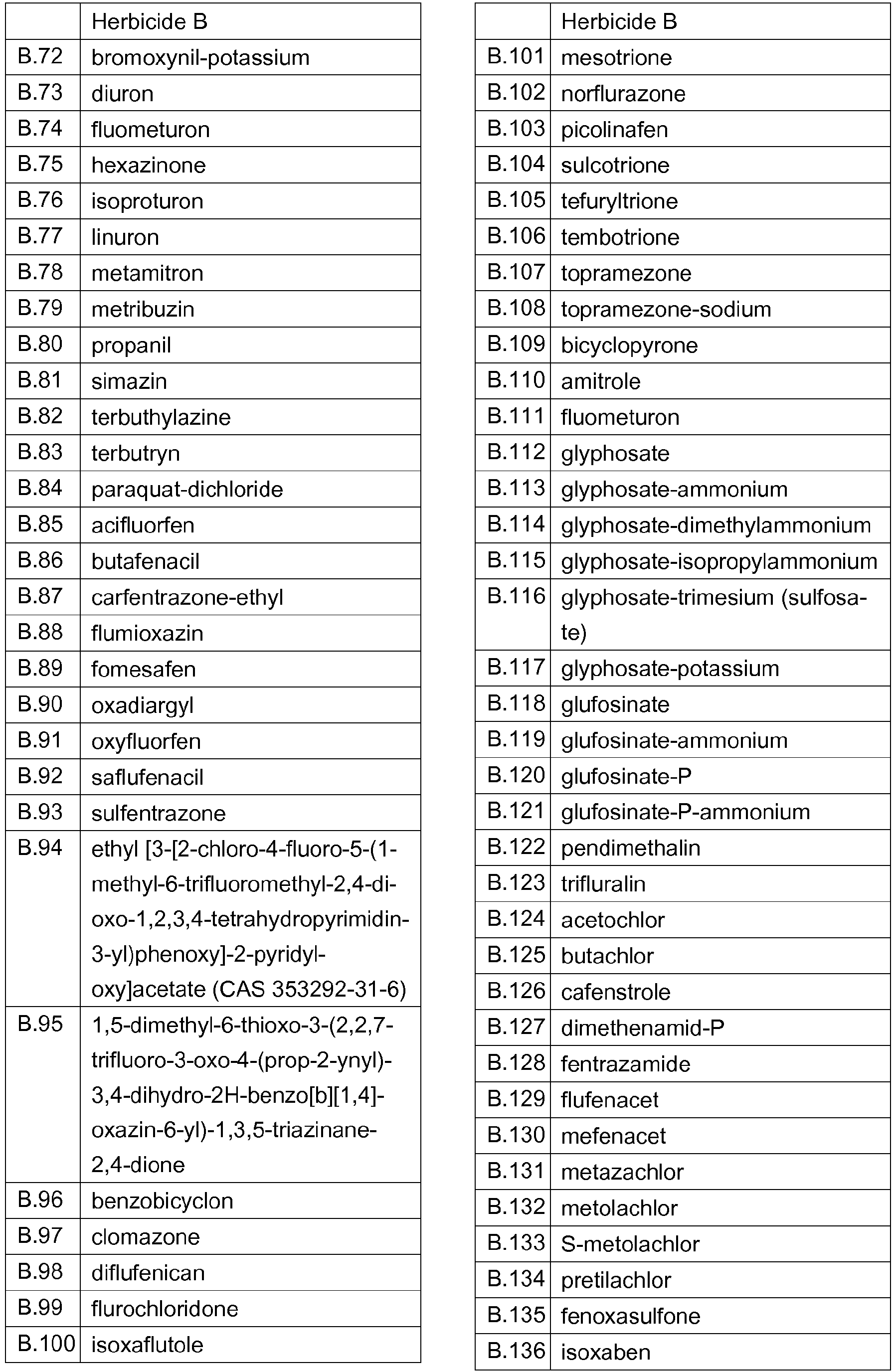

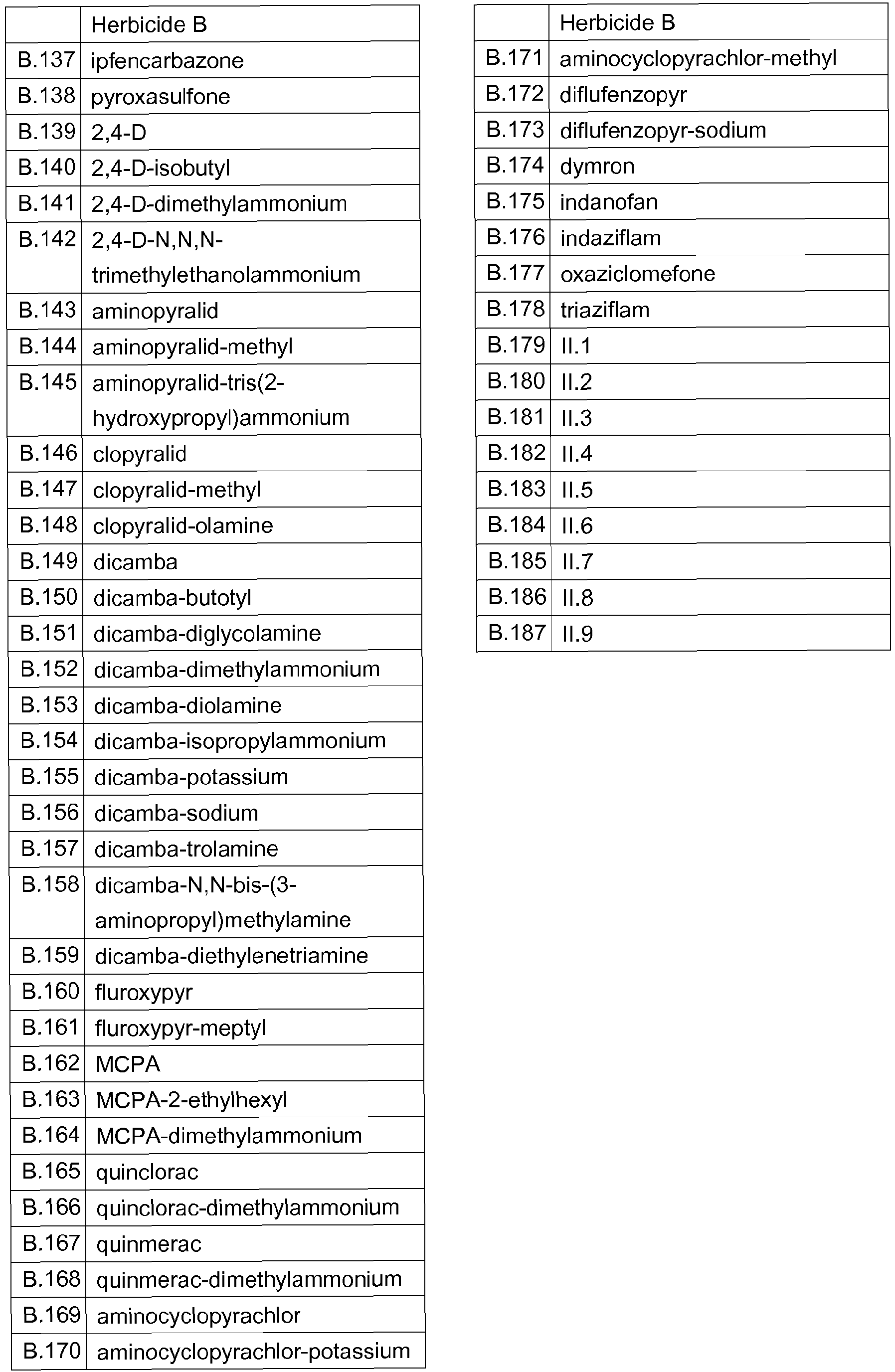

Particularly preferred safeners C, which, as component C, are constituent of the composition according to the invention are the safeners C as defined above; in particular the safeners C.1 - C.17 listed below in table C:

**Table C**

| | Safener C |
|---|---|
| C.1 | benoxacor |
| C.2 | cloquintocet |
| C.3 | cloquintocet-mexyl |
| C.4 | cyprosulfamide |
| C.5 | dichlormid |
| C.6 | fenchlorazole |
| C.7 | fenchlorazole-ethyl |
| C.8 | fenclorim |
| C.9 | furilazole |
| C.10 | isoxadifen |
| C.11 | isoxad ifen-ethyl |
| C.12 | mefenpyr |
| C.13 | mefenpyr-diethyl |
| C.14 | naphtalic acid anhydride |
| C.15 | 4-(dichloroacetyl)-1-oxa-4-azaspiro[4.5]decane (MON4660, CAS 71526-07-3) |
| C.16 | 2,2,5-trimethyl-3-(dichloroacetyl)-1,3-oxazolidine (R-29148, CAS 52836-31-4) |
| C.17 | N-(2-Methoxybenzoyl)-4-[(methylaminocarbonyl)amino]benzenesulfonamide (CAS 129531-12-0) |

The weight ratios of the individual components in the preferred mixtures mentioned below are within the limits given above, in particular within the preferred limits.

Particularly preferred are the compositions mentioned below comprising the compounds of formula I as defined and the substance(s) as defined in the respective row of table 1;
especially preferred comprising as only herbicidal active compounds the compounds of formula I as defined and the substance(s) as defined in the respective row of table 1; most preferably comprising as only active compounds the compounds of formula I as defined and the substance(s) as defined in the respective row of table 1.

Particularly preferred are compositions 1.1 to 1.3383, comprising the compound I.a and the substance(s) as defined in the respective row of table 1:

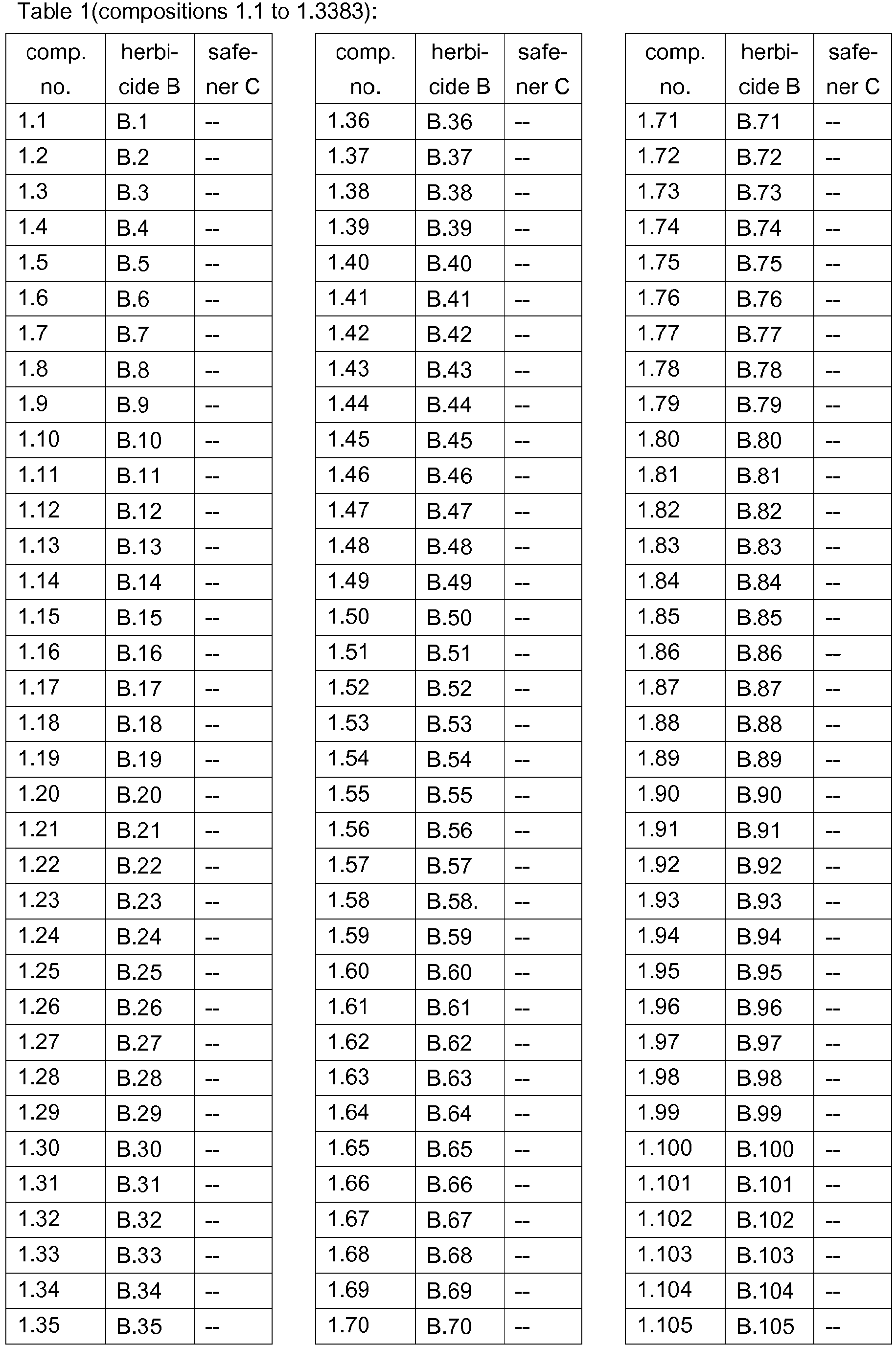

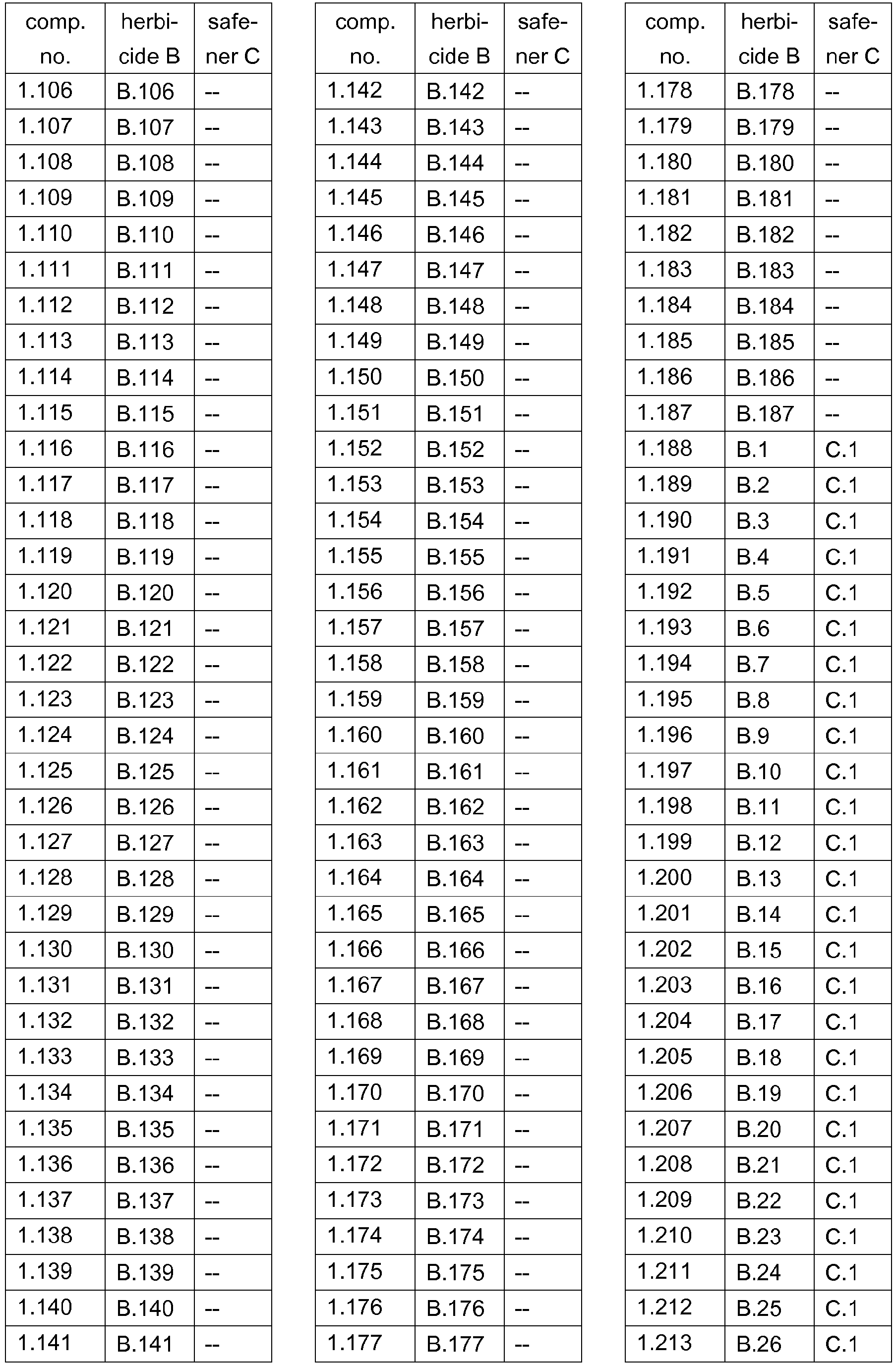

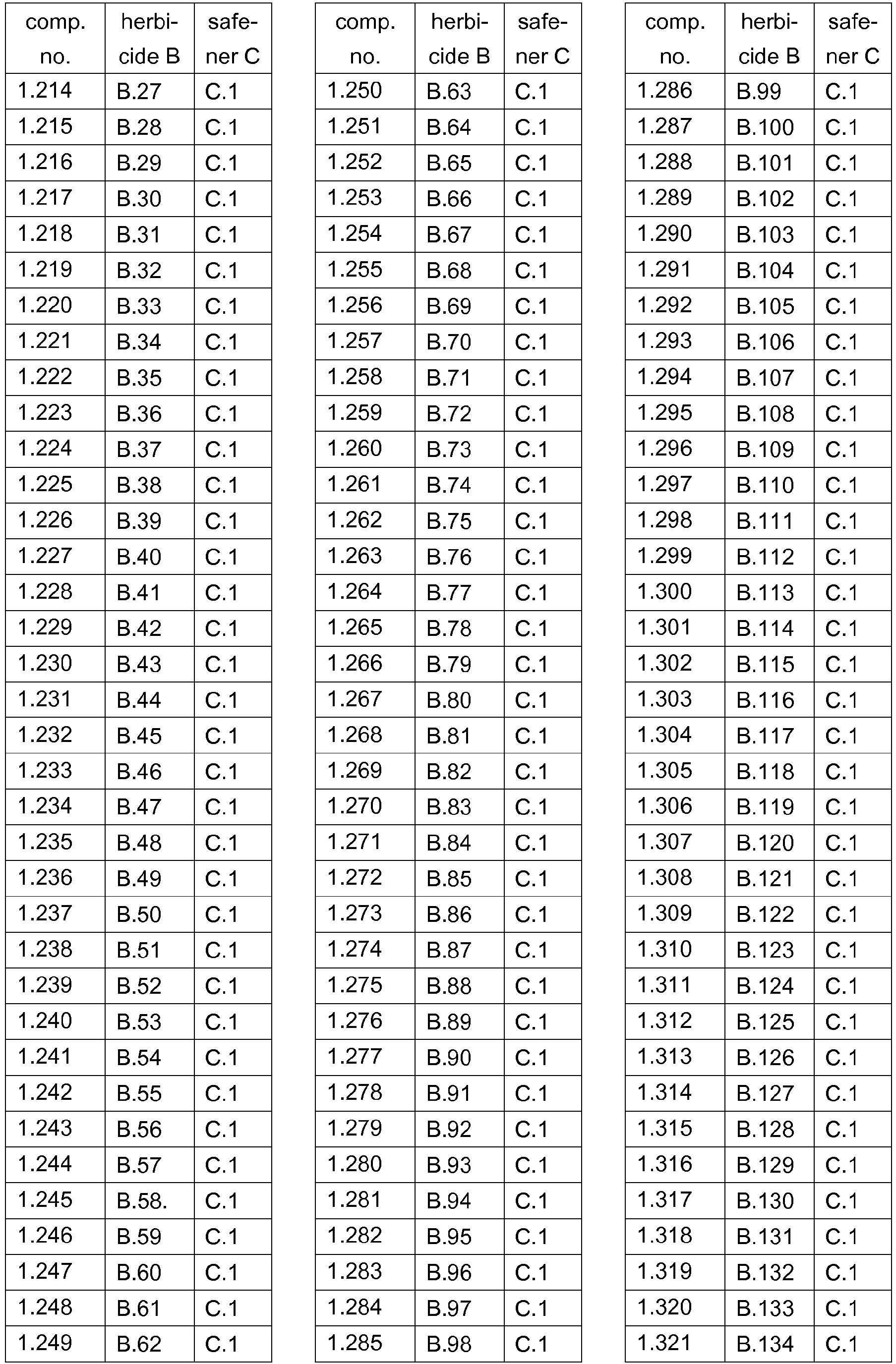

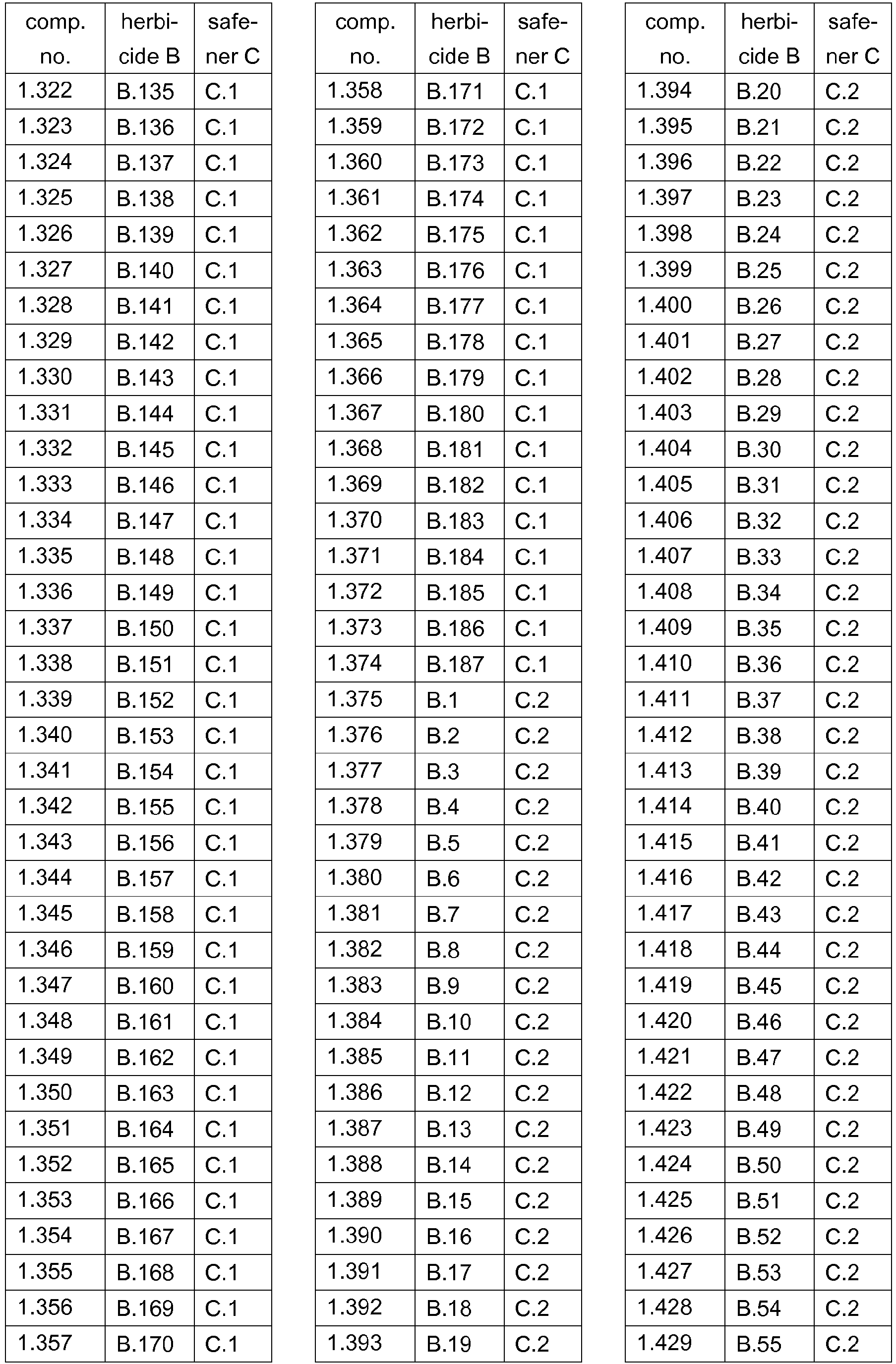

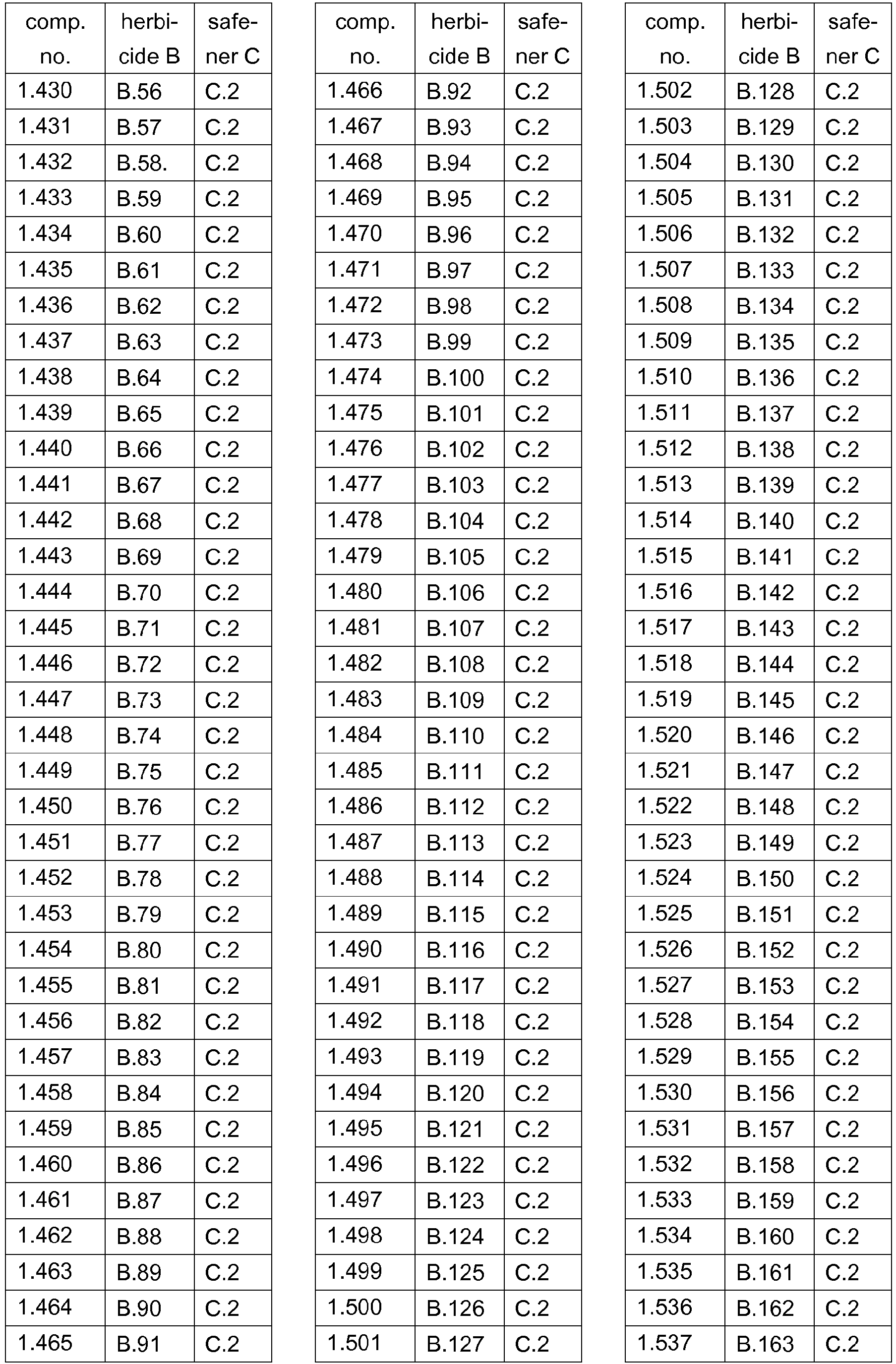

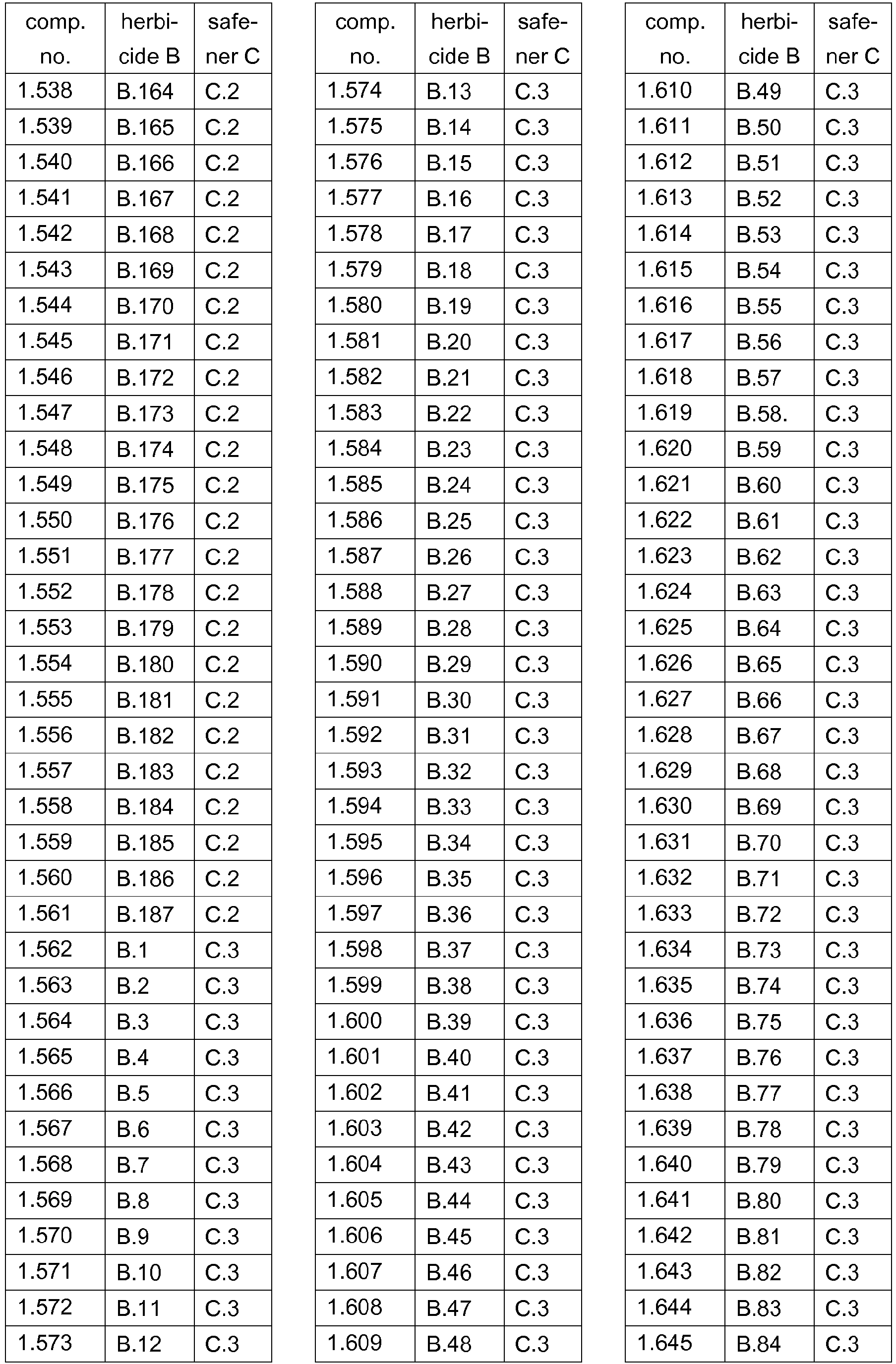

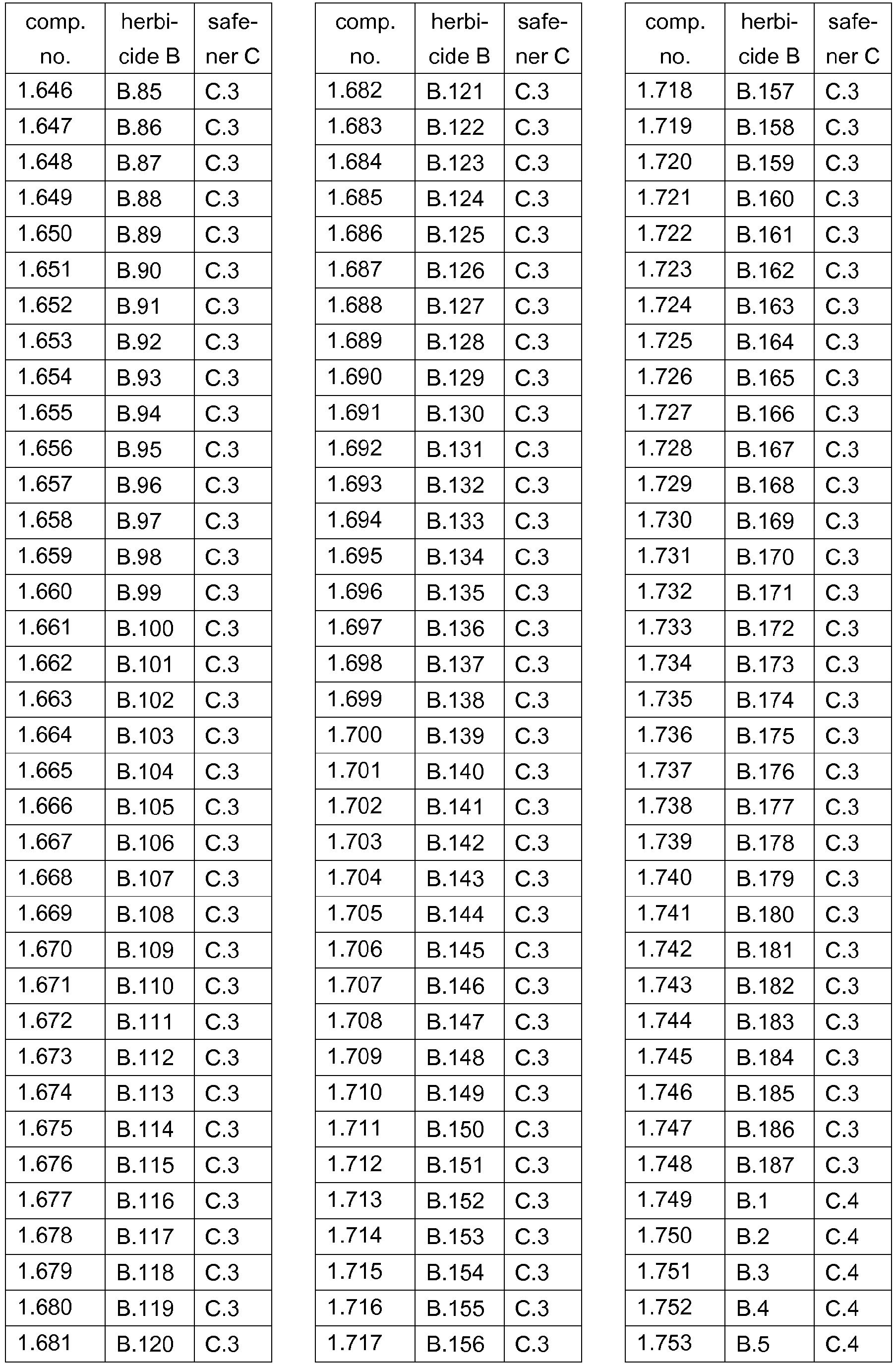

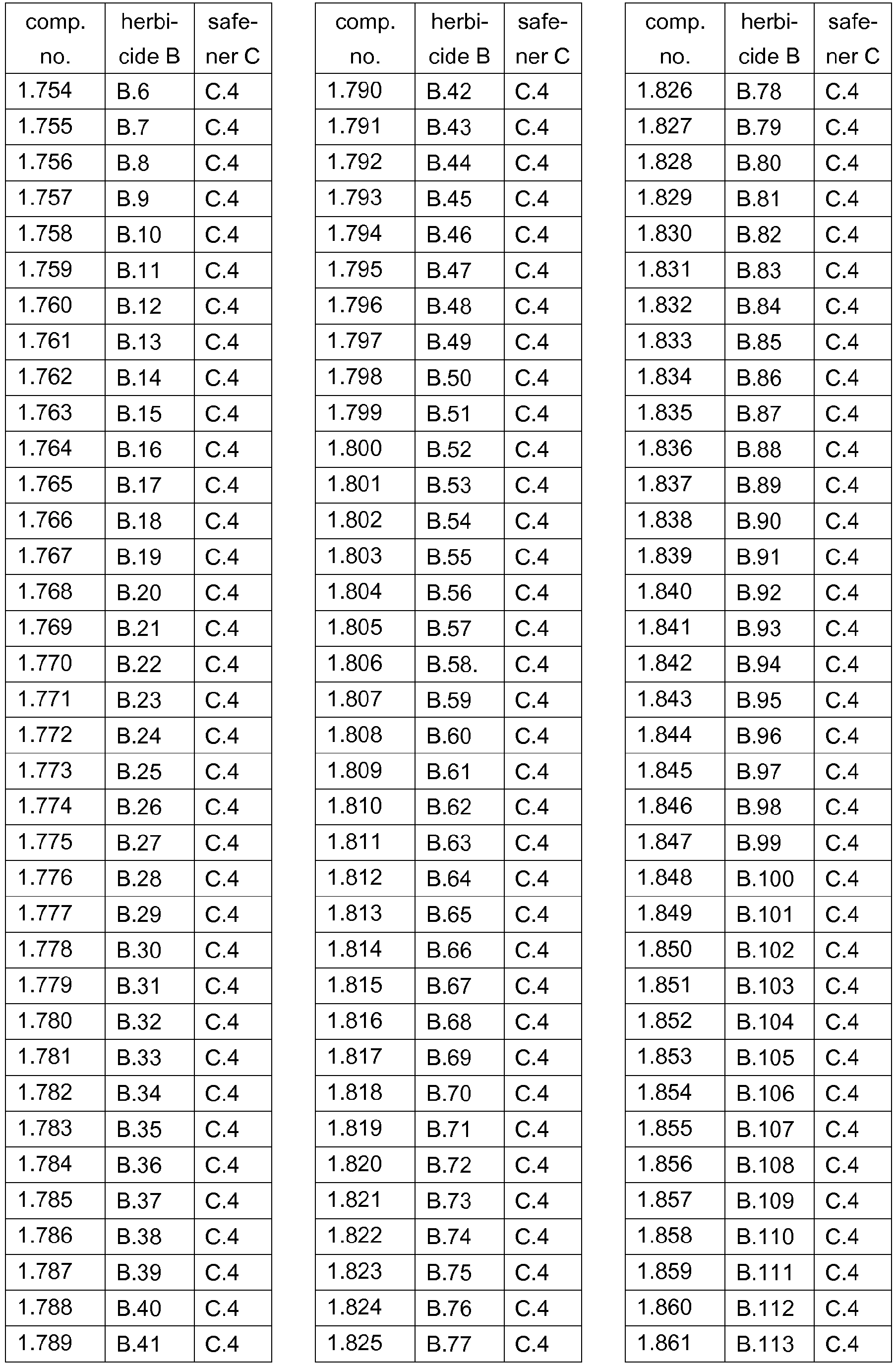

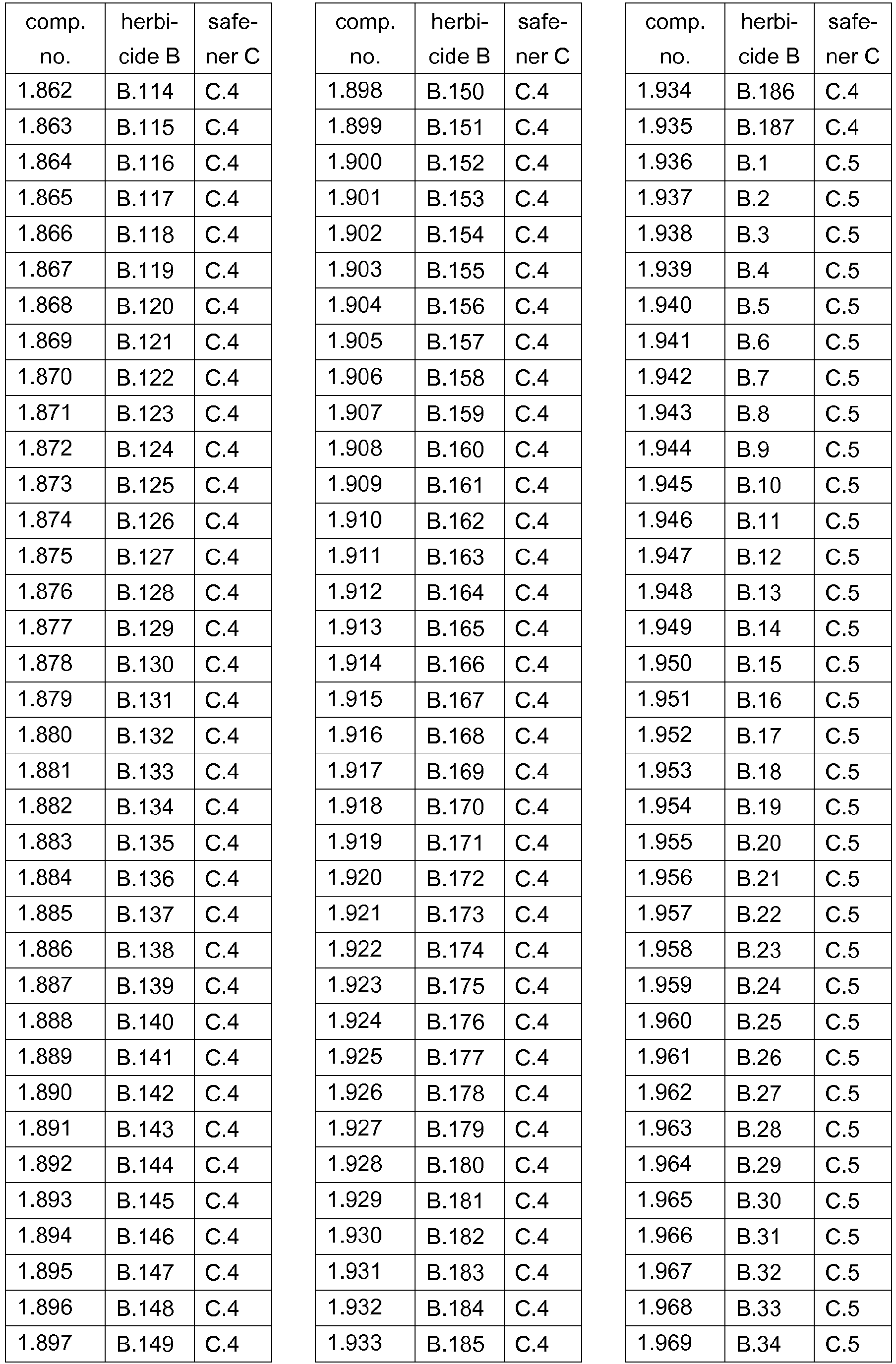

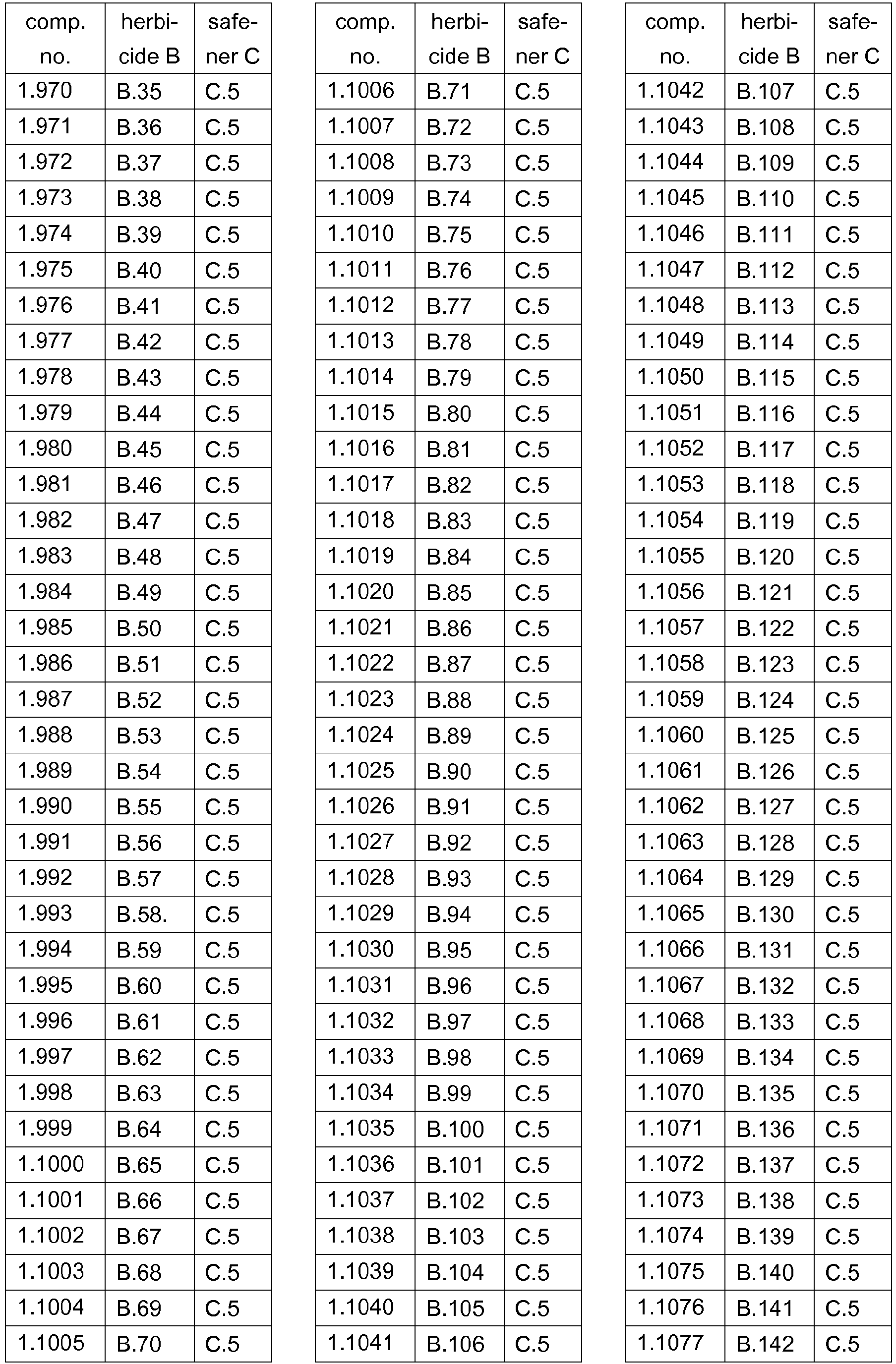

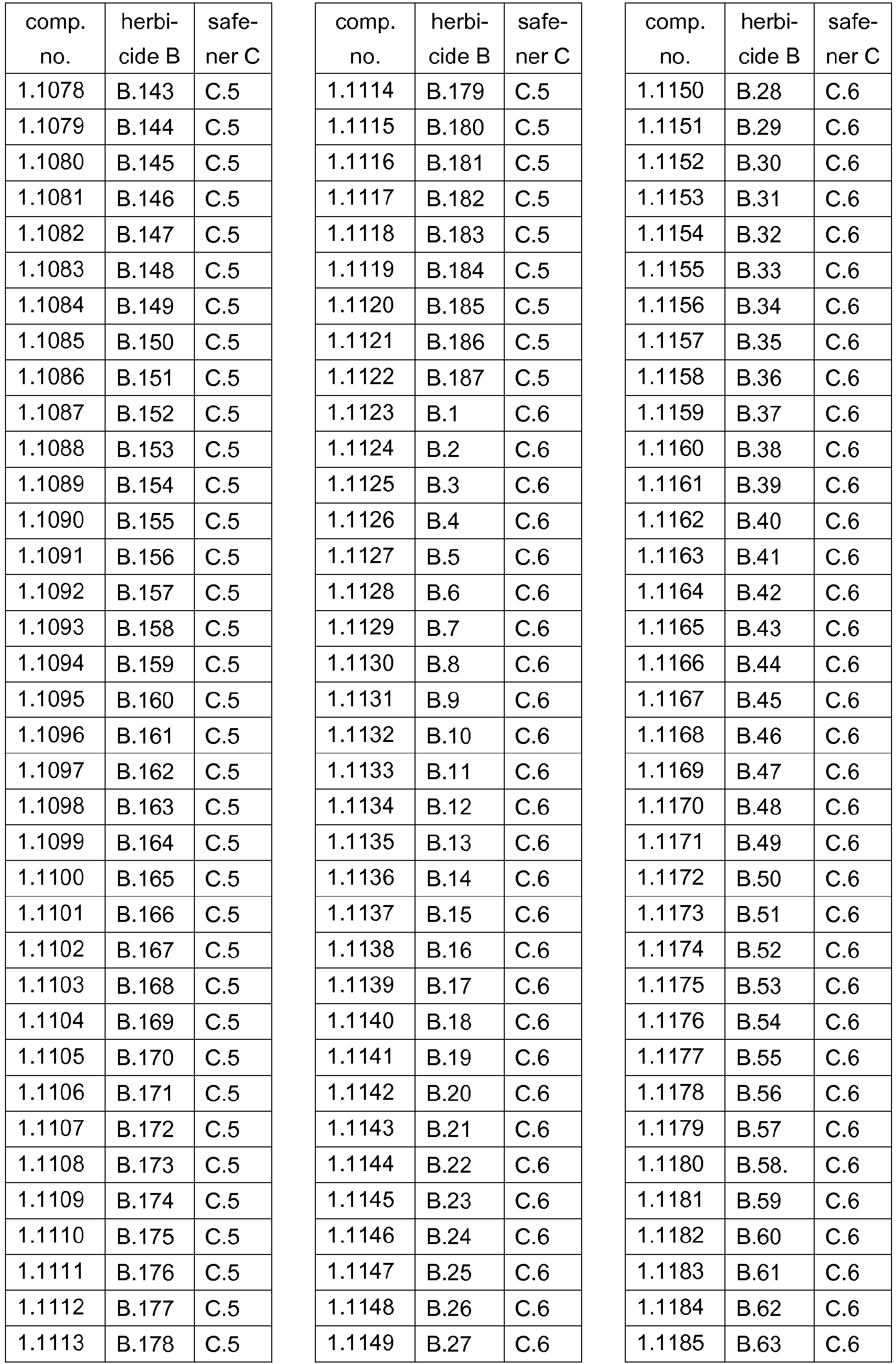

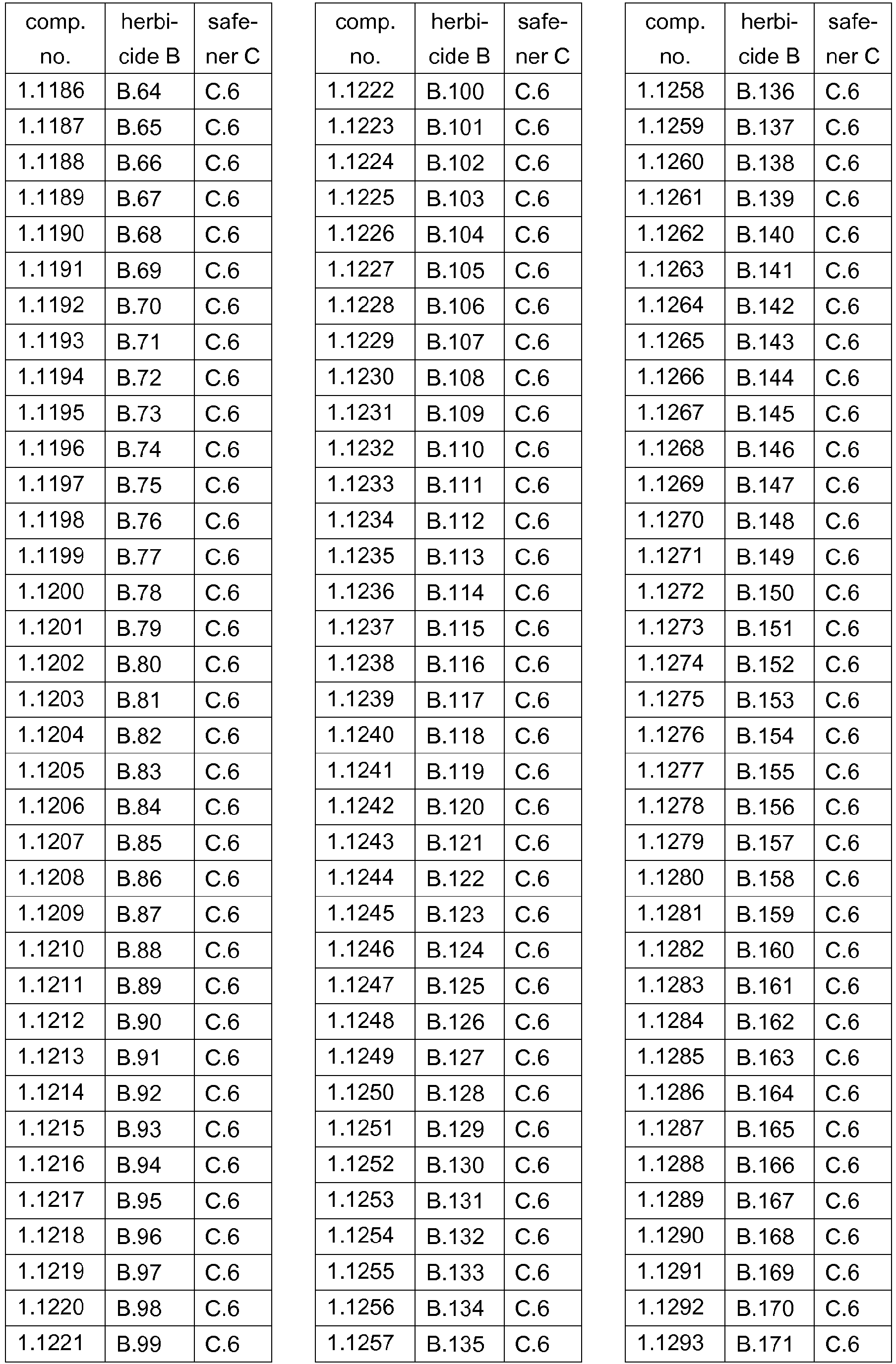

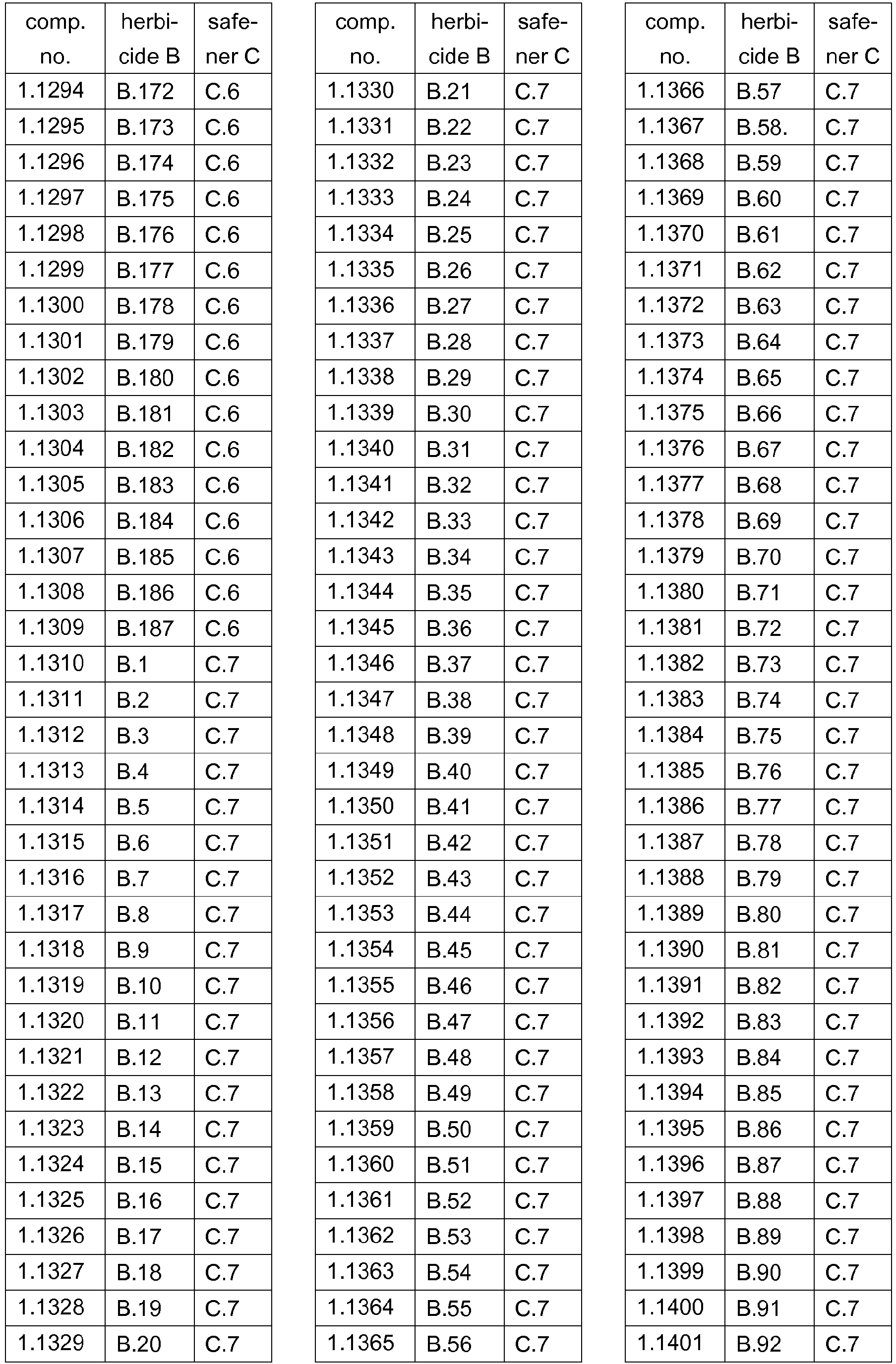

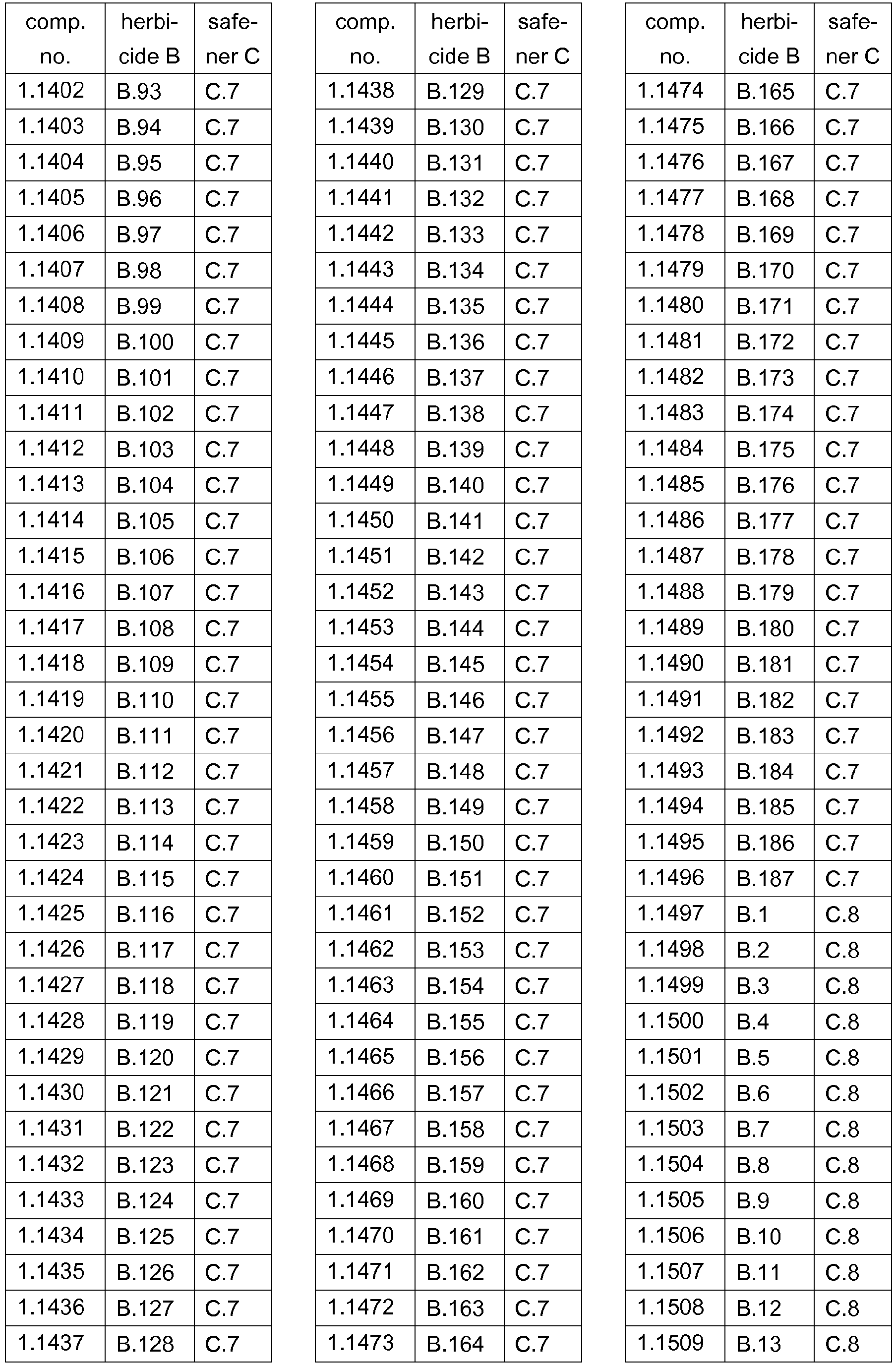

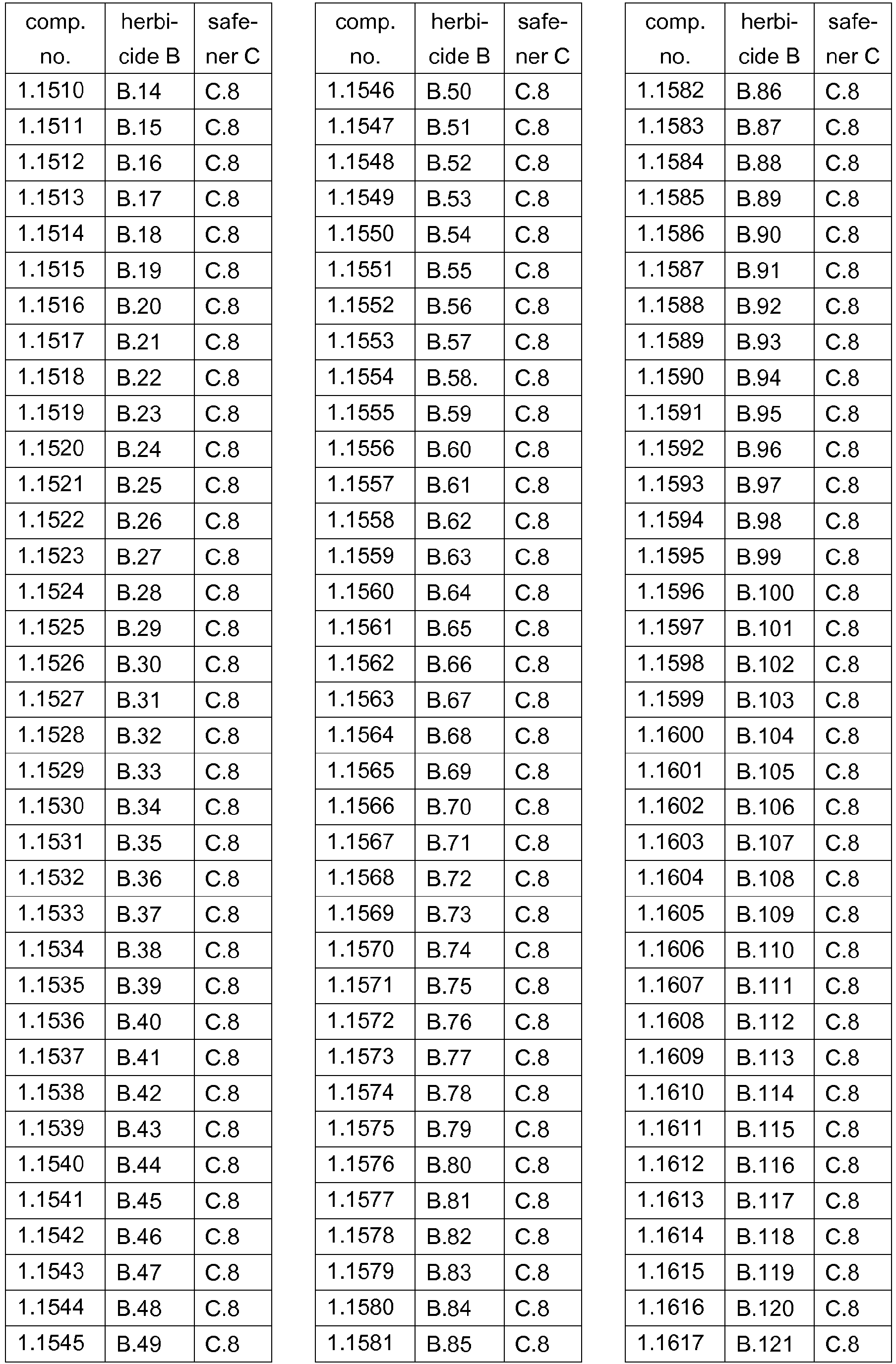

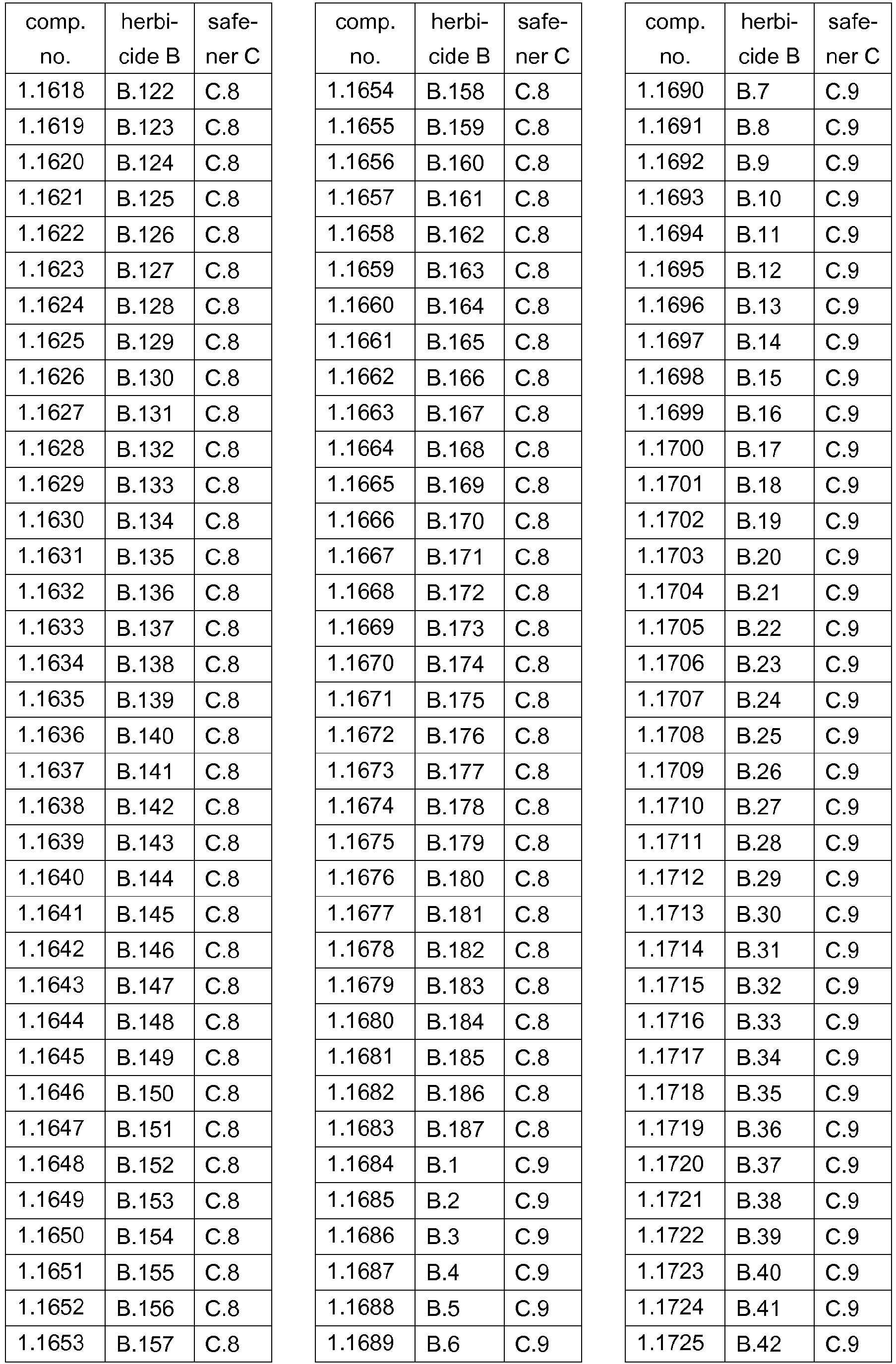

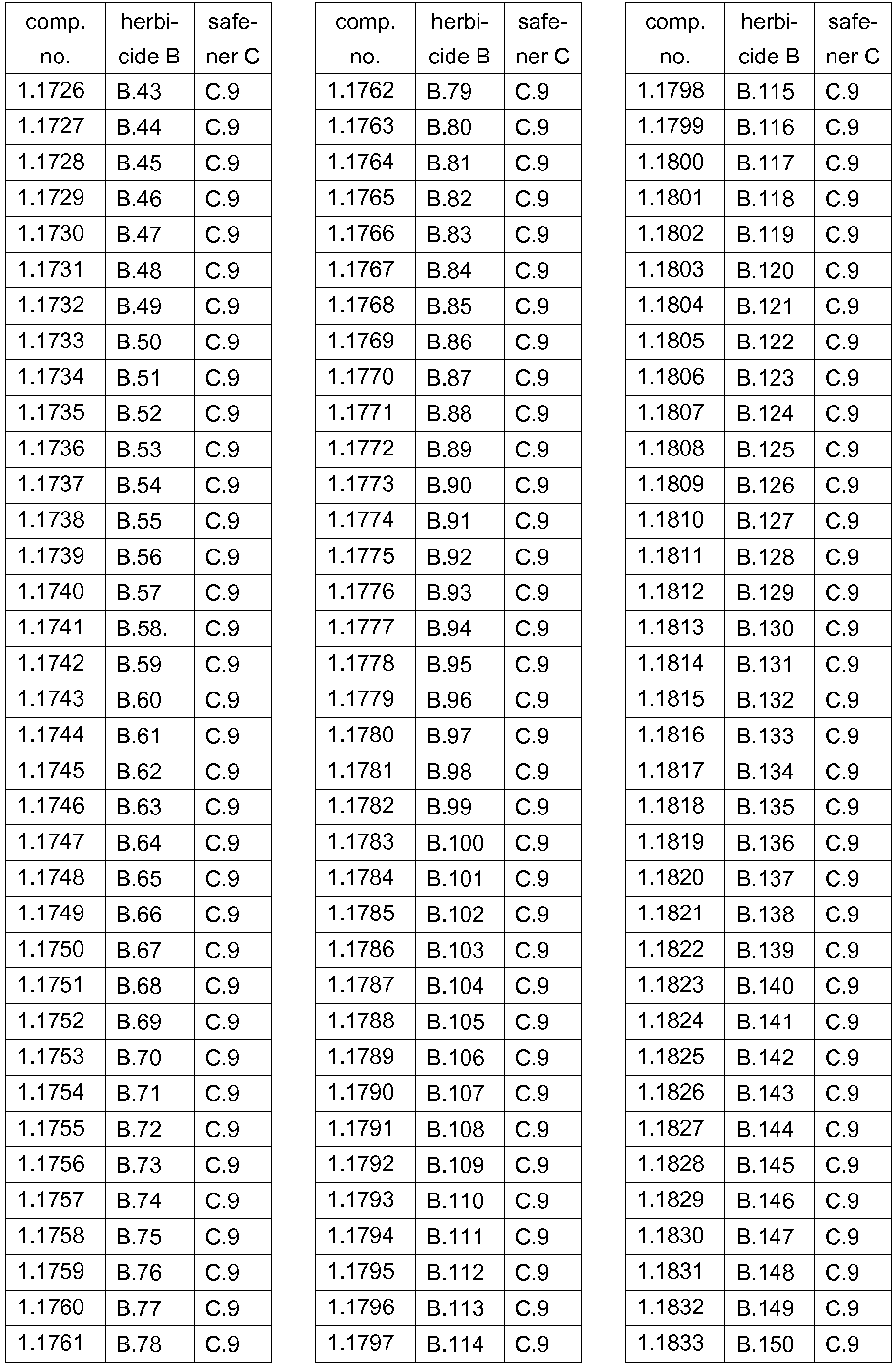

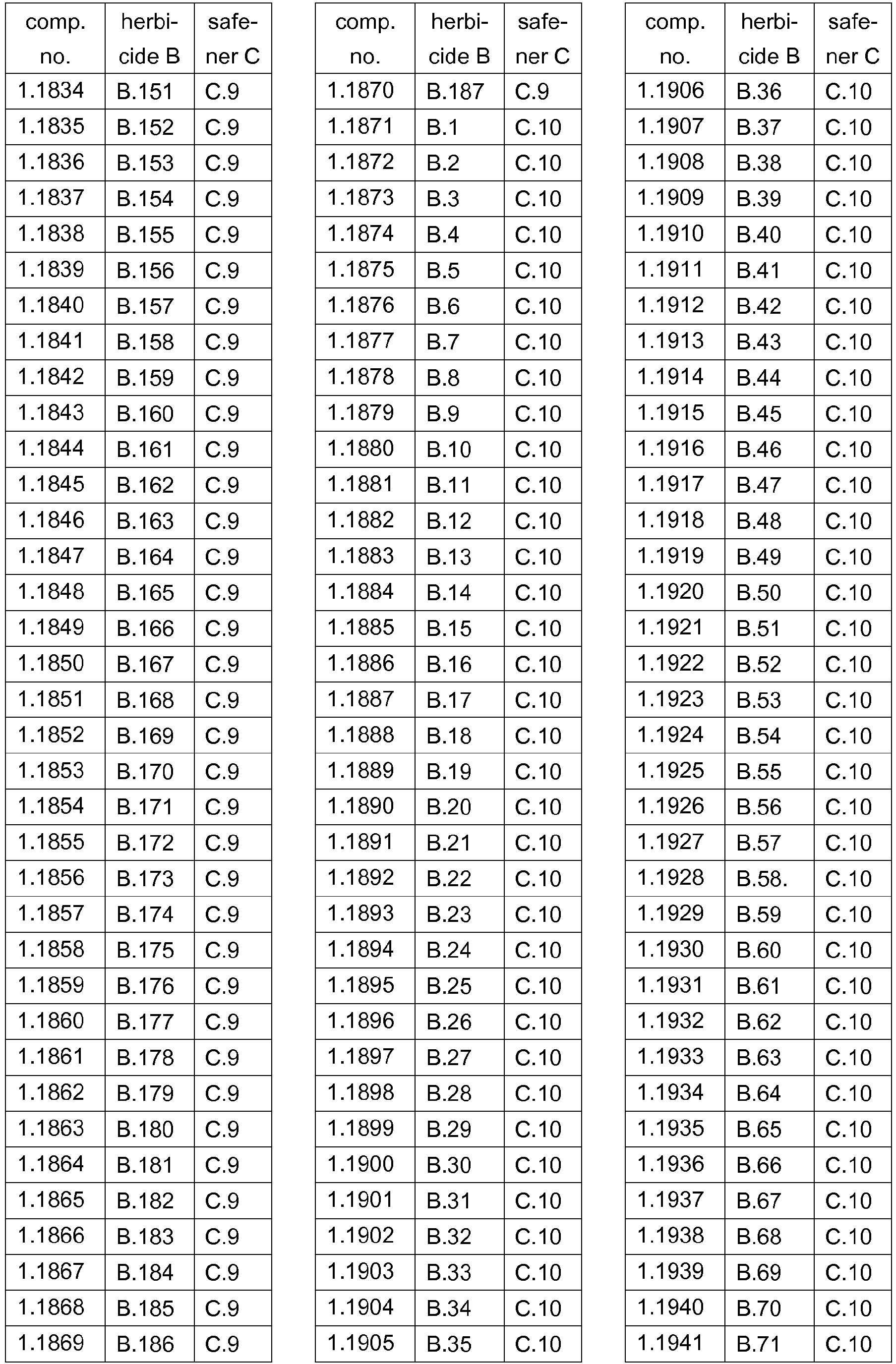

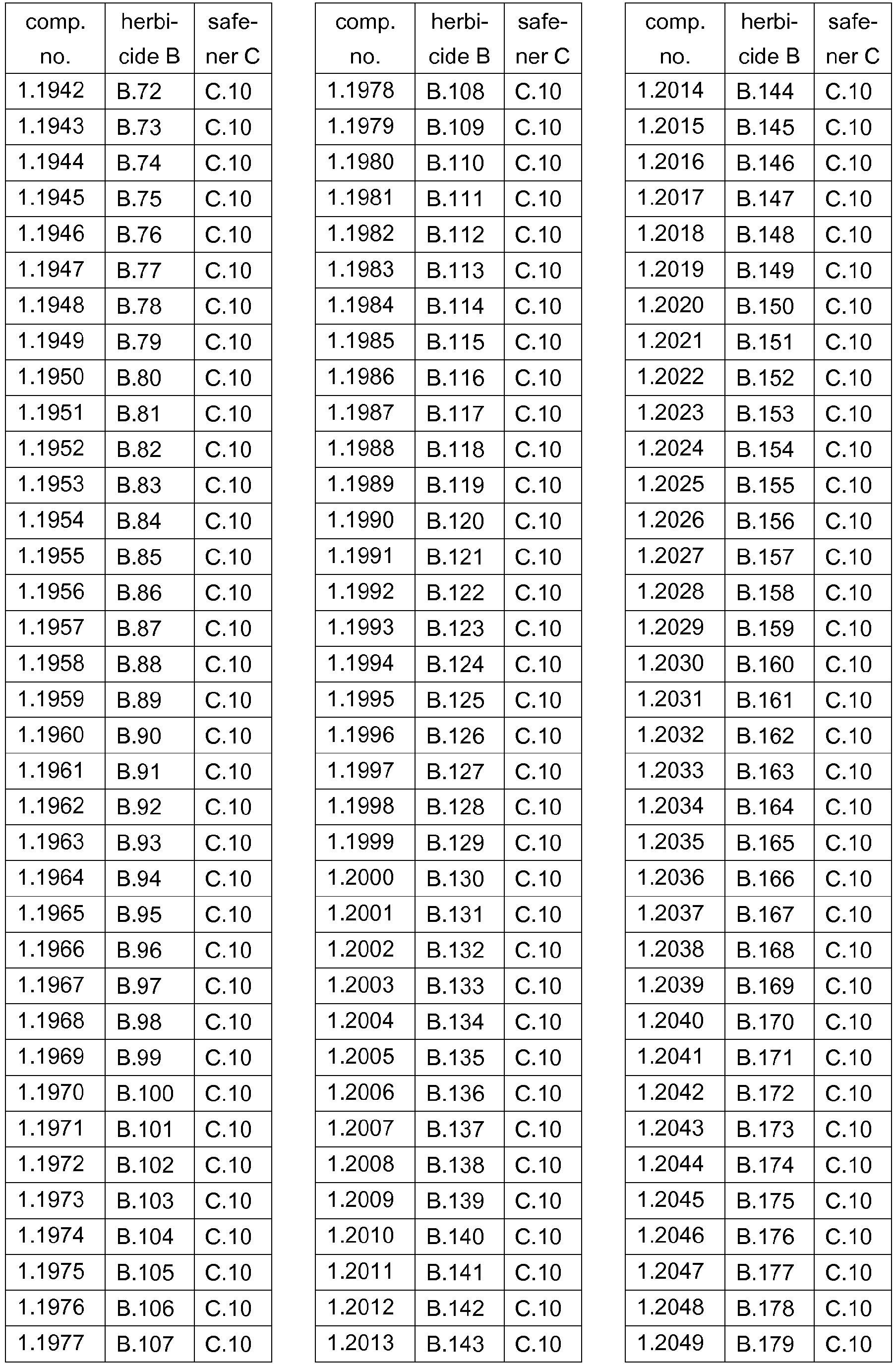

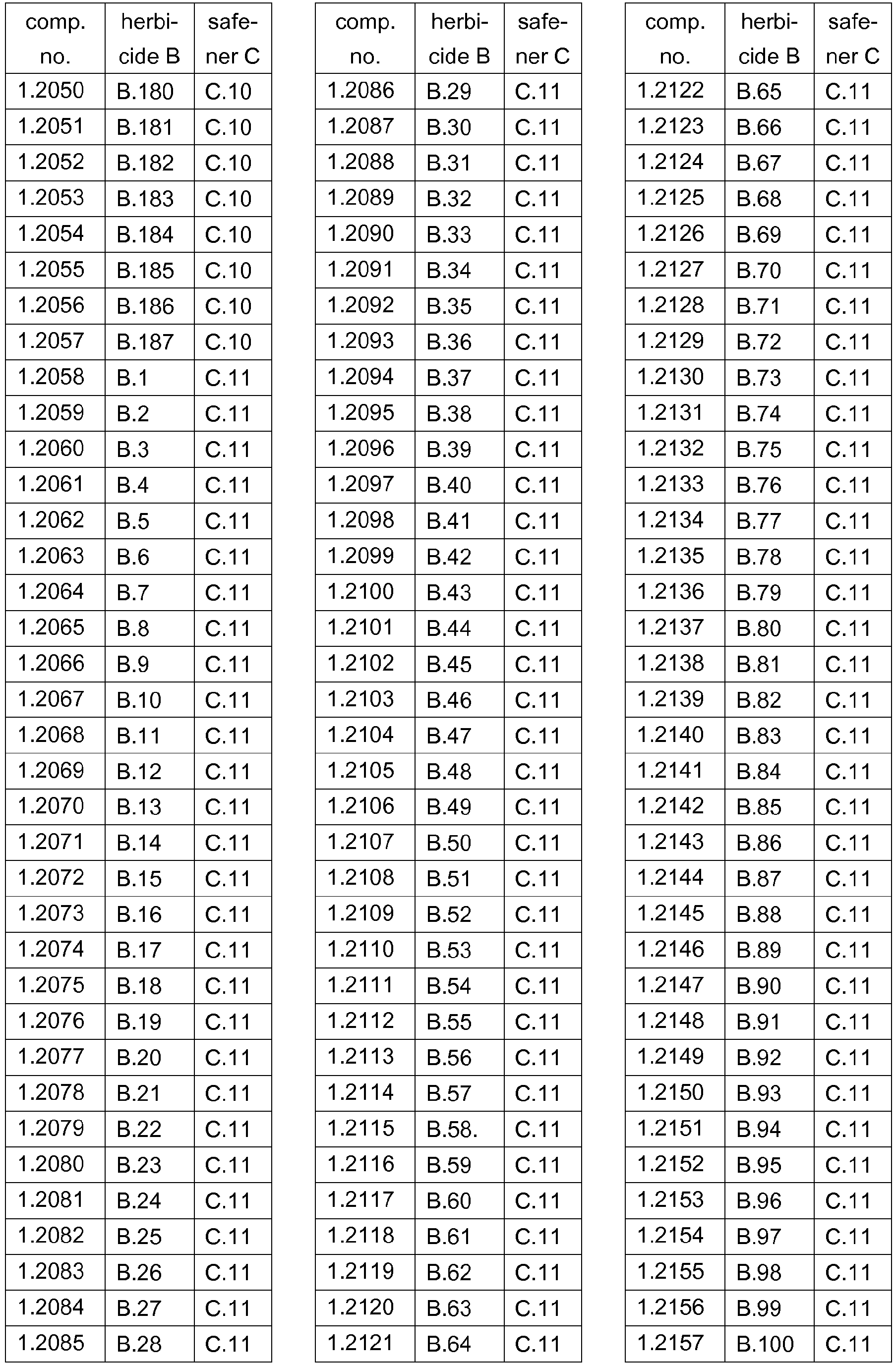

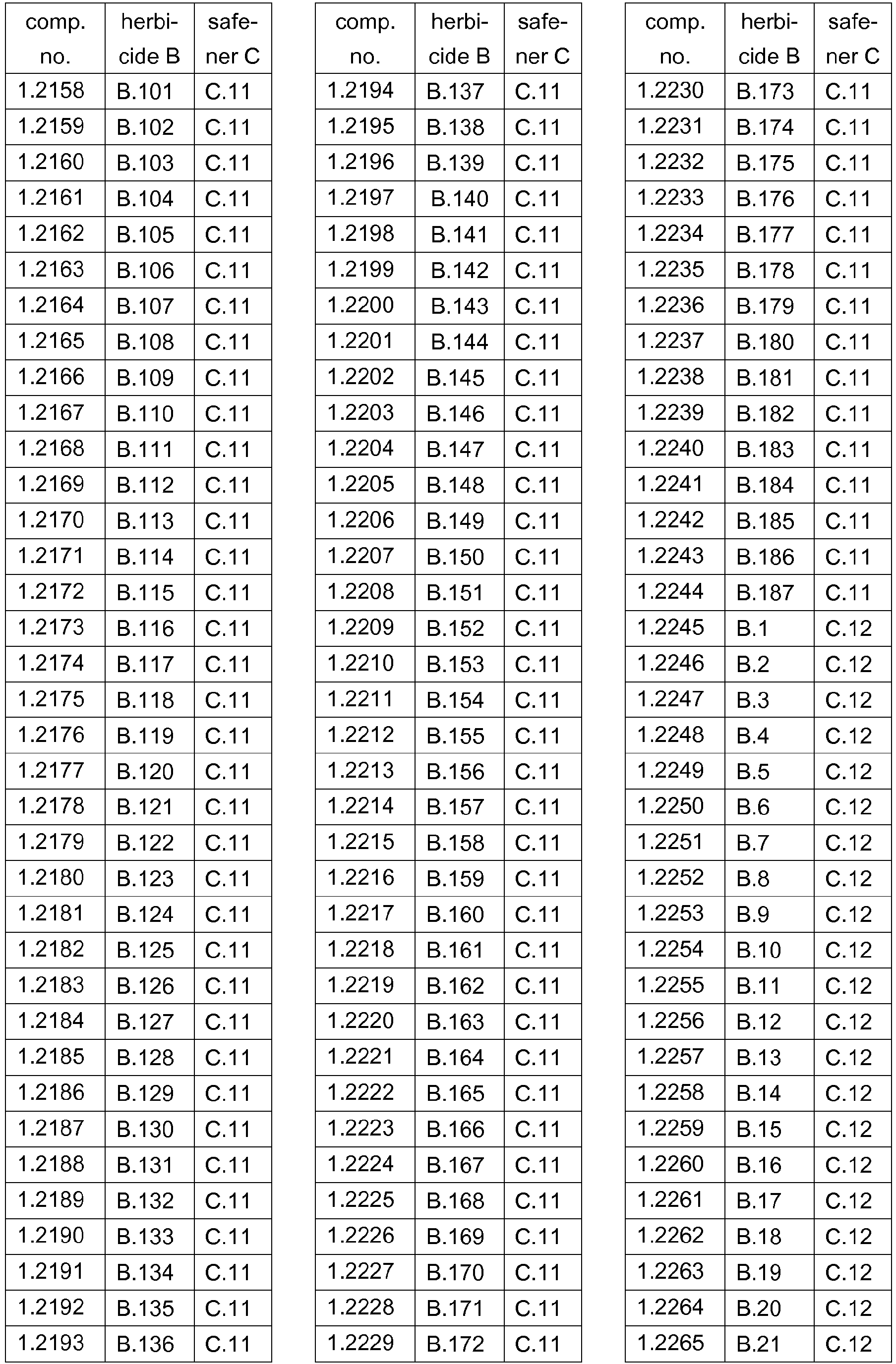

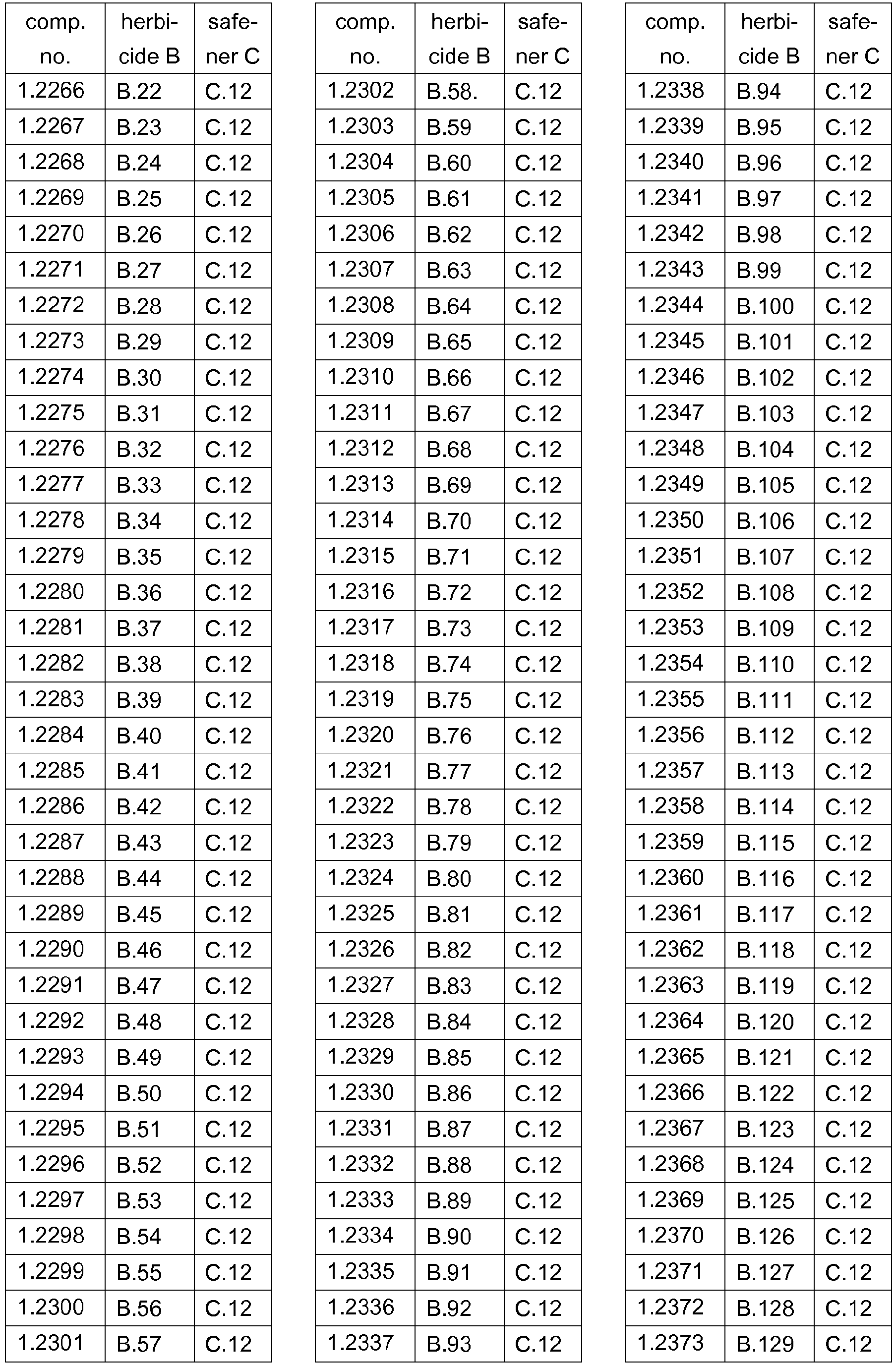

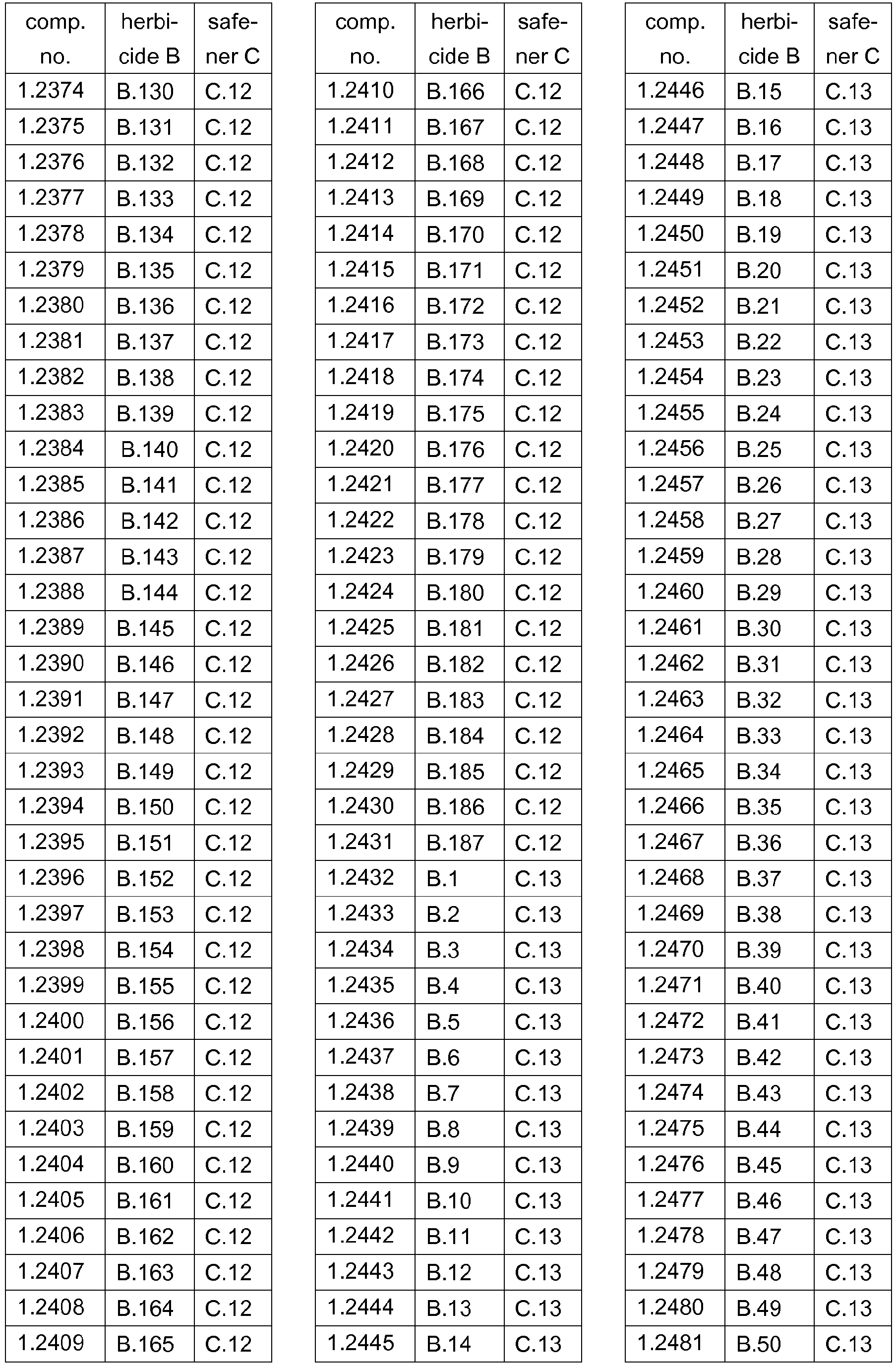

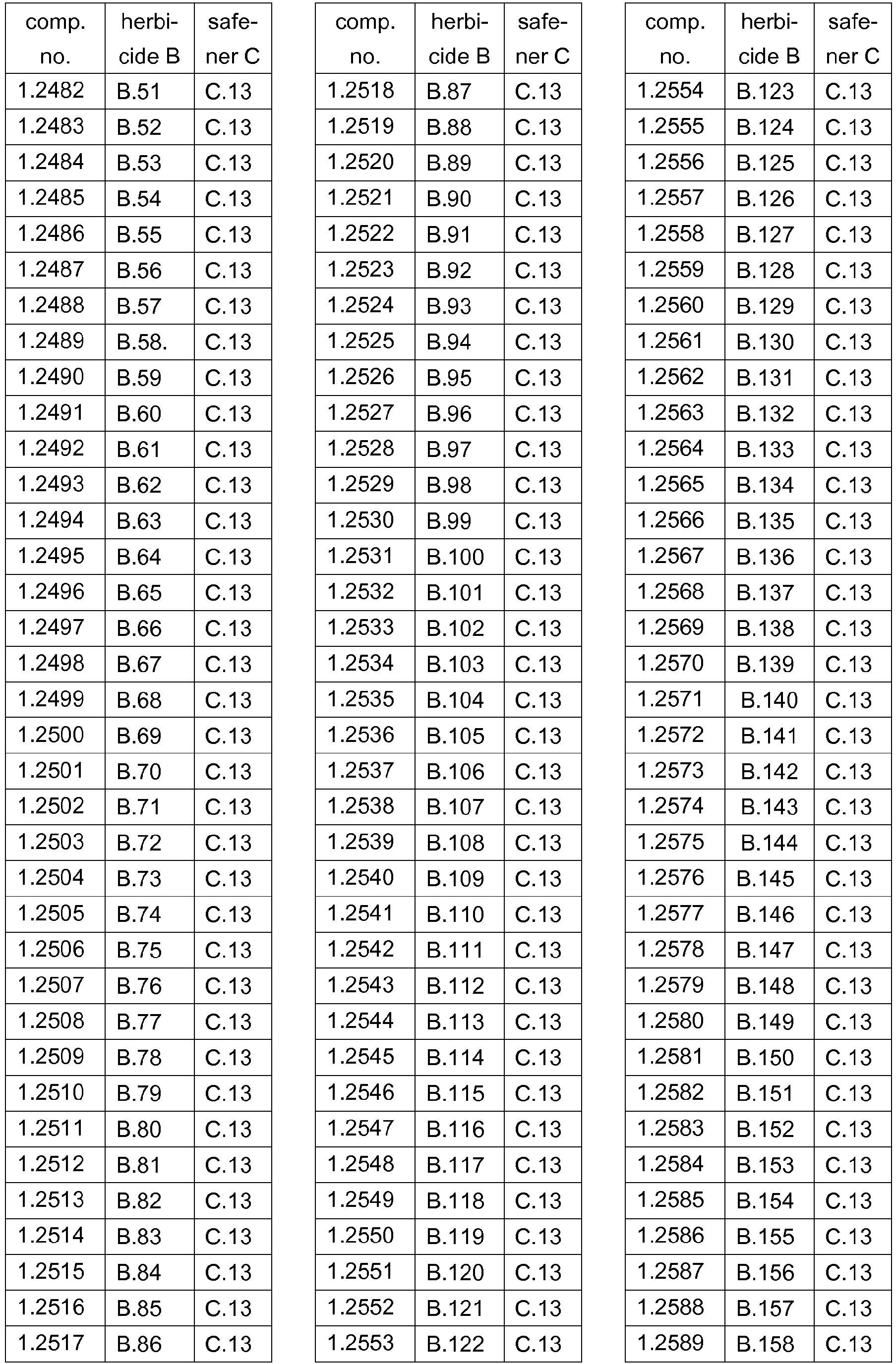

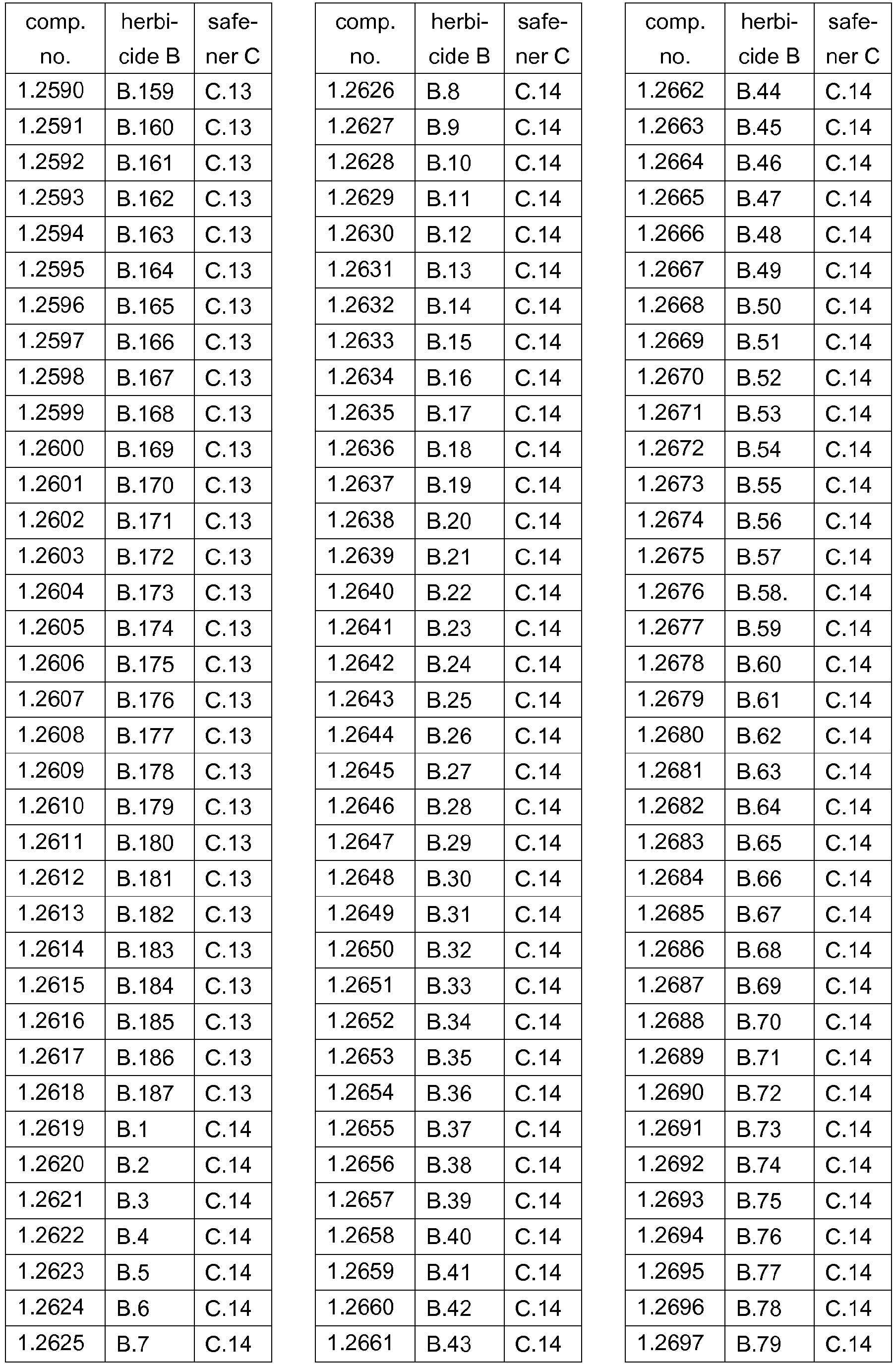

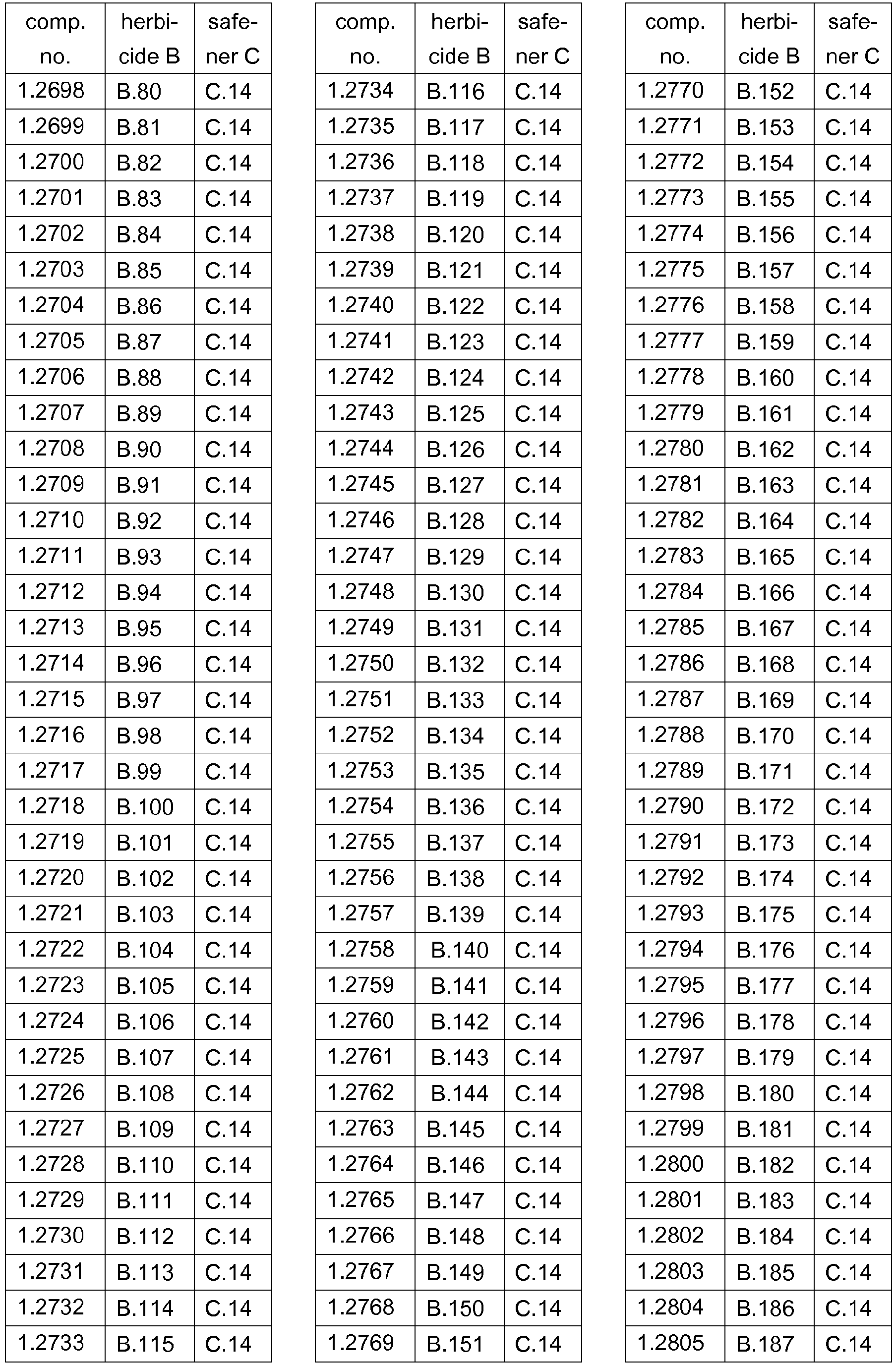

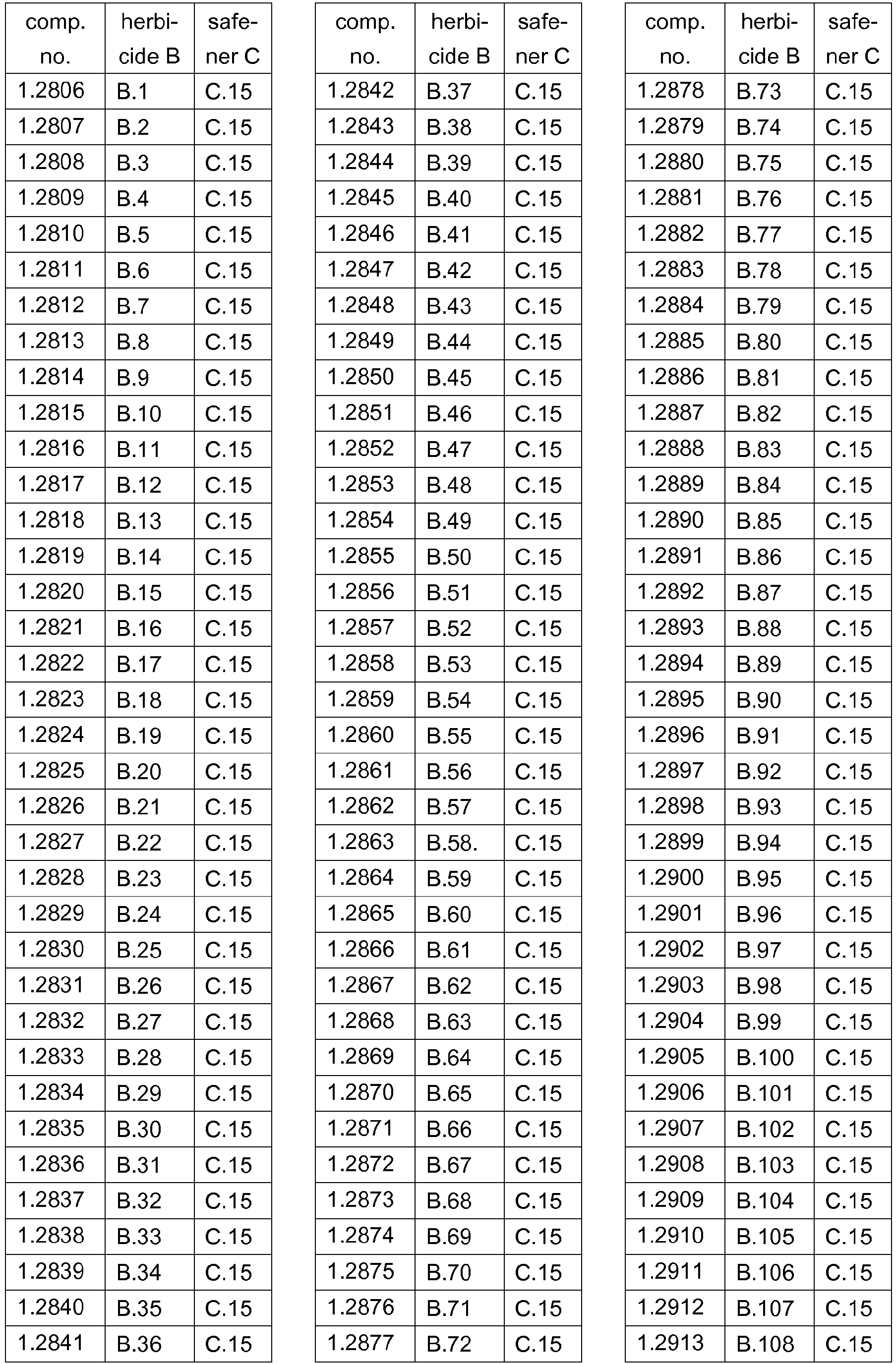

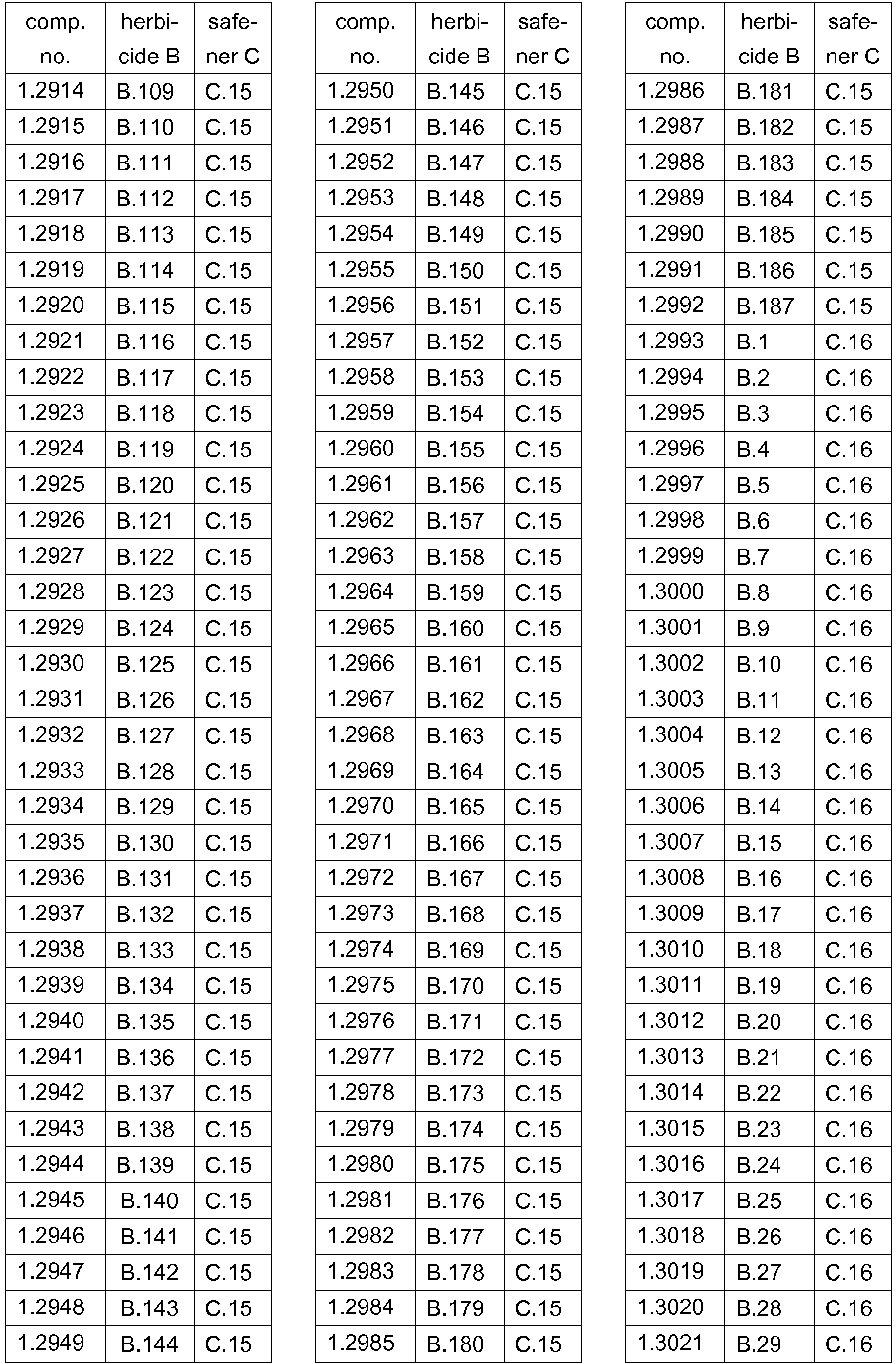

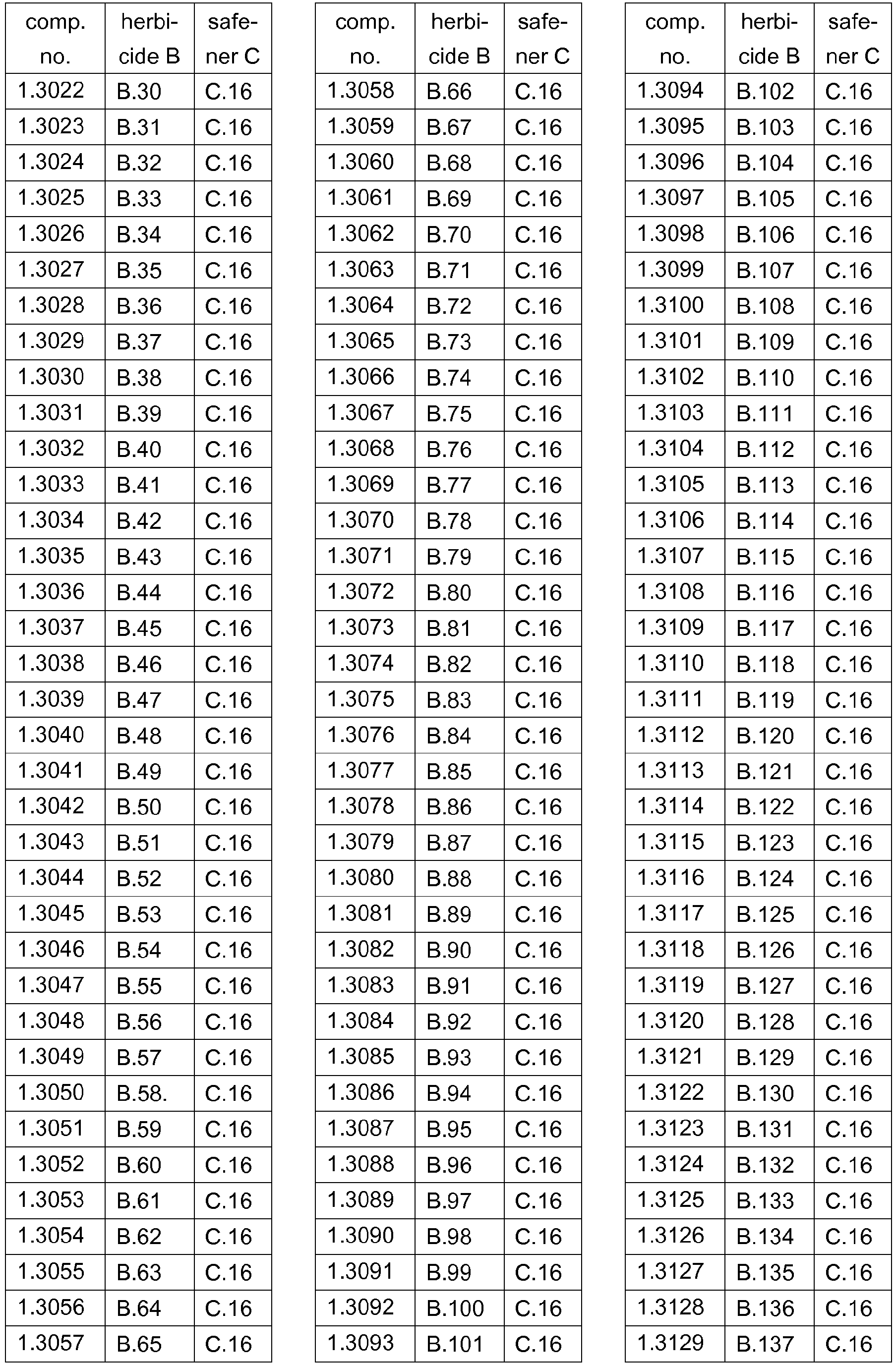

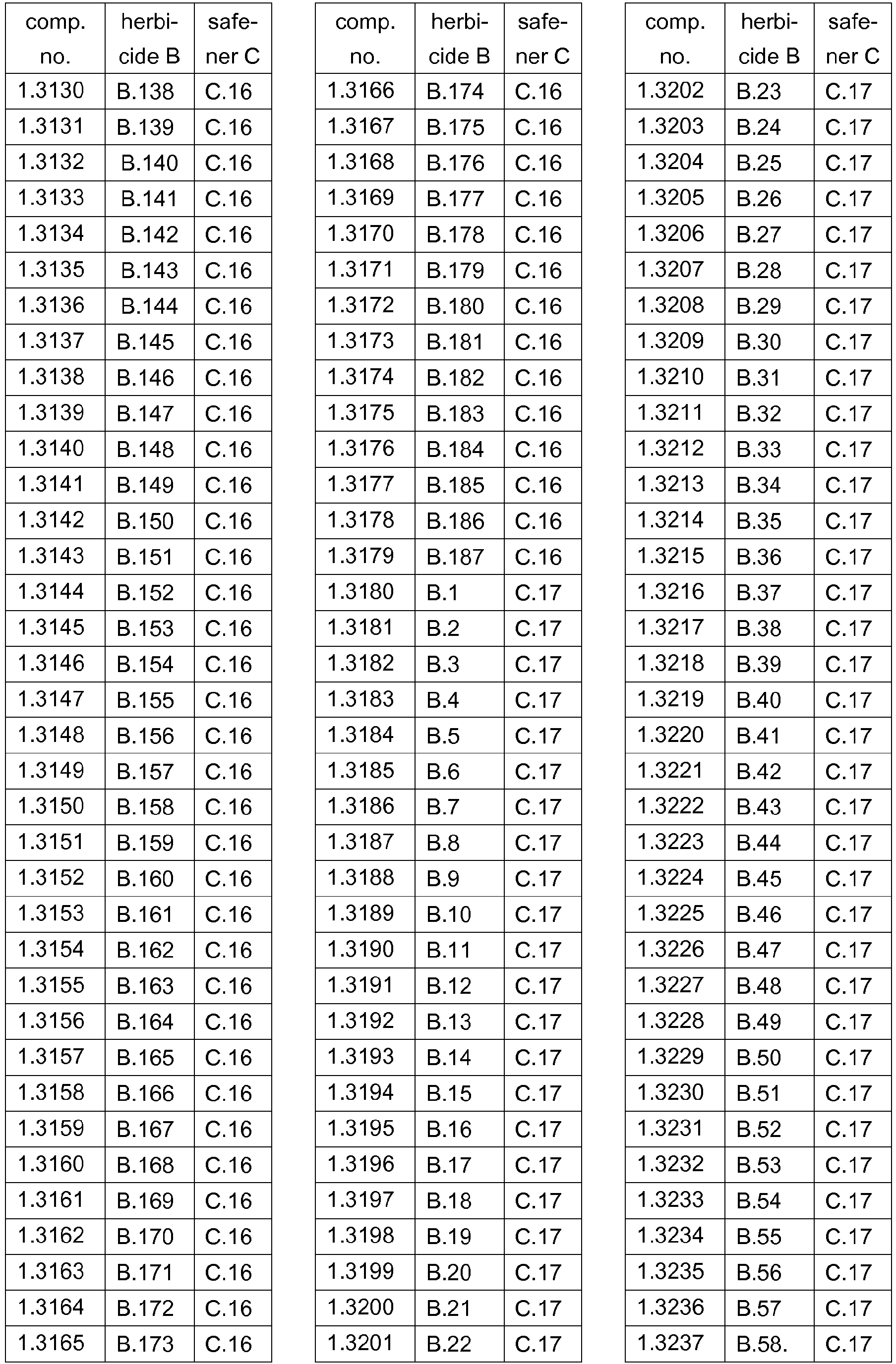

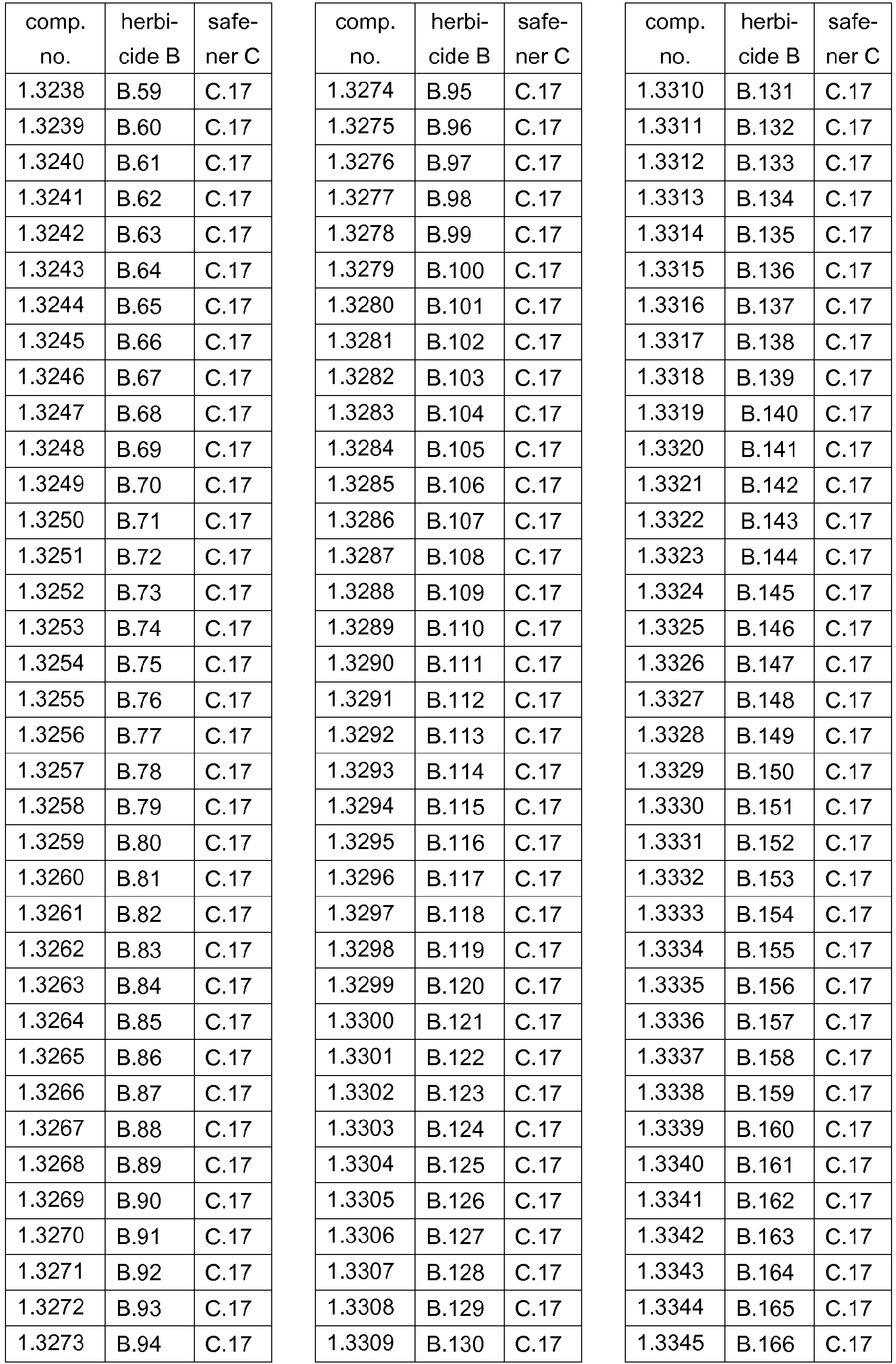

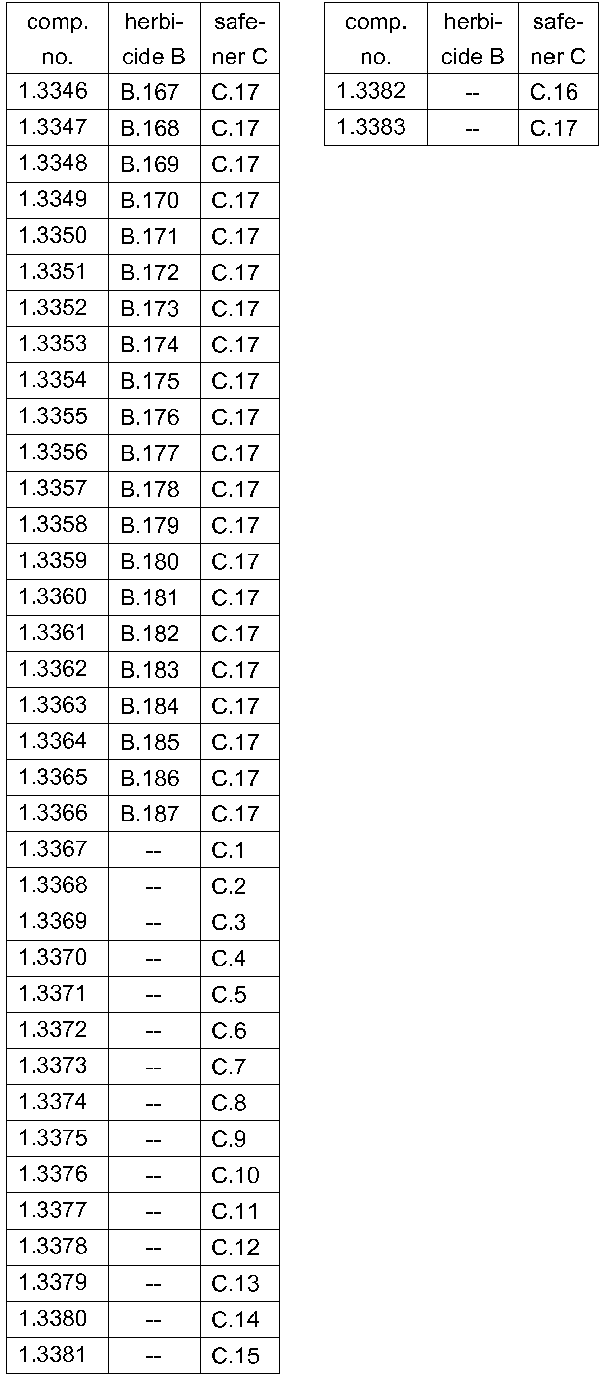

The specific number for each single composition is deductible as follows:
Composition 1.777 for example comprises the compound I, foramsulfuron (B.29) and cyprosulfamide (C.4) (*see table 1, entry 1.777; as well as table B, entry B.29 and table C*, *entry C.4*).
Composition 2.777 for example comprises the compound I (see *the definition for compositions 2.1 to 2.3383 below*), foramsulfuron (B.29) and cyprosulfamide (C.4) (see *table 1, entry 1.777; as well as table B, entry B.29 and table C*, *entry C.4*).
Composition 7.777 for example comprises imazapyr (B.35) (see *the definition for compositions 7.1 to 7.3383 below),* and the compound I, foramsulfuron (B.29) and cyprosulfamide (C.4) (*see table 1, entry 1.777; as well as table B, entry B.29 and table C*, *entry* C.4).
Also especially preferred are compositions 2.1. to 2.3383 which differ from the corresponding compositions 1.1 to 1.3383 only in that they comprise as the active compound A the compound I.1.

Also especially preferred are compositions 3.1. to 3.3383 which differ from the corresponding compositions 1.1 to 1.3383 only in that they additionally comprise B.2 as further herbicide B.

Also especially preferred are compositions 4.1. to 4.3383 which differ from the corresponding compositions 1.1 to 1.3383 only in that they additionally comprise B.8 as further herbicide B.

Also especially preferred are compositions 5.1. to 5.3383 which differ from the corresponding compositions 1.1 to 1.3383 only in that they additionally comprise B.30 as further herbicide B.

Also especially preferred are compositions 6.1. to 6.3383 which differ from the corresponding compositions 1.1 to 1.3383 only in that they additionally comprise B.32 as further herbicide B.

Also especially preferred are compositions 7.1. to 7.3383 which differ from the corresponding compositions 1.1 to 1.3383 only in that they additionally comprise B.35 as further herbicide B.

Also especially preferred are compositions 8.1. to 8.3383 which differ from the corresponding compositions 1.1 to 1.3383 only in that they additionally comprise B.38 as further herbicide B.

Also especially preferred are compositions 9.1. to 9.3383 which differ from the corresponding compositions 1.1 to 1.3383 only in that they additionally comprise B.40 as further herbicide B.

Also especially preferred are compositions 10.1. to 10.3383 which differ from the corresponding compositions 1.1 to 1.3383 only in that they additionally comprise B.51 as further herbicide B.

Also especially preferred are compositions 11.1. to 11.3383 which differ from the corresponding compositions 1.1 to 1.3383 only in that they additionally comprise B.55 as further herbicide B.

Also especially preferred are compositions 12.1. to 12.3383 which differ from the corresponding compositions 1.1 to 1.3383 only in that they additionally comprise B.56 as further herbicide B.

Also especially preferred are compositions 13.1. to 13.3383 which differ from the corresponding compositions 1.1 to 1.3383 only in that they additionally comprise B.64 as further herbicide B.

Also especially preferred are compositions 14.1. to 14.3383 which differ from the corresponding compositions 1.1 to 1.3383 only in that they additionally comprise B.66 as further herbicide B.

Also especially preferred are compositions 15.1. to 15.3383 which differ from the corresponding compositions 1.1 to 1.3383 only in that they additionally comprise B.67 as further herbicide B.

Also especially preferred are compositions 16.1. to 16.3383 which differ from the corresponding compositions 1.1 to 1.3383 only in that they additionally comprise B.68 as further herbicide B.

Also especially preferred are compositions 17.1. to 17.3383 which differ from the corresponding compositions 1.1 to 1.3383 only in that they additionally comprise B.69 as further herbicide B.

Also especially preferred are compositions 18.1. to 18.3383 which differ from the corresponding compositions 1.1 to 1.3383 only in that they additionally comprise B.73 as further herbicide B.

Also especially preferred are compositions 19.1. to 19.3383 which differ from the corresponding compositions 1.1 to 1.3383 only in that they additionally comprise B.76 as further herbicide B.

Also especially preferred are compositions 20.1. to 20.3383 which differ from the corresponding compositions 1.1 to 1.3383 only in that they additionally comprise B.81 as further herbicide B.

Also especially preferred are compositions 21.1. to 21.3383 which differ from the corresponding compositions 1.1 to 1.3383 only in that they additionally comprise B.82 as further herbicide B.

Also especially preferred are compositions 22.1. to 22.3383 which differ from the corresponding compositions 1.1 to 1.3383 only in that they additionally comprise B.85 as further herbicide B.

Also especially preferred are compositions 23.1. to 23.3383 which differ from the corresponding compositions 1.1 to 1.3383 only in that they additionally comprise B.88 as further herbicide B.

Also especially preferred are compositions 24.1. to 24.3383 which differ from the corresponding compositions 1.1 to 1.3383 only in that they additionally comprise B.89 as further herbicide B.

Also especially preferred are compositions 25.1. to 25.3383 which differ from the corresponding compositions 1.1 to 1.3383 only in that they additionally comprise B.92 as further herbicide B.

Also especially preferred are compositions 26.1. to 26.3383 which differ from the corresponding compositions 1.1 to 1.3383 only in that they additionally comprise B.93 as further herbicide B.

Also especially preferred are compositions 27.1. to 27.3383 which differ from the corresponding compositions 1.1 to 1.3383 only in that they additionally comprise B.96 as further herbicide B.

Also especially preferred are compositions 28.1. to 28.3383 which differ from the corresponding compositions 1.1 to 1.3383 only in that they additionally comprise B.97 as further herbicide B.

Also especially preferred are compositions 29.1. to 29.3383 which differ from the corresponding compositions 1.1 to 1.3383 only in that they additionally comprise B.100 as further herbicide B.

Also especially preferred are compositions 30.1. to 30.3383 which differ from the corresponding compositions 1.1 to 1.3383 only in that they additionally comprise B.100 and B.67 as further herbicides B.

Also especially preferred are compositions 31.1. to 31.3383 which differ from the corresponding compositions 1.1 to 1.3383 only in that they additionally comprise B.100 and B.76 as further herbicides B.

Also especially preferred are compositions 32.1. to 32.3383 which differ from the corresponding compositions 1.1 to 1.3383 only in that they additionally comprise B.100 and B.82 as further herbicides B.

Also especially preferred are compositions 33.1. to 33.3383 which differ from the corresponding compositions 1.1 to 1.3383 only in that they additionally comprise B.101 as further herbicide B.

Also especially preferred are compositions 34.1. to 34.3383 which differ from the corresponding compositions 1.1 to 1.3383 only in that they additionally comprise B.101 and B.67 as further herbicides B.

Also especially preferred are compositions 35.1. to 35.3383 which differ from the corresponding compositions 1.1 to 1.3383 only in that they additionally comprise B.101 and B.76 as further herbicides B.

Also especially preferred are compositions 36.1. to 36.3383 which differ from the corresponding compositions 1.1 to 1.3383 only in that they additionally comprise B.101 and B.82 as further herbicides B.

Also especially preferred are compositions 37.1. to 37.3383 which differ from the corresponding compositions 1.1 to 1.3383 only in that they additionally comprise B.103 as further herbicide B.

Also especially preferred are compositions 38.1. to 38.3383 which differ from the corresponding compositions 1.1 to 1.3383 only in that they additionally comprise B.104 as further herbicide B.

Also especially preferred are compositions 39.1. to 39.3383 which differ from the corresponding compositions 1.1 to 1.3383 only in that they additionally comprise B. 104 and B.67 as further herbicides B.

Also especially preferred are compositions 40.1. to 40.3383 which differ from the corresponding compositions 1.1 to 1.3383 only in that they additionally comprise B. 104 and B.76 as further herbicides B.

Also especially preferred are compositions 41.1. to 41.3383 which differ from the corresponding compositions 1.1 to 1.3383 only in that they additionally comprise B. 104 and B.82 as further herbicides B.

Also especially preferred are compositions 42.1. to 42.3383 which differ from the corresponding compositions 1.1 to 1.3383 only in that they additionally comprise B.106 as further herbicide B.

Also especially preferred are compositions 43.1. to 43.3383 which differ from the corresponding compositions 1.1 to 1.3383 only in that they additionally comprise B.107 as further herbicide B.

Also especially preferred are compositions 44.1. to 44.3383 which differ from the corresponding compositions 1.1 to 1.3383 only in that they additionally comprise B.107 and B.67 as further herbicides B.

Also especially preferred are compositions 45.1. to 45.3383 which differ from the corresponding compositions 1.1 to 1.3383 only in that they additionally comprise B.107 and B.76 as further herbicides B.

Also especially preferred are compositions 46.1. to 46.3383 which differ from the corresponding compositions 1.1 to 1.3383 only in that they additionally comprise B.107 and B.82 as further herbicides B.

Also especially preferred are compositions 47.1. to 47.3383 which differ from the corresponding compositions 1.1 to 1.3383 only in that they additionally comprise B.112 as further herbicide B.

Also especially preferred are compositions 48.1. to 48.3383 which differ from the corresponding compositions 1.1 to 1.3383 only in that they additionally comprise B.112 and B.67 as further herbicides B.

Also especially preferred are compositions 49.1. to 49.3383 which differ from the corresponding compositions 1.1 to 1.3383 only in that they additionally comprise B.112 and B.92 as further herbicides B.

Also especially preferred are compositions 50.1. to 50.3383 which differ from the corresponding compositions 1.1 to 1.3383 only in that they additionally comprise B.112 and B.100 as further herbicides B.

Also especially preferred are compositions 51.1. to 51.3383 which differ from the corresponding compositions 1.1 to 1.3383 only in that they additionally comprise B.112 and B.124 as further herbicides B.

Also especially preferred are compositions 52.1. to 52.3383 which differ from the corresponding compositions 1.1 to 1.3383 only in that they additionally comprise B.112 and B.101 as further herbicides B.

Also especially preferred are compositions 53.1. to 53.3383 which differ from the corresponding compositions 1.1 to 1.3383 only in that they additionally comprise B.112 and B.104 as further herbicides B.

Also especially preferred are compositions 54.1. to 54.3383 which differ from the corresponding compositions 1.1 to 1.3383 only in that they additionally comprise B.112 and B.107 as further herbicides B.

Also especially preferred are compositions 55.1. to 55.3383 which differ from the corresponding compositions 1.1 to 1.3383 only in that they additionally comprise B.118 as further herbicide B.

Also especially preferred are compositions 56.1. to 56.3383 which differ from the corresponding compositions 1.1 to 1.3383 only in that they additionally comprise B.122 as further herbicide B.

Also especially preferred are compositions 57.1. to 57.3383 which differ from the corresponding compositions 1.1 to 1.3383 only in that they additionally comprise B.124 as further herbicide B.

Also especially preferred are compositions 58.1. to 58.3383 which differ from the corresponding compositions 1.1 to 1.3383 only in that they additionally comprise B.127 as further herbicide B.

Also especially preferred are compositions 59.1. to 59.3383 which differ from the corresponding compositions 1.1 to 1.3383 only in that they additionally comprise B.128 as further herbicide B.

Also especially preferred are compositions 60.1. to 60.3383 which differ from the corresponding compositions 1.1 to 1.3383 only in that they additionally comprise B.129 as further herbicide B.

Also especially preferred are compositions 61.1. to 61.3383 which differ from the corresponding compositions 1.1 to 1.3383 only in that they additionally comprise B.131 as further herbicide B.

Also especially preferred are compositions 62.1. to 62.3383 which differ from the corresponding compositions 1.1 to 1.3383 only in that they additionally comprise B.133 as further herbicide B.

Also especially preferred are compositions 63.1. to 63.3383 which differ from the corresponding compositions 11.1 to 1.3383 only in that they additionally comprise B.134 as further herbicide B.

Also especially preferred are compositions 64.1. to 64.3383 which differ from the corresponding compositions 1.1 to 1.3383 only in that they additionally comprise B.139 as further herbicide B.

Also especially preferred are compositions 65.1. to 65.3383 which differ from the corresponding compositions 1.1 to 1.3383 only in that they additionally comprise B.146 as further herbicide B.

Also especially preferred are compositions 66.1. to 66.3383 which differ from the corresponding compositions 1.1 to 1.3383 only in that they additionally comprise B.149 as further herbicide B.

Also especially preferred are compositions 67.1. to 67.3383 which differ from the corresponding compositions 1.1 to 1.3383 only in that they additionally comprise B.162 as further herbicide B.

Also especially preferred are compositions 68.1. to 68.3383 which differ from the corresponding compositions 1.1 to 1.3383 only in that they additionally comprise B.165 as further herbicide B.

Also especially preferred are compositions 69.1. to 69.3383 which differ from the corresponding compositions 1.1 to 1.3383 only in that they additionally comprise B.176 as further herbicide B.

Hereinbelow, the compounds of the formula I are illustrated by way of examples, without limiting the subject matter of the present invention to the examples shown.

### I. Synthesis examples

Compounds I of the invention were characterized by retention time in minutes (RT) and the mass [m/z] in atomic mass units of the positive ion resulting from protonation of the compound (M+1). For compounds I containing chlorine or bromine, the value reported is for the species that contains only the most abundant isotopes, CI³⁵ and Br⁷⁹. Values for RT and M+1 were determined by high performance liquid chromatography (HPLC)-coupled chemical ionization mass spectrometry, using the following method:
Column: Reverse-phase 1.7 micron C-18 (Kinetex XB), 2.1 x 50 mm.
Mobile Phases: A: water + 0.1 % trifluoroacetic acid; B: acetonitrile + 0.1 % trifluoroacetic acid.
Gradient: from 5% A + 95% B to 100% A + 0% B during 1.5 minutes.
Column Temperature: 60°C.
Flow rate: 0.8 to 1.0 mL/minute.
Mass Detection: Quadrupole electrospray ionisation, 80 V, positive mode.
Apparatus: Shimadzu (Nexera LC-30, LCMS-2020).

Some compounds I were also characterized by their ¹H-NMR spectrum, recorded on a Bruker Avance II 300.

Preparative column chromatography was performed on a Teledyne Isco Combiflash Rf unit.

### Example 1: Preparation of 5-difluoromethyl-1-ethylpyrazole-4-carboxylate, cyclopropyl amide (see compound I-5 in the Table I below)

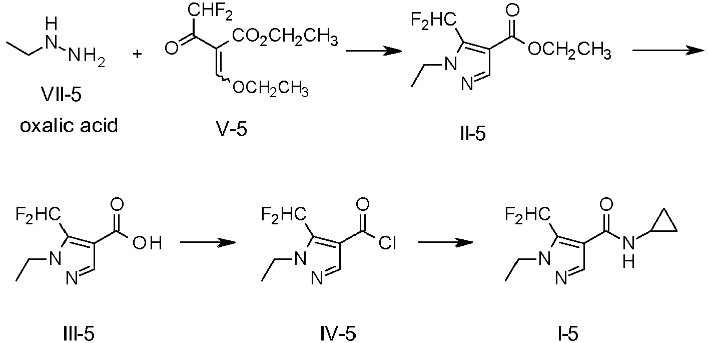

Step 1: 150 mL 1 N NaOH was cooled to 0°C with stirring, enol ether V-5 (30.0 g) was added in one portion, and the mixture was stirred for a further 30 minutes at this temperature. Then 200 mL of chloroform was added, and maintaining a temperature of 0°C, the oxalic acid salt of the hydrazine VII-5 (30.4 g) was added in portions over 30 minutes. After another 30 minutes of stirring at 0°C, 130 mL 2N HCl was added, and the resulting mixture stirred a further 30 minutes at 0°C. The phases were then separated, the water phase was washed with three portions of methylene chloride, and the combined organic phases were washed with brine, dried over magnesium sulfate, and evaporated to dryness under reduced pressure. The resulting crude product was purified by chromatography (silica gel, cyclohexane-ethyl acetate gradient) to give 14.6 g of the ester II-5, M+1 = 219.3, RT 1.083.
Step 2: A mixture of ester II-5 (14.6 g), NaOH (10.8 g) and 200 mL 1:1 ethanol-water was refluxed for two hours, the ethanol was removed by evaporation under reduced pressure, and the resulting aqueous phase was acidified with 2N HCl to pH1. The resulting precipitate was collected by filtration, washed with water and hexanes, and dried to give 12.9 g of the acid III-5, M+1 = 191.3, RT 0.788.
Step 3: To a stirred mixture of the acid III-5 (12.9 g) and 100 mL toluene was added thionyl chloride (14.9 mL, 24.3 g), dropwise at room temperature (gas evolution). The mixture was then refluxed for 2 hours, cooled, and evaporated to dryness with a rotary evaporator, leaving 14.0 g of crude acid chloride IV-5, which was used without further purification in the next step.
Step 4: A stirred mixture of cyclopropylamine (5.6 mL, 4.6 g), triethylamine (18.6 mL, 13.6 g) and 50 mL dichloromethane was cooled to 0°C, and a solution of acid chloride IV-5 (14.0 g) in 50 mL chloroform was added dropwise at this temperature with continued stirring. The mixture was then allowed to warm to room temperature and stirred overnight. Addition of water, separation of the phases, washing the aqueous phase with three portions of methylene chloride, washing the combined organic phases with brine, drying over magnesium sulfate, and evaporation to dryness under reduced pressure provided 12.9 g of compound I-5 as a tan solid, M+1 = 230.4, RT 0.848.

Alternatively, the ethyl hydrazine VII-5 can also be employed in step 1 above as the bis-HCl salt. The preparation of the bis-HCl salt of VII-5 is illustrated in the scheme below and will be further outlined in the following. 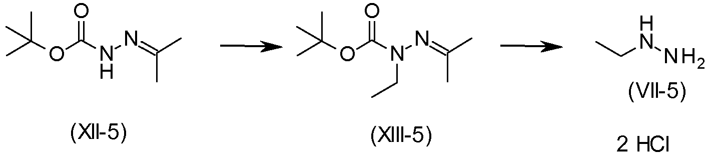
Step 1: A mixture of t-butylcarbazate acetone hydrazine XII-5 (10.3 g), powdered KOH (4.4 g), tetrabutylammonium hydrogen carbonate (2.0 g) and 200 mL of toluene was stirred and warmed to 50 °C, at which temperature neat ethyl bromide (5.2 mL, 7.8 g) was added dropwise. When addition was complete, the reaction mixture was warmed to 80 °C for two hours, then cooled to room temperature and washed with water until the washings were neutral. The resulting toluene solution was dried over magnesium sulfate and evaporated at reduced pressure to give 12.5 g crude product as an oil. Chromatography (silica gel, cyclohexane-ethyl acetate gradient) provided 8.32 g of pure N-ethyl carbazate XIII-5 as an oil; ¹H-NMR (CDCl₃): δ = 1.1 (3H, triplet), 1.4 (9H, singlet), 1.8 (3H, singlet), 2.0 (3H, singlet), 3.6 (2H, quartet). Such compounds did not give M+1 peaks in the mass spectrometer used.
Step 2: A mixture of compound XIII-5 (5.0 g), 25 mL 2N HCl and 50 mL tetrahydrofuran was refluxed for three hours, and then solvents were removed under reduced pressure. To remove traces of water, toluene was added to the residue and removed under reduced pressure. This procedure was repeated a total of 3 times, leaving a white solid that was washed with a small amount of ethanol and dried to give 3.3 g of the bis-HCl salt of the hydrazine VII-5; ¹H-NMR (DMSO-d6): δ = 1.1 (3H, triplet), 3.0 (2H, quartet). Such compounds did not give M+1 peaks in the mass spectrometer used.

### Example 2: Preparation of 1-allyl-5-difluoromethylpyrazole-4-carboxylate, cyclopropyl amide (see compound I-20 in the Table I below)

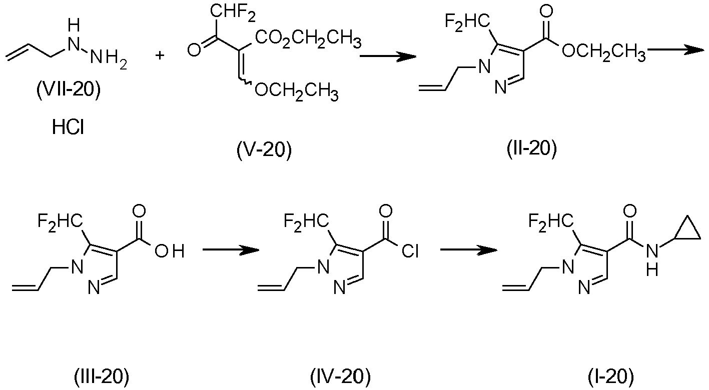

Step 1: A solution of allyl hydrazine VII-20 (23.4 g) in 800 mL ethanol was cooled to 0°C with stirring, and 27.1 mL 12N HCl was added at this temperature, followed by a solution of enol ether V-20 (65.0 g) in 200 mL ethanol. The resulting solution was stirred overnight at room temperature, after which ethanol was removed under reduced pressure, the residue was diluted with water, and 5% NaHCO₃ solution was added to bring the pH to 7. The resulting mixture was extracted with three portions of ethyl acetate, and the combined organic phases were washed twice with brine, dried over magnesium sulfate, and evaporated to dryness under reduced pressure to give 45.5 g of ester II-20, which was carried on to the next step without further purification; M+1 = 231.2, RT 0.998.
Step 2: To a solution of ester II-20 (45.5 g) in 200 mL ethanol was added a solution of NaOH (23.7 g) in 200 mL water, and the resulting mixture was stirred overnight at room temperature. Ethanol was then removed by evaporation under reduced pressure, and the resulting aqueous phase was acidified with 2N HCl to pH1 followed by extraction with three portions of ethyl acetate. The combined organic phases were washed twice with brine, dried over magnesium sulfate, and evaporated to dryness under reduced pressure to give 29.8 g of acid III-20, which was carried on to the next step without further purification; M+1 = 203.3, RT 0.807.
Step 3: A mixture of the acid III-20 (29.8 g) and 500 mL toluene was brought to reflux with stirring, and thionyl chloride (22.8 g) was added dropwise. The resulting mixture was refluxed for 2 hours, cooled, and evaporated to dryness with a rotary evaporator, leaving 31.3 g crude acid chloride IV-20, which was used without further purification in the next step.
Step 4: A mixture of cyclopropylamine (14.8 mL, 12.1 g), triethylamine (39.3 mL, 28.7 g) and 500 mL dichloromethane was stirred at room temperature while a solution of acid chloride IV-20 (14.0 g) in 50 mL chloroform was added dropwise at this temperature with continued stirring. The mixture was then allowed to warm to room temperature and stirred overnight. Addition of water, separation of the phases, washing the aqueous phase with three portions of methylene chloride, washing the combined organic phases with brine, drying over magnesium sulfate, and evaporation to dryness under reduced pressure provided 29.0 g of crude product, which was recrystallized from n-hexanes to give 17.2 g of compound I-20 as a tan solid, M+1 = 242.2, RT 0.880.

With appropriate modification of the starting materials, the procedures given in the synthesis examples above were used to obtain further pyrazole amide compounds of formula I. The compounds obtained in this manner are listed in the table that follows, together with their retention time RT and observed mass M+1, as described above.

**Table I: Compounds of the formula I**

| No. | R¹ | R2 | R3 | M+1 | RT |
|---|---|---|---|---|---|
| I-01 | H | cyclopropyl | H | 202.3 | 0.579 |
| I-02 | methyl | methyl | H | 190.0 | 0.659 |
| I-03 | methyl | cyclopropyl | H | 216.3 | 0.756 |
| I-04 | ethyl | methyl | H | 204.0 | 0.767 |
| I-05 | ethyl | cyclopropyl | H | 230.3 | 0.858 |
| I-06 | 2-fluoroethyl | methyl | H | 222.3 | 0.670 |
| I-07 | 2-fluoroethyl | cyclopropyl | H | 248.3 | 0.770 |
| I-08 | 2,2-difluoroethyl | methyl | H | 240.1 | 0.766 |
| I-09 | 2,2-difluoroethyl | cyclopropyl | H | 266.2 | 0.865 |
| I-10 | 2,2,2-trifluoroethyl | methyl | H | 258.2 | 0.824 |
| I-11 | 2,2,2-trifluoroethyl | cyclopropyl | H | 284.3 | 0.923 |
| I-12 | 2-methoxyethyl | methyl | H | 234.3 | 0.687 |
| I-13 | 2-methoxyethyl | cyclopropyl | H | 260.3 | 0.778 |
| I-14 | 2-ethoxyethyl | methyl | H | 248.2 | 0.804 |
| I-15 | 2-ethoxyethyl | cyclopropyl | H | 274.2 | 0.888 |
| I-16 | n-propyl | cyclopropyl | H | 244.1 | 0.943 |
| I-17 | i-propyl | methyl | H | 218.3 | 0.852 |
| I-18 | i-propyl | cyclopropyl | H | 244.3 | 0.946 |
| I-19 | allyl | methyl | H | 216.3 | 0.787 |
| I-20 | allyl | cyclopropyl | H | 242.2 | 0.880 |
| I-21 | 3,3-dimethylallyl | methyl | H | 244.2 | 0.985 |
| I-22 | 3,3-dimethylallyl | cyclopropyl | H | 270.2 | 1.060 |
| I-23 | 1,1-d ifl uoroprop-2-yl | methyl | H | 254.2 | 0.890 |
| I-24 | 1,1-d ifl uoroprop-2-yl | cyclopropyl | H | 280.1 | 0.972 |
| I-25 | n-butyl | methyl | H | 232.3 | 0.945 |
| I-26 | n-butyl | cyclopropyl | H | 258.3 | 1.026 |
| I-27 | i-butyl | methyl | H | 232.1 | 0.944 |
| I-28 | i-butyl | cyclopropyl | H | 258.1 | 1.023 |
| I-29 | t-butyl | methyl | H | 232.0 | 0.921 |
| I-30 | t-butyl | cyclopropyl | H | 258.0 | 1.012 |
| I-31 | vinyl | methyl | H | 202.3 | 0.772 |
| I-32 | vinyl | cyclopropyl | H | 228.3 | 0.873 |
| I-33 | cyclopropylmethyl | methyl | H | 230.2 | 0.884 |
| I-34 | cyclopropylmethyl | cyclopropyl | H | 256.2 | 0.969 |
| I-35 | cyclobutyl | methyl | H | 230.3 | 0.909 |
| I-36 | cyclobutyl | cyclopropyl | H | 256.3 | 0.997 |
| I-37 | cyclopentyl | methyl | H | 244.2 | 1.017 |
| I-38 | cyclopentyl | cyclopropyl | H | 270.2 | 1.090 |
| I-39 | 4,4-difluoro-3-butenyl | methyl | H | 266.1 | 0.943 |
| I-40 | 4,4-difluoro-3-butenyl | cyclopropyl | H | 292.2 | 1.019 |
| I-41 | cyclopropyl | methyl | H | 216.3 | 0.762 |
| I-42 | cyclopropyl | cyclopropyl | H | 242.4 | 0.858 |
| I-43 | 2-hydroxyethyl | cyclopropyl | H | 246.1 | 0.667 |
| I-44 | 3-chloro-3-methylbutyl | cyclopropyl | H | 306.1 | 1.094 |
| I-45 | tetrahydrofurfuryl | cyclopropyl | H | 286.2 | 0.866 |
| I-46 | 2-methylallyl | cyclopropyl | H | 256.3 | 0.928 |
| I-47 | s-butyl | cyclopropyl | H | 258.4 | 1.002 |
| I-48 | t-butyl | methoxy | H | 248.2 | 0.893 |
| I-49 | t-butyl | methoxy | methyl | 262.4 | 0.970 |
| I-50 | 2-butynyl | cyclopropyl | H | 254.3 | 0.878 |
| I-51 | propargyl | cyclopropyl | H | 240.3 | 0.795 |
| I-52 | 2-chloroallyl | cyclopropyl | H | 276.2 | 0.929 |
| I-53 | H | cyclopropyl | methyl | 216.3 | 0.646 |
| I-54 | t-butyl | cyclopropyl | methyl | 272.4 | 0.898 |
| I-55 | 3,7-dimethyl-7-hydroxyoctyl (*) | cyclopropyl | H | 378.3 | 1.044 |
| I-56 | allenyl | cyclopropyl | H | 240.3 | 0.862 |
| I-57 | 3-hydroxy-3-methylbutyl | cyclopropyl | H | 288.3 | 0.802 |

| | | | | | |
|---|---|---|---|---|---|
| (*) Peak is M+23 (M+Na⁺). A small peak at 356.3 (M+1) is also visible. | | | | | |

### II. Use examples

The herbicidal activity of the compounds of formula I was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this has been impaired by the active ingredients.

For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the plants were kept at 10 - 25°C or 20 - 35°C. The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A good herbicidal activity is given at values of at least 70 and a very good herbicidal activity is given at values of at least 85.

The plants used in the greenhouse experiments belonged to the following species:

| Bayer code | Scientific name | English name |
|---|---|---|
| ABUTH | Abutilon theophrasti | velvetleaf |
| ALOMY | Alopecurus myosuroides | blackgrass |
| AMARE | Amaranthus retroflexus | common amaranth |
| APESV | Apera spica-venti | windgrass |
| AVEFA | Avena fatua | wild oat |
| CHEAL | Chenopodium album | lambsquarters |
| ECHCG | Echinocloa crus-galli | common barnyardgrass |
| LOLMU | Lolium multiflorum | Italian ryegrass |
| POLCO | Polygonum convolvulus | wild buckwheat |
| SETFA | Setaria faberi | giant foxtail |
| SETVI | Setaria viridis | green foxtail |

At an application rate of 500 g/ha, applied by the post-emergence method to ALOMY, the following pyrazole amide compounds of formula I showed a very good herbicidal activity: I-42; and the following pyrazole amide compounds of formula I showed a good herbicidal activity: I-03, I-40.

At an application rate of 500 g/ha, applied by the post-emergence method to AMARE, the following pyrazole amide compounds of formula I showed a very good herbicidal activity: I-02, I-03, I-04, I-05, I-07, I-09, I-11, I-16, I-18, I-19, I-20, I-22, I-24, I-25, I-26, I-27, I-28, I-29, I-30, I-32, I-33, I-34, I-35, I-36, I-37, I-38, I-39, I-40, I-41, I-42; and the following pyrazole amide compounds of formula I showed a good herbicidal activity: I-17, I-21, I-23.

At an application rate of 500 g/ha, applied by the post-emergence method to AVEFA, the following pyrazole amide compounds of formula I showed a very good herbicidal activity: I-03. I-05, I-30, I-32, I-42; and the following pyrazole amide compounds of formula I showed a good herbicidal activity: I-09, I-34, I-40, I-41.

At an application rate of 500 g/ha, applied by the post-emergence method to CHEAL, the following pyrazole amide compounds of formula I showed a very good herbicidal activity: I-02, I-03, I-04, I-05, I-06, I-07, I-08, I-09, I-11, I-13, I-15, I-16, I-17, I-18, I-19, I-20, I-22, I-23, I-24, I-25, I-26-I-27, I-28, I-29, I-30, I-31, I-32, I-33, I-34, I-35, I-36, I-37, 1-38, I-39, I-40, I-41, I-42; and the following pyrazole amide compounds of formula I showed a good herbicidal activity: I-12, I-43.

At an application rate of 500 g/ha, applied by the post-emergence method to ECHCG, the following pyrazole amide compounds of formula I showed a very good herbicidal activity: I-16, I-22, I-26, I-30, I-34, I-35, I-36, I-38, I-40, I-42; and the following pyrazole amide compounds of formula I showed a good herbicidal activity: I-03, I-05, I-07, I-09, I-18, I-20, I-23.

At an application rate of 500 g/ha, applied by the post-emergence method to LOLMU, the following pyrazole amide compounds of formula I showed a very good herbicidal activity: I-03, I-05, I-07, I-30, I-32, I-34, I-36, I-40, I-42; and the following pyrazole amide compounds of formula I showed a good herbicidal activity: 1-09, I-16, I-20, I-24, I-41.

At an application rate of 500 g/ha, applied by the post-emergence method to POLCO, the following pyrazole amide compounds of formula I showed a very good herbicidal activity: I-03, I-09, I-18, I-20, I-22, I-24, I-26, I-30, I-32, I-36, I-38, I-40, I-42; and the following pyrazole amide compounds of formula I showed a good herbicidal activity: I-05, I-07, I-16, I-40, I-41.

At an application rate of 500 g/ha, applied by the post-emergence method to SETVI, the following pyrazole amide compounds of formula I showed a very good herbicidal activity: I-05, I-09, I-16, I-18, I-24, I-26, I-28, I-30, I-32, I-34, I-35, I-36, 1-38, I-40, I-42; and the following pyrazole amide compounds of formula I showed a good herbicidal activity: I-03, I-07, I-20.

At an application rate of 500 g/ha, applied by the pre-emergence method to ABUTH, the following pyrazole amide compounds of formula I showed a very good herbicidal activity: I-03, I-04, I-05, I-07, I-09, I-16, I-18, I-20, I-24, I-30, I-32, I-34, I-35, I-42; and the following pyrazole amide compounds of formula I showed a good herbicidal activity: I-33, I-41.

At an application rate of 500 g/ha, applied by the pre-emergence method to ALOMY, the following pyrazole amide compounds of formula I showed a very good herbicidal activity: I-03, I-05, I-30, I-32, I-42.

At an application rate of 500 g/ha, applied by the pre-emergence method to AMARE, the following pyrazole amide compounds of formula I showed a very good herbicidal activity: I-03, I-04, I-05, I-07, I-09, I-11, I-16, I-17, I-18, I-20, I-23, I-24, I-30, I-33, I-34, I36, I-41, I-42; and the following pyrazole amide compounds of formula I showed a good herbicidal activity: I-02, I-06, I-08, I-19, I-28, I-29.

At an application rate of 500 g/ha, applied by the pre-emergence method to APESV, the following pyrazole amide compounds of formula I showed a very good herbicidal activity: I-02, I-03, I-04, I-05, I-07, I-09, I-11, I-16, I-18, I-19, I-20, I-24, I-28, I-29, I-30, I-31, I-32, I-33, I-34, I-36, I-40, I-41, I-42; and the following pyrazole amide compounds of formula I showed a good herbicidal activity: I-08.

At an application rate of 500 g/ha, applied by the pre-emergence method to ECHCG, the following pyrazole amide compounds of formula I showed a very good herbicidal activity: I-03, I-05, I-07, I-20, I-30, I-41, I-42; and the following pyrazole amide compounds of formula I showed a good herbicidal activity: I-04, I-16, I-24, I-36.

At an application rate of 500 g/ha, applied by the pre-emergence method to SETFA, the following pyrazole amide compounds of formula I showed a very good herbicidal activity: I-03, I-05, I-07, I-09, I-16, I-18, I-20, I-24, I-30, I-32, I-40, I-41, I-42; and the following pyrazole amide compounds of formula I showed a good herbicidal activity: I-04, I-28, I-29, I-34.

## Claims

1. A pyrazole amide compound of the formula I 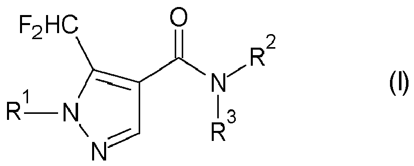 wherein
R¹ is H, C₁-C₁₀-alkyl, C₁-C₁₀-haloalkyl, C₂-C₁₀-hydroxyalkyl, C₁-C₆-alkoxy-C₁-C₆-alkyl, C₁-C₆-haloalkoxy-C₁-C₆-alkyl, C₁-C₆-alkylthio-C₁-C₆-alkyl, C₁-C₆-haloalkylthio-C₁-C₆-alkyl, C₁-C₆-alkylsulfinyl-C₁-C₆-alkyl, C₁-C₆-haloalkylsulfinyl-C₁-C₆-alkyl, C₁-C₆-alkylsulfonyl-C₁-C₆-alkyl, C₁-C₆-haloalkylsulfonyl-C₁-C₆-alkyl, C₃-C₆-cydoalkyl, 1-methylcycloprop-1-yl, 2-methylcycloprop-1-yl, 2,2-dimethylcycloprop-1-yl, 2,2,3,3-tetramethylcycloprop-1-yl, C₃-C₆-halocycloalkyl, C₃-C₆-cycloalkyl-C₁-C₆-alkyl, C₃-C₆-halocycloalkyl-C₁-C₆-alkyl, C₂-C₁₀-alkenyl, C₂-C₁₀-haloalkenyl, C₂-C₁₀-hydroxyalkenyl, C₃-C₁₀-alkadienyl, C₃-C₆-alkynyl, C₃-C₆-haloalkynyl, CH₂CN, CH(CN)₂, N,N-di-(C₁-C₆)-alkylamino-C₁-C₆-alkyl, C₁-C₆-dialkoxy-C₁-C₆-alkyl, C₁-C₆-dialkylthio-C₁-C₆-alkyl, C₁-C₃-alkoxy-C₁-C₃-alkylthio-C₁-C₆-alkyl, or a heterocyclic group selected from the formulae H1, H2 or H3 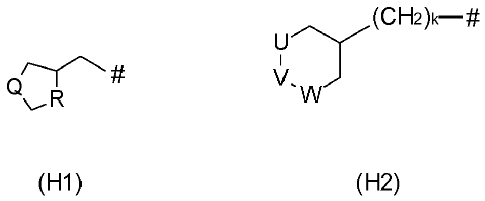 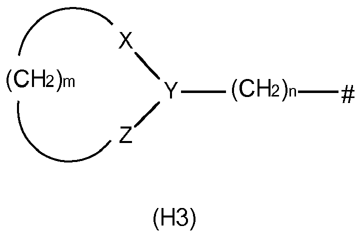 wherein
each of Q and R in the formula H1 is O or S;
U-V-W in the formula H2 is selected from the group consisting of CH₂-CH₂-O, CH₂-CH₂-NH, CH₂-CH₂-N(CH₃), CH₂-0-CH₂, CH₂-NH-CH₂, CH₂-N(CH₃)-CH₂, O-CH₂-O,
O-CH₂-S, and S-CH₂-S;
k in the formula H2 is 0 or 1;
X-Y-Z in the formula H3 is selected from the group consisting of CH₂-N-CH₂, O-CH-CH₂, O-CH-O, S-CH-CH₂, S-CH-S, and O-CH-S;
m in the formula H3 is 1, 2 or 3;
n in the formula H3 is 0, 1 or 2, with the proviso that, when n is 0, X-Y-Z is not CH₂-N-CH₂;
# in each of the formulae H1, H2 or H3 denotes the bonding site to the remainder of the formula I;
R² is hydrogen, C₁-C₄-alkyl, C₃-C₆-cycloalkyl or C₁-C₄-alkoxy; and
R³ is hydrogen, C₁-C₄-alkyl, C₃-C₆-cycloalkyl or C₁-C₄-alkoxy;
or an agriculturally acceptable salt thereof.

2. The compound according to claim 1 wherein
R¹ is C₁-C₄-alkyl, cyclopropyl, 1-methylcycloprop-1-yl, cyclopropylmethyl, cyclobutyl, C₁-C₄-haloalkyl, C₂-C₄-hydroxyalkyl, C₁-C₂-alkoxy-C₁-C₂-alkyl, C₂-C₅-alkenyl, allenyl, C₃-C₅-haloalkenyl, C₃-C₄-alkynyl, CH₂CN or CH(CN)₂;
R² is C₁-C₄-alkyl or C₃-C₆-cycloalkyl; and
R³ is hydrogen or methyl;
or an agriculturally acceptable salt thereof.

3. The compound according to claim 1 or 2 wherein
R¹ is C₁-C₄-alkyl, cyclopropyl, 1-methylcycloprop-1-yl, cyclopropylmethyl, cyclobutyl, C₁-C₄-haloalkyl, C₂-C₄-hydroxyalkyl, C₁-C₂-alkoxy-C₁-C₂-alkyl, C₂-C₅-alkenyl, allenyl, C₃-C₅-haloalkenyl, C₃-C₄-alkynyl, CH₂CN or CH(CN)₂;
R² is cyclopropyl; and
R³ is hydrogen;
or an agriculturally acceptable salt thereof.

4. The compound according to any of claims 1 to 3 wherein
R¹ is ethyl, 1-propyl, allyl, cyclopropyl, 1-methylcycloprop-1-yl, cyclopropylmethyl, cyclobutyl or t-butyl;
R² is cyclopropyl; and
R³ is hydrogen;
or an agriculturally acceptable salt thereof.

5. A composition comprising a herbicidally effective amount of at least one compound of the formula I as defined in any of claims 1 to 4 or an agriculturally acceptable salt thereof, and auxiliaries customary for formulating crop protection agents.

6. The composition according to claim 5, further comprising one or more further active substances independently selected from the group consisting of herbicides, fungicides, insecticides, safeners, and plant growth regulators.

7. A method for preparing a composition as defined in claim 5, which comprises mixing a herbicidally effective amount of at least one compound of the formula I as defined in any of claims 1 to 4 or of an agriculturally acceptable salt thereof, and auxiliaries customary for formulating crop protection agents.

8. A method for preparing a composition as defined in claim 6, which comprises mixing a herbicidally effective amount of at least one compound of formula I as defined in any of claims 1 to 4 or of an agriculturally acceptable thereof, auxiliaries customary for formulating crop protection agents, and one or more further active substances.

9. A method for preparing a composition as defined in claim 6, which comprises mixing the composition as defined in claim 5, at least one composition containing one or more further active substances independently selected from the group consisting of herbicides, fungicides, insecticides, safeners, and plant growth regulators, and water.

10. A method for controlling unwanted vegetation, which comprises allowing a herbicidally effective amount of at least one compound of formula I as defined in any of claims 1 to 4 or of an agriculturally acceptable salt thereof, or a composition as defined in claim 5 or 6, to act on plants, their seeds and/or their habitat.
